# EUROPEAN PATENT SPECIFICATION

(11) **EP 2 556 169 B1**
(45) Date of publication and mention of the grant of the patent: **14.02.2018**
(21) Application number: 11717710.5
(22) Date of filing: 06.04.2011
(51) Int. Cl.: C12Q 1/68

(54) **METHODS FOR CATEGORISING CANCER SUCH AS BREAST CANCER**
VERFAHREN ZUR KLASSIFIZIERUNG VON KREBS WIE ETWAS BRUSTKREBS
PROCÉDÉS

(30) Priority: 09.12.2010 GB 201020854; 30.09.2010 GB 201016459; 07.04.2010 GB 201005761
(43) Date of publication of application: 13.02.2013
(73) Proprietor: Imperial Innovations Limited, London SW7 2PG (GB)
(72) Inventor: GIAMAS, Georgios, London W12 0NN (GB); STEBBING, Justin, London W6 8RF (GB)
(74) Representative: Potter Clarkson LLP
(86) International application number: PCT/GB2011/000529
(87) International publication number: WO 2011/124884

(56) References cited:
- WO-A2-2004/091511
- S. NAIK ET AL: "Vascular Endothelial Growth Factor Receptor-1 Is Synthetic Lethal to Aberrant -Catenin Activation in Colon Cancer", CLINICAL CANCER RESEARCH, vol. 15, no. 24, 15 December 2009 (2009-12-15), pages 7529-7537, XP55001214, ISSN: 1078-0432, DOI: 10.1158/1078-0432.CCR-09-0336
- "Supplementary data for XP55001214", , 14 December 2009 (2009-12-14), XP55001222, Retrieved from the Internet: URL:http://clincancerres.aacrjournals.org/ content/suppl/2009/12/18/15.24.7529.DC2/Su pplementary_Data.pdf [retrieved on 2011-06-22]
- KASAMATSU A ET AL: "Identification of candidate genes associated with salivary adenoid cystic carcinomas using combined comparative genomic hybridization and oligonucleotide microarray analyses", INTERNATIONAL JOURNAL OF BIOCHEMISTRY AND CELL BIOLOGY, EXETER, GB, vol. 37, no. 9, 1 September 2005 (2005-09-01), pages 1869-1880, XP004974399, ISSN: 1357-2725, DOI: DOI:10.1016/J.BIOCEL.2005.04.004
- "Affymetrix page demonstrating that SNP arrays 5.0 and 6.0 comprise probes for LMTK3", , 15 October 2009 (2009-10-15), XP55001229, [retrieved on 2011-06-22]
- C. D. GREENMAN ET AL: "PICNIC: an algorithm to predict absolute allelic copy number variation with microarray cancer data", BIOSTATISTICS, vol. 11, no. 1, 1 January 2010 (2010-01-01), pages 164-175, XP55001227, ISSN: 1465-4644, DOI: 10.1093/biostatistics/kxp045
- ALI S ET AL: "ENDOCRINE-RESPONSIVE BREAST CANCER AND STRATEGIES FOR COMBATING RESISTANCE", NATURE REVIEWS. CANCER, NATUR PUBLISHING GROUP, LONDON, GB, vol. 2, no. 2, 1 February 2002 (2002-02-01), pages 102-112, XP009018798, ISSN: 1474-175X, DOI: 10.1038/NRC721

## Description

With an annual incidence of 1.3 million cases and 465000 deaths, breast cancer (BC) remains the most frequently diagnosed type of cancer and the leading cause of cancer mortality in females worldwide (1). As two-thirds of breast tumors have been shown to be ERα-positive (2), it is not surprising that ERα represents one of the most successful therapeutic targets for BC (3). Various studies have demonstrated that ERα over-expression increases the risk of BC. Holst et al. (4) showed that 21% of breast tumours had amplification of the *ESR1* gene. Moreover, 99% of these tumors which had *ESR1* gene amplification also over-expressed ERα, demonstrating the functional importance of *ESR1* gene amplification (4). Moreover, immunohistochemical analysis of breast tissue revealed higher expression levels of ERα in breast cancer patients and a correlation between ERα expression and breast cancer risk in post-menopausal women (5). In addition, deregulation of ERα expression in transgenic mice resulted in the development of ductal carcinoma in situ (6). A recent study showed that the positive cross-talk between ER and NFkB increases the transcription of cell survivor genes, thus protecting breast cancer cells against apoptosis (7). Based on these observations, classifying breast tumors as ER-positive or -negative is important in order to determine endocrine responsive therapy (8).

However, emergence of resistance to endocrine therapies is not uncommon, particularly in breast cancer; ERα activity is regulated by phosphorylation and it is one of the proposed mechanisms leading to resistance. Phosphorylation of ERα at various sites by different kinases may increase or decrease its transcriptional activity (9,10). In addition, it has been shown that phosphorylation of AIB1, a protein frequently over-expressed in breast tumors, by aPKC results in AIB1 stabilization and enhancement of ERα activity (11). Apart from regulating the transcriptional activity of ERα, phosphorylation can also alter the stability of the protein. In particular, GSK-3 silencing reduces estrogen-dependent phosphorylation of ERα at S118, and targets ERα for proteasomal degradation (12). Furthermore, we have identified CK1δ as a new kinase that is able to phosphorylate ERα, while CK1δ silencing stabilized ERα despite reducing ERα-mediated transcriptional activity (13). Recently, a study using antibodies specific to individual identified phosphorylated sites within ERα demonstrated the existence of multiple phosphorylated sites in breast tumours (S104/106, S118, S167, S282, S294, T311, and S559(14)).

When estrogen binds to ERα, the receptor undergoes dimerisation; subsequently ERα binds to estrogen-responsive elements (EREs) in the promoter region of target genes or interacts with other transcription factor complexes thereby regulating transcription of genes involved in cell proliferation and differentiation (8,15). In a similar way, the regulation of ERα transcription is controlled by other transcription factors, namely GATA3 (16), FoxM1 (17) and FoxO3a (18-20), that bind to specific elements within *ESR1* and allowing RNA polymerase II recruitment to ERα promoters.

Lemur Tyrosine Kinase (LMTK) represent a group within the superfamily of tyrosine kinases that is able to phosphorylate serine, threonine and tyrosine residues (21). So far, 3 different isoforms have been described: i) LMTK1, also known as apoptosis-associated tyrosine kinase (AATYK; LMR1) is mainly expressed in the nervous system and appears to be involved in neurite extension and apoptosis (22), ii) LMTK2, also known as brain-enriched kinase (BREK; AATYK2; LMR2), is a myosin VI-binding protein found to be important in endosomal membrane trafficking (23) and spermatogenesis in mice (24) and iii) LMTK3 (LMR3; TYKLM3). An RNAi screen of primary leukemic cells found that cells from a patient with JAK2-positive chronic neutrophilic leukemia were sensitive to downregulation of LMTK3 (25). Another study showed that LMTK3 is associated with the Wnt pathway which is known to play a pivotal role in colon cancer and inhibition of LMTK3 specifically reduced β-catenin-dependent transcription (26). However, until now no reports or linkage of LMTK3, ERα and BC (or, for example, hepatocellular, bladder or gastric cancer) has been suggested or described.

We have specifically identified LMTK3 as a novel kinase that is able to down-regulate the transcriptional activity of ERα. We show that silencing of LMTK3 results in decreased ERα mRNA and protein levels, and that FoxO3a is implicated in this mechanism. We also show that LMTK3 is over-expressed in cancer, particularly BC (and, for example, in hepatocellular, bladder and gastric cancer) and represents a prognostic and predictive factor in cancer, particularly BC (and, for example, hepatocellular, bladder and gastric cancer) and a therapeutic target for cancer, particularly BC (and, for example, hepatocellular, bladder and gastric cancer). We also show that there are statistically significant associations between LMTK3 polymorphisms and the most relevant measures of disease progression, namely progression free survival and overall survival. We also show that LMTK3 phosphorylates both ERα and FoxO3a. Inhibition of LMTK3 is shown herein to overcome resistance to endocrine therapy in breast cancer cell lines.

The listing or discussion of an apparently prior-published document in this specification should not necessarily be taken as an acknowledgement that the document is part of the state of the art or is common general knowledge.

Naik et al (2009) Clin Cancer Res 15(24), 7529-7537 and supporting data notes that the Vascular Endothelial Growth Factor Receptor-1 is synthetic lethal to aberrant β-Catenin activation in colon cancer.

Kasamatsu et al ((2005) Int J Biochem Cell Biol 37(9), 1869-1880 identifies candidate genes associated with salivary adenoid cystic carcinomas using combined comparative genomic hybridization and oligonucleotide microarray analyses.

Greenman et al (2010) Biostatistics 11(1), 164-175 describes PICNIC: an algorithm to predict absolute allelic copy number variation with microarray cancer data, and refers to the affymetrix genome-wide SNP 6.0 array.

WO 2004/091511 relates to compositions and methods to diagnose and treat lung cancer, and methods of screening for a potential therapeutic agents for the reversal of the neoplastic condition.

Ali et al (2002) Nature Reviews Cancer 2(2), 102-112 reviews endocrine-responsive breast cancer and strategies for combating resistance.

A first aspect of the invention provides a method for aiding in determining prognosis in a patient with breast cancer, or in selecting a therapeutic strategy for a patient with breast cancer, the method comprising the step of assessing the level of LMTK3 nucleic acid, protein or activity in a sample obtained from the patient. The method may further comprise the step of selecting a treatment regime making use of the information on the level of LMTK3 nucleic acid, protein or activity in the sample.

The method may further comprise the step of assessing the level of ERα nucleic acid, protein or activity in a sample obtained from the patient
In one disclosure the method may further or alternatively comprise the step of assessing the patient's genotype for LMTK3 at position rs8108419 (in intron 2 of the LMTK3 gene) and/or position rs9989661 (in intron 15 of the LMTK3 gene). As noted in the Examples we correlated the presence of these polymorphisms with overall survival.

The method of the invention may further comprise the step of selecting a treatment regime making use of the information on the level of LMTK3 nucleic acid, protein or activity in the sample. Methods of assessing a patient's genotype will be well known to those skilled in the art. Typically, the genotype for LMTK3 is determined on a sample obtained from the patient. The sample need not be a sample thought to contain cancer cells: any sample that contains genomic DNA in sufficient quantity to be assessed by the chosen method may be used. Typically, polymerase chain reaction (PCR) methods may be used, for example PCR restriction fragment length polymorphism (PCR-RFLP) methods. Examples of suitable methods are described in Example 3, for example. Examples of suitable amplification primers for each polymorphic site are also given in Example 3.

It will be appreciated that determining the level of LMTK3 and optionally ERα nucleic acid, protein or activity in the sample may in itself allow determining prognosis in a patient with breast cancer, or selection of a therapeutic strategy for a patient with breast cancer; or more typically it may be used by the clinician as an aid in categorising or determining prognosis or selection of a therapeutic strategy.

For example, in relation to breast cancer, it is useful if the clinician undertakes a histopathological examination of biopsy tissue or carries out external digital examination or carries out imaging. Clinical examination of breast cancer is done currently through morphological assessment of cells removed in a needle aspirate or core biopsy and also by mammography. Mammography is also dependent on morphological changes on the mammogram. There is currently no biochemical assessment which is used routinely to distinguish between cancer and non cancer in relation to breast cancer. Screening tests mentioned relating to BRCA1 and BRCA2 may be used. It will be appreciated that the clinician will wish to take in to account these or other factors, as well as consider the level of LMTK3, before categorising or determining prognosis or selection of a therapeutic strategy.

Determination of the level of LMKT3 in the sample will be useful to the clinician in determining how to manage the cancer in the patient. For example, since elevated levels of LMKT3 are considered to be associated with relapse, the clinician may use the information concerning the level of LMTK3 to facilitate decision making regarding treatment of the patient. Thus, if the level of LMTK3 is not elevated and is therefore indicative of a lower probability of relapse of the breast cancer, unnecessary treatments may be avoided. Similarly, if the level of LMTK3 is elevated and therefore indicative of a higher probability of relapse of the breast cancer, combination therapies may be the preferred treatment. Even if it is not appropriate to alter the type of therapy carried out, determining whether the level of LMTK3 is indicative of a higher probability of relapse may help the clinician to decide whether the patient needs systemic treatment or not.

In some disclosures, determination of the patient's genotype for LMTK3 is also considered to be useful to the clinician in determining how to manage the cancer in the patient. Since LMTK3 rs8108419 AA or LMTK3 rs9989661 CT or CC are associated with reduced survival, more aggressive treatment or more frequent monitoring may be chosen if a patient has one or more of these genotypes. Thus, combination therapies may be the preferred treatment; and/or systemic treatment may be appropriate. If the patient has LMTK3 rs8108419 GG or GA or LMTK3 rs9989661 TT then this suggests a lower probability of relapse of the breast cancer. For such patients, unnecessary treatments may be avoided.

At present, a major aim in oncology is to be able to distinguish those breast cancers with a higher probability of relapse from those with a low probability of relapse, because those with a low probability of relapse should not need to be put through six months of very toxic chemotherapy treatment in the early setting.

It is preferred if the nucleic acid is derived from a sample of the tissue in which cancer is suspected or in which cancer may be or has been found. For example, it is preferred if the sample containing nucleic acid is derived from the breast (including armpit tissue, for example lymph node tissue) of the patient. Samples of breast may be obtained by surgical excision, "true cut" biopsies, needle biopsy, nipple aspirate, aspiration of a lump or image-guided biopsy. The image may be generated by X-ray, ultrasound or (less preferably) technetium-99-labelled antibodies or antibody fragments which bind or locate selectively at the breast. Magnetic resonance imaging (MRI) may be used to distinguish fibrosis from breast cancer. The sample may also be biopsy or needle aspirate of a suspected metastatic site such as the liver or lungs or bones.

The sample may be directly derived from the patient, for example, by biopsy of the tissue, or it may be derived from the patient from a site remote from the tissue, for example because cells from the tissue have migrated from the tissue to other parts of the body. Alternatively, the sample may be indirectly derived from the patient in the sense that, for example, the tissue or cells therefrom may be cultivated *in vitro,* or cultivated in a xenograft model; or the nucleic acid sample may be one which has been replicated (whether *in vitro* or *in vivo*) from nucleic acid from the original source from the patient. Thus, although the nucleic acid derived from the patient may have been physically within the patient, it may alternatively have been copied from nucleic acid which was physically within the patient. The tumour tissue may be taken from the primary tumour or from metastases. The sample may be lymph nodes, lymph or blood and the spread of disease detected.

It will be appreciated that the aforementioned methods may be used for presymptomatic screening of a patient who is in a risk group for cancer. High risk patients for screening include patients over 50 years of age or patients who carry a gene resulting in increased susceptibility (eg predisposing versions of BRCA1, BRCA2 or p53); patients with a family history of breast/ovarian cancer; patients with affected siblings; nulliparous women; and women who have a long interval between menarche and menopause. Similarly, the methods may be used for the pathological classification of tumours such as breast tumours.

The level of LMTK3 which is indicative of a high probability of relapse may be defined as the increased level present in at least a subset of known cancerous breast cells (preferably epithelial cells but possibly also or alternatively other cell types such as neuroendocrine or myoepithelial cells) over known corresponding non-cancerous cells, for example non-cancerous breast cells. The level of LMTK3 protein may be, for example, at least 1½ fold higher in cancerous cells with a high probability of relapse, or it may be at least 2-fold or 3-fold higher. Quantitative analysis by immunohistochemically processed tissue sections may be used. It may be useful to assess separately nuclear and cytoplasmic LMTK3 protein, for example as described in the Examples. An antibody that is considered to react with LMTK3 may be used. Examples are described in the Examples, but the skilled person would be able to select other suitable antibodies. The level of LMTK3 protein may be, for example, at least visualised as being much higher in cancerous cells with a high probability of relapse than in cancerous cells with a low probability of relapse or in non-cancerous cells as measured by immunohistochemistry.

For example, nuclear LMTK3 protein levels may be expressed as weak, moderate or strong, for example as set out in the Examples. Cytoplasmic protein levels may be expressed as absent (0), weak (1), moderate (2) or strong (3). These may further be grouped as low (scores 0 and 1) and high (scores 2 and 3). Strong nuclear or high cytoplasmic LMTK3 protein levels are considered to indicate a higher probability of relapse. It may be helpful in relation to samples assessed as moderate to investigate further, for example using fluorescence in situ hibridisation or assessing mRNA levels, but this is not considered to be essential.

In another embodiment of the invention it is determined whether the level of LMTK3 nucleic acid, in particular mRNA, is a level associated with a higher probability of relapse.

Preferably, the sample contains nucleic acid, such as mRNA, and the level of LMTK3 is measured by contacting said nucleic acid with a nucleic acid which hybridises selectively to LMTK3 nucleic acid.

By "selectively hybridising" is meant that the nucleic acid has sufficient nucleotide sequence similarity with the said human nucleic acid that it can hybridise under moderately or highly stringent conditions. As is well known in the art, the stringency of nucleic acid hybridization depends on factors such as length of nucleic acid over which hybridisation occurs, degree of identity of the hybridizing sequences and on factors such as temperature, ionic strength and CG or AT content of the sequence. Thus, any nucleic acid which is capable of selectively hybridising as said is useful in the practice of the invention.

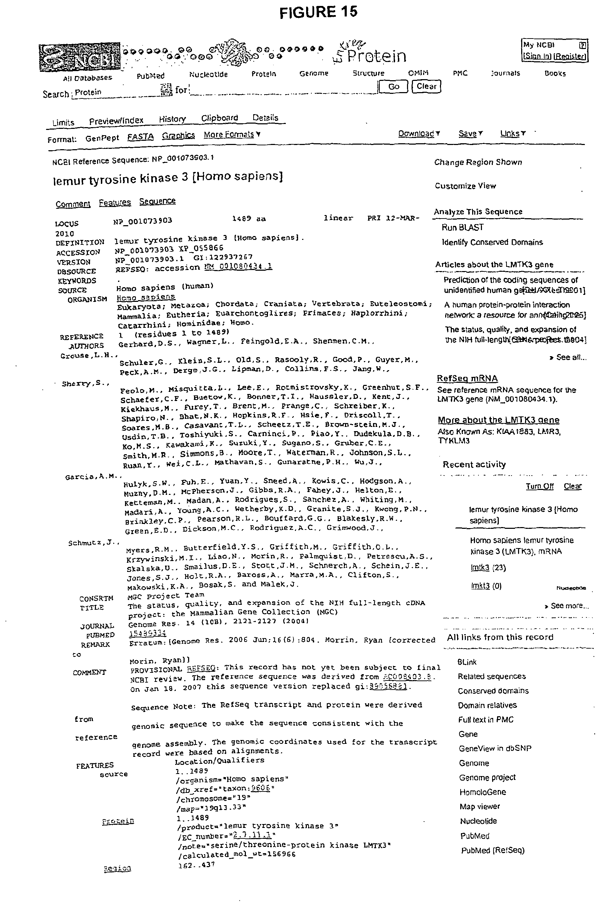

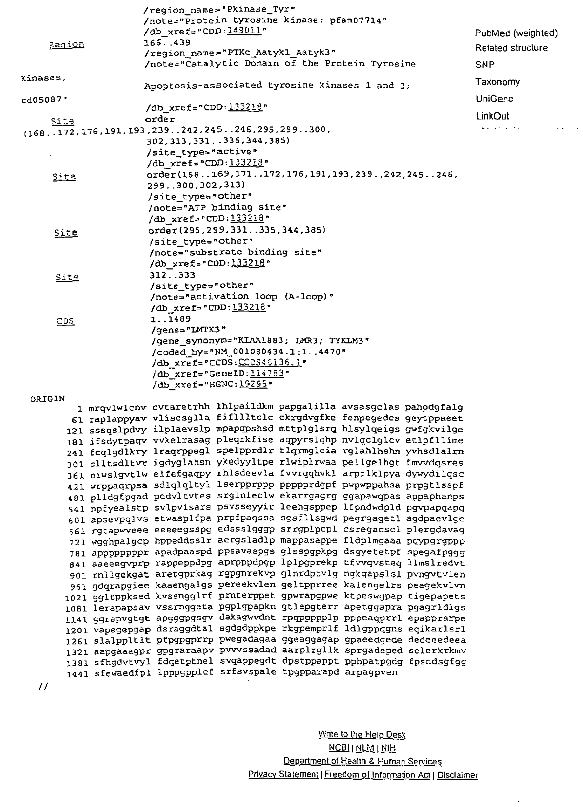

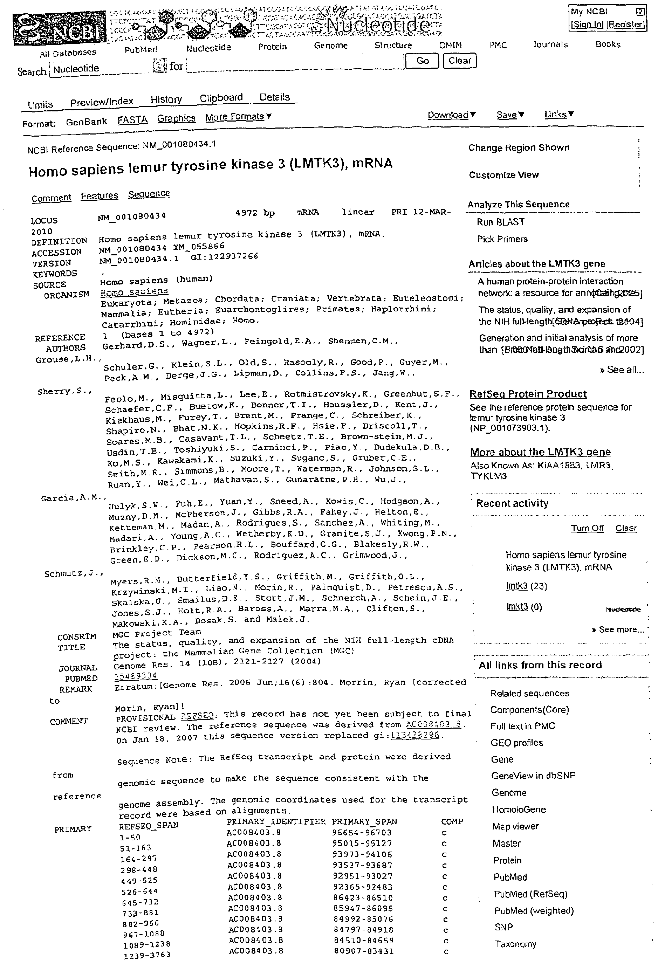

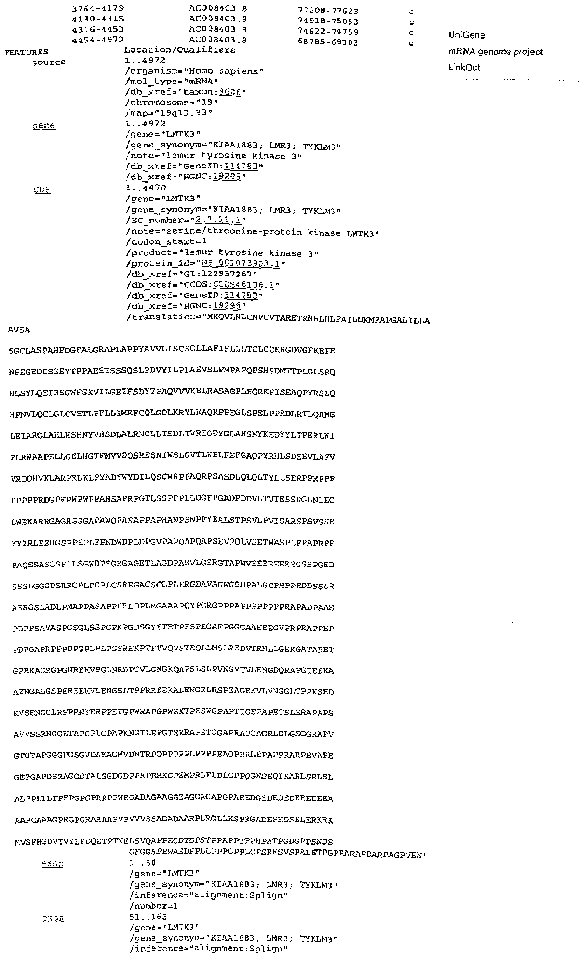

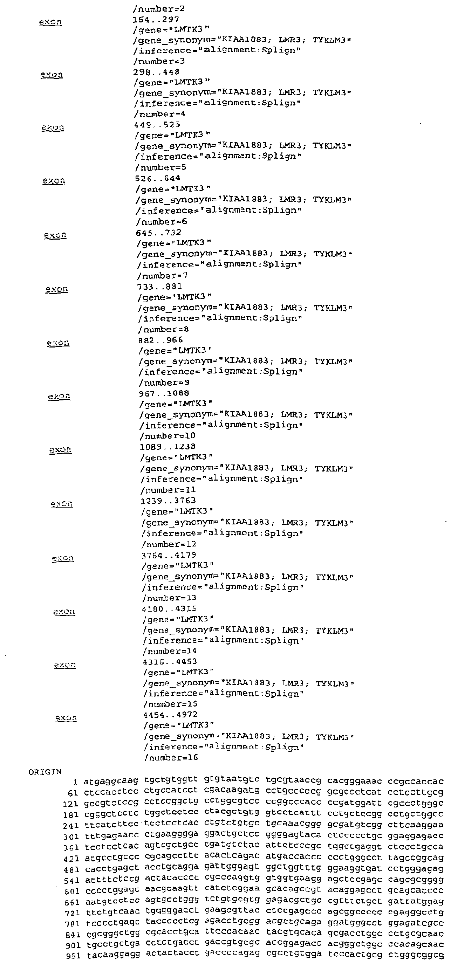

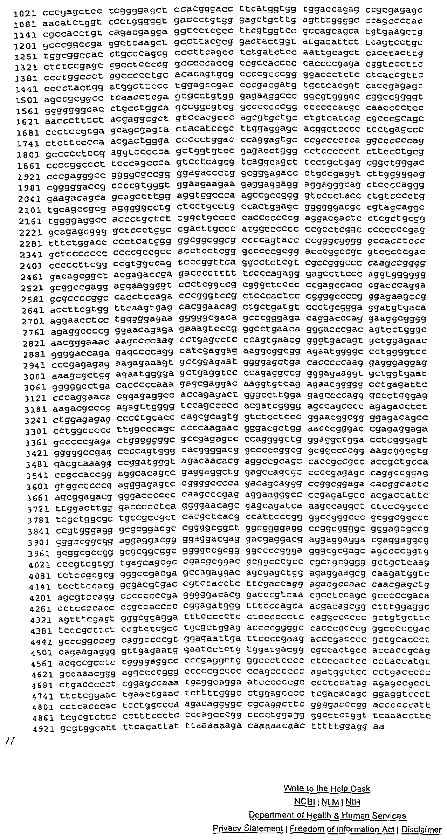

LMTK3 sequences are well known and may be found at, for example, Pubmed/ EMBL http://www.ncbi.nlm.nih.gov/nuccore/Genecards http://www.genecards.org/. Figure 15 shows the LMTK3 protein and nucleic acid sequence.

Nucleic acids which can selectively hybridise to the said human nucleic acid include nucleic acids which have >95% sequence identity, preferably those with >98%, more preferably those with >99% sequence identity, over at least a portion of the nucleic acid with the said human nucleic acid. As is well known, human genes usually contain introns such that, for example, a mRNA or cDNA derived from a gene would not match perfectly along its entire length with the said human genomic DNA but would nevertheless be a nucleic acid capable of selectively hybridising to the said human DNA. Thus, the invention specifically includes nucleic acids which selectively hybridise to said LMTK3 mRNA or cDNA but may not hybridise to a said LMTK3 gene. For example, nucleic acids which span the intron-exon boundaries of the said LMTK3 gene may not be able to selectively hybridise to the said LMTK3 mRNA or cDNA.

Typical moderately or highly stringent hybridisation conditions which lead to selective hybridisation are known in the art, for example those described in Molecular Cloning, a laboratory manual, 2nd edition, Sambrook et al (eds), Cold Spring Harbor Laboratory Press, Cold Spring Harbor, NY, USA.

An example of a typical hybridisation solution when a nucleic acid is immobilised on a nylon membrane and the probe nucleic acid is ∼ 500 bases or base pairs is:
6 x SSC (saline Na⁺ citrate)
0.5% Na⁺ dodecyl sulphate (SDS)
100 :g/ml denatured, fragmented salmon sperm DNA

The hybridisation is performed at 68 °C. The nylon membrane, with the nucleic acid immobilised, may be washed at 68°C in 1 x SSC or, for high stringency, 0.1 x SSC.

20 x SSC may be prepared in the following way. Dissolve 175.3 g of NaCl and 88.2 g of Na⁺ citrate in 800 ml of H₂O. Adjust the pH to 7.0 with a few drops of a 10 N solution of NaOH. Adjust the volume to 1 litre with H₂O. Dispense into aliquots. Sterilize by autoclaving.

An example of a typical hybridisation solution when a nucleic acid is immobilised on a nylon membrane and the probe is an oligonucleotide of between 15 and 50 bases is:
3.0 M trimethylammonium chloride (TMACI)
0.01 M Na⁺ phosphate (pH 6.8)
1 mm EDTA (pH 7.6)
0.5% SDS
100 :g/ml denatured, fragmented salmon sperm DNA
0.1 % nonfat dried milk

The optimal temperature for hybridization is usually chosen to be 5 °C below the Tᵢ for the given chain length. Tᵢ is the irreversible melting temperature of the hybrid formed between the probe and its target sequence. Jacobs et al (1988) Nucl. Acids Res. 16, 4637 discusses the determination of Tᵢs. The recommended hybridization temperature for 17-mers in 3 M TMACI is 48-50 °C; for 19-mers, it is 55-57 °C; and for 20-mers, it is 58-66 °C.

By "nucleic acid which selectively hybridises" is also included nucleic acids which will amplify DNA from the LMTK3 mRNA by any of the well known amplification systems such as those described in more detail below, in particular the polymerase chain reaction (PCR). Suitable conditions for PCR amplification include amplification in a suitable 1 x amplification buffer:
10 x amplification buffer is 500 mM KCI; 100 mM Tris.Cl (pH 8.3 at room temperature); 15 mM MgCl₂; 0.1 % gelatin.

A suitable denaturing agent or procedure (such as heating to 95°C) is used in order to separate the strands of double-stranded DNA.

Suitably, the annealing part of the amplification is between 37 °C and 60 °C, preferably 50 °C.

Although the nucleic acid which is useful in the methods of the invention may be RNA or DNA, DNA is preferred. Although the nucleic acid which is useful in the methods of the invention may be double-stranded or single-stranded, single-stranded nucleic acid is preferred under some circumstances such as in nucleic acid amplification reactions.

The nucleic acid which is useful in the methods of the invention may be any suitable size. However, for certain diagnostic, probing or amplifying purposes, it is preferred if the nucleic acid has fewer than 10 000, more preferably fewer than 1000, more preferably still from 10 to 100, and in further preference from 15 to 30 base pairs (if the nucleic acid is double-stranded) or bases (if the nucleic acid is single stranded). As is described more fully below, single-stranded DNA primers, suitable for use in a polymerase chain reaction, are particularly preferred.

The nucleic acid for use in the methods of the invention is a nucleic acid capable of hybridising to the LMTK3 mRNA or mRNAs. Fragments of the said LMTK3 gene and cDNAs derivable from the mRNA encoded by the said LMTK3 gene are also preferred nucleic acids for use in the methods of the invention.

It is particularly preferred if the nucleic acid for use in the methods of the invention is an oligonucleotide primer which can be used to amplify a portion of the said LMTK3 nucleic acid, particularly LMTK3 mRNA.

Preferred nucleic acids for use in the invention are those that selectively hybridise to the LMTK3 mRNA and do not hybridise to other LMTK mRNAs. Such selectively hybridising nucleic acids can be readily obtained, for example, by reference to whether or not they hybridise (or are predicted to hybridise, using well known calculations) to the said LMTK3 mRNA and not to other LMTK mRNAs.

Conveniently, the nucleic acid capable of hybridising to the LMTK3 mRNA and which is used in the methods of the invention further comprises a detectable label.

By "detectable label" is included any convenient radioactive label such as ³²P, ³³P or ³⁵S which can readily be incorporated into a nucleic acid molecule using well known methods; any convenient fluorescent or chemiluminescent label which can readily be incorporated into a nucleic acid is also included. In addition the term "detectable label" also includes a moiety which can be detected by virtue of binding to another moiety (such as biotin which can be detected by binding to streptavidin); and a moiety, such as an enzyme, which can be detected by virtue of its ability to convert a colourless compound into a coloured compound, or *vice versa* (for example, alkaline phosphatase can convert colourless o-nitrophenylphosphate into coloured *o*-nitrophenol). Conveniently, the nucleic acid probe may occupy a certain position in a fixed assay and whether the nucleic acid hybridises to the said LMTK3 nucleic acid can be determined by reference to the position of hybridisation in the fixed assay. The detectable label may also be a fluorophore-quencher pair as described in Tyagi & Kramer (1996) Nature Biotechnology 14, 303-308.

Other types of labels and tags are disclosed above. The nucleic acid may be branched nucleic acid (see Urdea et al (1991) Nucl. Acids Symposium Series 24, 197-200).

Conveniently, in the methods of the invention the nucleic acid which is capable of the said selective hybridisation (whether labelled with a detectable label or not) is contacted with nucleic acid (eg mRNA) derived from the patient under hybridising conditions. Suitable hybridising conditions include those described above.

The presence of a complex which is selectively formed by the nucleic acid hybridising to the LMTK3 mRNA may be detected, for example the complex may be a DNA:RNA hybrid which can be detected using antibodies. Alternatively, the complex formed upon hybridisation may be a substrate for an enzymatic reaction the product of which may be detected (suitable enzymes include polymerases, ligases and endonucleases).

It is preferred that if the sample containing nucleic acid (eg mRNA) derived from the patient is not a substantially pure sample of the tissue or cell type in question that the sample is enriched for the said tissue or cells. For example, enrichment for breast cells in a sample such as a blood sample may be achieved using, for example, cell sorting methods such as fluorescent activated cell sorting (FACS) using a breast cell-selective antibody, or at least an antibody which is selective for an epithelial cell. For example, anti-MUC1 antibodies such as HMFG-1 and HMFG-2 may be used (Taylor-Papadimitriou et al (1986) J. Exp. Pathol. 2, 247-260); other anti-MUC1 antibodies which may be useful are described in Cao et al (1998) Tumour Biol, 19, (Suppl 1), 88-99. The source of the said sample also includes biopsy material as discussed above and tumour samples, also including fixed paraffin mounted specimens as well as fresh or frozen tissue. The nucleic acid sample from the patient may be processed prior to contact with the nucleic acid which selectively hybridises to the LMTK3 mRNA. For example, the nucleic acid sample from the patient may be treated by selective amplification, reverse transcription, immobilisation (such as sequence specific immobilisation), or incorporation of a detectable marker.

Cells may be analysed individually, for example using single-cell immobilisation techniques. Methods by which single cells may be analysed include methods in which the technique of Laser Capture Microdissection (LCM) is used. This technique may be used to collect single cells or homogeneous cell populations for molecular analysis and is described in, for example, Jin et al (1999) Lab Invest 79(4), 511-512; Simone et al (1998) Trends Genet 14(7), 272-276; Luo et al (1999) Nature Med 5(1), 117-122; Arcuturs Updates, for example June 1999 and February 1999; US 5,859,699. The cells of interest are visualised, for example by immunohistochemical techniques, and transferred to a polymer film that is activated by laser pulses. The technique may also be used for isolation of cells which are negative for a particular component. Microscopes useful in performing LCM are manufactured by Arcturus Engineering, Inc., 1220 Terra Bella Avenue, Mountain View, CA 94042, USA.

LCM may be used with other isolation or enrichment methods. For example, LCM may be used following a method which enriches the sample for the target cell type.

It will be appreciated that the LMTK3 mRNA may be identified by reverse-transcriptase polymerase chain reaction (RT-PCR) using methods well known in the art.

Any of the nucleic acid amplification protocols can be used in the method of the invention including the polymerase chain reaction, QB replicase and ligase chain reaction. Also, NASBA (nucleic acid sequence based amplification), also called 3SR, can be used as described in Compton (1991) Nature 350, 91-92 and AIDS (1993), Vol 7 (Suppl 2), S108 or SDA (strand displacement amplification) can be used as described in Walker et al (1992) Nucl. Acids Res. 20, 1691-1696. The polymerase chain reaction is particularly preferred because of its simplicity.

Other methods of detecting mRNA levels are included.

Methods for determining the relative amount of the said LMTK3 mRNA include: *in situ* hybridisation (In Situ Hybridization Protocols. Methods in Molecular Biology Volume 33. Edited by K H A Choo. 1994, Humana Press Inc (Totowa, NJ, USA) pp 480p and In Situ Hybridization: A Practical Approach. Edited by D G Wilkinson. 1992, Oxford University Press, Oxford, pp 163), *in situ* amplification, northerns, nuclease protection, probe arrays, and amplification based systems;

The mRNA may be amplified prior to or during detection and quantitation. 'Real time' amplification methods wherein the product is measured for each amplification cycle may be particularly useful (eg Real time PCR Hid et al (1996) Genome Research 6, 986-994, Gibson et al (1996) Genome Research 6, 995-1001; Real time NASBA Oehlenschlager et al (1996 Nov 12) PNAS (USA) 93(23), 12811-6. Primers should be designed to preferentially amplify from an mRNA template rather than from the DNA, or be designed to create a product where the mRNA or DNA template origin can be distinguished by size or by probing. NASBA may be particularly useful as the process can be arranged such that only RNA is recognised as an initial substrate.

Detecting mRNA includes detecting mRNA in any context, or detecting that there are cells present which contain mRNA (for example, by *in situ* hybridisation, or in samples obtained from lysed cells). It is useful to detect the presence of mRNA or that certain cells are present (either generally or in a specific location) which can be detected by virtue of their expression of the said LMTK3 mRNA. As noted, the presence *versus* absence of the said LMTK3 mRNA may be a useful marker, or low levels versus high levels of the said LMTK3 mRNA may be a useful marker, or specific quantified levels may be associated with a specific disease state. It will be appreciated that similar possibilities exist in relation to using the said LMTK3 polypeptide as a marker.

In a further preferred embodiment, the level of said LMTK3 protein is measured. Preferably, the level of said protein is measured by contacting the protein with a molecule which selectively binds to said LMKT3 polypeptide.

The sample containing protein derived from the patient is conveniently a sample tissue. It may be useful to measure the elevated level (tumour) versus lower level (normal) of the said LMTK3 polypeptide in some circumstances, such as when assessing breast tissue. The methods of the invention also include the measurement and detection of the said LMTK3 polypeptide in test samples and their comparison in a control sample.

The sample containing protein derived from the patient is conveniently a sample of the tissue in which cancer is suspected or in which cancer may be or has been found. Methods of obtaining suitable samples are described in relation to earlier methods and will be apparent to the skilled person. For example the sample may be any one of needle biopsy, core biopsy, nipple or lymph node aspirate. The sample may also be lymph node-derived material which may be particularly useful in determining whether a cancer has spread. Single cells may be analysed, as noted above.

The methods of the invention involving detection of LMTK3 protein are particularly useful in relation to historical samples such as those containing paraffin-embedded sections of tumour samples.

The level of LMTK3 protein may be determined in a sample in any suitable way.

It is particularly preferred if the molecule which selectively binds to the said LMTK3 is an antibody. Examples of such antibodies will be well known to those skilled in the art. Some examples are given in the Examples. For example, a suitable anti-LMTK3 mouse monoclonal antibody may be obtained from Santa Cruz, UK.

The antibodies may be monoclonal or polyclonal. Suitable antibodies are available commercially or may be prepared by known techniques, for example those disclosed in "Monoclonal Antibodies: A manual of techniques", H Zola (CRC Press, 1988) and in "Monoclonal Hybridoma Antibodies: Techniques and applications", J G R Hurrell (CRC Press, 1982). Other techniques for raising and purifying antibodies are well known in the art and any such techniques may be chosen to achieve the preparations useful in the methods claimed in this invention. In a preferred embodiment of the invention, antibodies will immunoprecipitate LMTK3 proteins from solution as well as react with LMKT3 protein on western or immunoblots of polyacrylamide gels. In another preferred embodiment, antibodies will detect LMTK3 proteins in paraffin or frozen tissue sections, using immunocytochemical techniques.

Preferred embodiments relating to methods for detecting LMTK3 protein include enzyme linked immunosorbent assays (ELISA), radioimmunoassay (RIA), immunoradiometric assays (IRMA) and immunoenzymatic assays (IEMA), including sandwich assays using monoclonal and/or polyclonal antibodies. Exemplary sandwich assays are described by David et al in US Patent Nos. 4,376,110 and 4,486,530.

It will be appreciated that other antibody-like molecules may be used in the method of the inventions including, for example, antibody fragments or derivatives which retain their antigen-binding sites, synthetic antibody-like molecules such as single-chain Fv fragments (ScFv) and domain antibodies (dAbs), and other molecules with antibody-like antigen binding motifs.

The level of LMTK3 which is indicative of a higher probability of relapse may be defined as the increased level present in at least a subset of known cancerous breast cells, preferably cancerous breast cells with a higher probability of relapse over known non-cancerous or lower probability of relapse corresponding cells, for example breast cells, as discussed above. Typically the cells may be breast epithelial cells, as discussed above. The level may be, for example, at least 1½ fold higher in cancerous cells or cells with a higher probability of relapse, or it may be at least 2-fold or 3-fold higher. The level of LMTK3 protein may be, for example, at least visualised as being much higher in cancerous cells with a high probability of relapse than in cancerous cells with a low probability of relapse or in non-cancerous cells as measured by immunohistochemistry, for example as described above and in the Examples.

By "the relative amount of LMTK3 protein" is meant the amount of said LMTK3 protein per unit mass of sample tissue or per unit number of sample cells compared to the amount of said LMTK3 protein per unit mass of known normal tissue or per unit number of normal cells. The relative amount may be determined using any suitable protein quantitation method. In particular, it is preferred if antibodies are used and that the amount of said LMTK3 protein is determined using methods which include quantitative western blotting, enzyme-linked immunosorbent assays (ELISA) or quantitative immunohistochemistry.

As noted above, an increased level of the said LMTK3 in a sample compared with a known normal tissue sample is suggestive of a tumorigenic sample with high relapse potential. In relation to breast tissue, the presence of elevated LMTK3 levels, compared to its absence, is suggestive of more aggressive carcinogenesis.

In a further embodiment the level of LMTK3 is measured by selectively assaying its activity in the sample. The activity of LMTK3 in a sample may be assayed by measuring phosphorylation/activity of a substrate of LMTK3, for example ERα. Phosphorylation/ activity of ERα may be measured using a reporter gene system, as will be well known to those skilled in the art. For example, expression of ERα-regulated genes can be assessed, for example pS2, PgR and GREB1, for example as described in the Examples.

Methods of the invention may make use of samples obtained by what can broadly be described as "invasive" methods or by "non-invasive" methods. Invasive methods include, for example, the taking of a biopsy for detection of LMTK3 level by, for example, (a) immunohistochemical application of an LMTK3-specific antibody, (b) *in situ* PCR on tissue sections, or (c) reverse transcription (RT)-PCR of cells, for example epithelial cells (and/or other cell types, for example neuroendocrine or myoepithelial cells) after separating them from the biopsy. Non-invasive methods include obtaining breast-derived cells from blood, which may be separated by affinity and assayed for LMTK3 level by PCR.

One disclosure provides a kit of parts useful for assessing breast cancer comprising (1) an agent which is specifically capable of use in determining the level of LMTK3 protein or nucleic acid in a sample, and either (2) means for separating breast cells from a sample or identifying breast cells in a sample in order to carry out said LMTK3 assay; and/or (3) an agent which is specifically capable of use in determining the level of ERα protein, nucleic acid or activity in a sample. Optionally the kit may include an agent or agents specifically capable of use in determining LMTK3 genotype at position LMTK3 rs8108419 and/or LMTK3 rs9989661. Thus the kit may contain PCR primers suitable for use in determining LMTK3 genotype at position LMTK3 rs8108419 and/or LMTK3 rs9989661. Examples of such primers include forward primer 5'-ATTCCACCACTCCCTCCAG-3' and reverse primer 5'-GACCCTGCAGTGCCTC AC-3' for rs8108419 and forward primer 5'-GGGCCTTCCCAAGTGGTT-3' and reverse primer 5'-ATCCAAGCCTGGGGTGAG-3' for rs9989661. These primers may be used for PCR amplification, typically followed by digestion of the PCR products by restriction enzyme BsrD1(rs8108419) or Btsc1(rs9989661)(New England Biolab, Massachusetts, USA), and separation of alleles by gel electrophoresis, for example on 4% NuSieve ethidium bromide-stained agarose gel.

The agent which is specifically capable of use in determining the level of LMTK3 protein or nucleic acid in a sample may be a nucleic acid which selectively hybridises to the LMTK3 nucleic acid or the agent may be a molecule which selectively binds to the LMTK3 protein.

Preferably, the kit further comprises a control sample containing LMTK3 nucleic acid or protein wherein the control sample may be a negative control (which contains a level of LMTK3 protein or nucleic acid which is not associated with breast cancer or a high probability of recurrence of breast cancer) or it may be a positive control (which contains a level of LMTK3 protein or nucleic acid which is associated with breast cancer or a high probability of recurrence of breast cancer). The kit may contain both negative and positive controls. The kit may usefully contain controls of the LMTK3 protein or nucleic acid which correspond to different amounts such that a calibration curve may be made.

Suitably, the kit further comprises means for separating breast cells (for example epithelial cells, neuroendocrine or myoepithelial cells) from a sample or identifying breast cells in a sample in order to carry out said LMTK3 assay. Preferably, the means for separating breast cells includes antibody-coated micro-beads or columns. These are coated with antibodies to cell membrane proteins. For example, as noted above, anti-MUC1 antibodies such as HMFG-1 and HMFG-2 may be used (Taylor-Papadimitriou et al (1986) J. Exp. Pathol. 2, 247-260); other anti-MUC1 antibodies which may be useful are described in Cao et al (1998) Tumour Biol. 19, (Suppl 1), 88-99. However, anti-MUC1 antibodies may bind to normal bone marrow cells. It is preferred to use an anti epithelial cell adhesion molecule antibody, preferably coated on magnetic beads. A preferred antibody is termed BER-EP-4.

A further disclosure provides a kit of parts useful for diagnosing breast cancer, comprising (1) an agent which is specifically capable of use in determining the level of
LMTK3 protein or nucleic acid in a sample, and (2) means for separating breast cells (for example epithelial cells, neuroendocrine or myoepithelial cells) from a sample or identifying breast cells in a sample in order to carry out said LMTK3 assay.
The kits may usefully further comprise a component for testing for a further cancer-related polypeptide such as antibodies which are reactive with one or more of the following cancer-related polypeptides, all of which are well known in the art: MAGE-1, MAGE-3, BAGE, GAGE-1, CAG-3, CEA, p53, oestrogen receptor (ER), progesterone receptor (PR), MUC1, p52 trefoil peptide, Her2, PCNA, Ki67, cyclin D, p90^{rak3}, p170 glycoprotein (mdr-1) CA-15-3, c-erbB1, cathepsin D, PSA, CA125, CA19-9, PAP, myc, cytokeratins, bcl-2, telomerase, glutathione S transferases, rad51, VEGF, thymidine phosphorylase, Flk1 or Flk2.
The kit may usefully still further or alternatively comprise a nucleic acid which selectively hybridises to a further cancer-related nucleic acid such as a gene or mRNA which encodes any of the cancer-related polypeptides as described above. In addition, useful nucleic acids which may be included in the kit are those which selectively hybridise with the genes or mRNAs: ras, APC, BRCA1, BRCA2, ataxia telangiectasia (ATM), hMSH2, hMCH1, hPMS2 or hPMS1. It is preferred if the further nucleic acid is one which selectively hybridises to the gene or mRNA of any of erbB2, p53, BRCA1, BRCA2 or ATM. Optionally the kit may further include an agent or agents specifically capable of use in determining LMTK3 genotype at position LMTK3 rs8108419 and/or LMTK3 rs9989661, as discussed further above.
ERα, for example, may suitably be assessed by standard Alred or immunohistochemical scoring, as will be well known to those skilled in the art.
The kits usefully may contain controls and detection material, (for example, for immunohistochemistry, secondary antibodies labelled fluorophores, or enzymes, or biotin, or digoxygenin or the like). For immunoassays, additional components to the kit may include a second antibody to a different epitope on the LMTK3 (optionally labelled or attached to a support), secondary antibodies (optionally labelled or attached to a support), and dilution and reaction buffers. Similar additional components may usefully be included in all of the kits of the disclosure. A further aspect of the disclosure provides an agent which is specifically capable of use in determining the level of LMTK3 protein, nucleic acid or activity in a sample for determining prognosis in a patient with breast cancer or for selecting a therapeutic strategy for a patient with breast cancer.

A further aspect of the disclosure provides an agent which is specifically capable of use in determining LMTK3 genotype at position LMTK3 rs8108419 and/or LMTK3 rs9989661, for determining prognosis in a patient with breast cancer or for selecting a therapeutic strategy for a patient with breast cancer.
A further disclosure provides the use of an agent which is specifically capable of use in determining the level of LMTK3 protein, nucleic acid or activity in a sample, and/or an agent which is specifically capable of use in determining LMTK3 genotype at position LMTK3 rs8108419 and/or LMTK3 rs9989661 (as discussed above), in the manufacture of a medicament for determining prognosis in a patient with breast cancer or selecting a therapeutic strategy for a patient with breast cancer. The agent may be a nucleic acid (or nucleic acids) which selectively hybridises to LMTK3 nucleic acid. Alternatively the agent may be a molecule which selectively binds to LMTK3 protein, for example an antibody or similar molecule, as discussed above.
If the level of LMTK3 nucleic acid, protein or activity in the sample is an elevated level and/or the genotype is LMTK3 rs8108419AA or LMTK3 rs9989661 CT or CC then the selected treatment regime may incorporate a treatment regime that is considered to be an aggressive treatment regime. This may include one or more of surgery, chemotherapy, radiotherapy, hormonal therapy as well as therapy directed against LMTK3.
If the level of LMTK3 nucleic acid, protein or activity in the sample is an elevated level and/or the genotype is LMTK3 rs8108419AA or LMTK3 rs9989661 CT or CC then the selected treatment regime may comprise treating the patient with an inhibitor of LMTK3 activity. The inhibitor of LMTK3 activity may, for example, be an siRNA molecule or an antibody, antibody fragment or antibody-type molecule directed against LMTK3, as will be well known to those skilled in the art. It is considered that genotype LMTK3 rs8108419AA or LMTK3 rs9989661 CT or CC may result in increased expression levels of LMTK3 and therefore treatment with an inhibitor of LMTK3 activity is considered to be beneficial.

If the level of LMTK3 nucleic acid, protein or activity in the sample is not elevated and/or the genotype is LMTK3 rs8108419GA or GG or LMTK3 rs9989661 TT then the selected treatment regime may be a less aggressive treatment regime. Typically the patient would be managed with best standard of care according to standardised criteria (www.asco.org). Examples may include watchful waiting or adjuvant hormonal treatment including tamoxifen, GnRH analogues and aromatase inhibitors such as anastrazole, letrozole and exemestane.

The majority of patients with breast cancer present with localised disease, also known as primary breast cancer. The usual treatment is surgical excision of the tumour, followed by adjuvant therapy. Adjuvant therapies for ERα-positive disease are designed to reduce oestrogen levels or block its activity by binding to the receptor, as exemplified by tamoxifen. Tamoxifen is now the first line adjuvant treatment for ERα-positive disease in pre- and post-menopausal women and is beneficial in the treatment of metastatic disease, as well as localized disease. However, approximately 30%, of patients with ERα-positive disease do not respond to tamoxifen. Moreover, a substantial proportion of patients presenting with localized disease and all patients presenting with metastatic disease that initially respond to tamoxifen treatment become resistant. In the case of patients who initially respond but become resistant to tamoxifen ERα expression is lost in only ∼10% of cases. Moreover, one-third of resistant patients show a clinical response to treatment with a different anti-estrogen such as Faslodex (ICI 182, 780) or the use of aromatase inhibitors, drugs that inhibit estrogen synthesis. Nevertheless and despite issues of de novo or acquired resistance to tamoxifen, it has become the adjuvant agent of first choice (Ali and Coombes (2002) Nature Rev. Cancer 2: 101-112). Assessing LMTK3 adds to the information available to the clinician in assessing the likelihood of relapse. It also enables the clinician to assess whether treatment with a molecule that reduces LMTK3 activity is likely to be beneficial for that patient.

A further aspect of the invention provides a method for aiding in selecting a therapeutic strategy for a patient with breast cancer who is receiving endocrine therapy, or a patient who has previously received or is receiving endocrine therapy and has relapsed, the method comprising the steps of (i) assessing the level of LMTK3 nucleic acid, protein or activity in a sample obtained from the patient and (ii) assessing the endocrine resistance status of the patient.

By "endocrine resistance status" we include the meaning of a measure of the level of resistance to endocrine therapy that that patient's cancer cells may demonstrate. It is considered that this, in combination with a measure of LMTK3 activity may usefully inform treatment. The assessment of "endocrine resistance status" may be carried out for the first time in the patient as part of the method of the preceding aspect, or the endocrine resistance status may be implied from patient notes following past treatment of said patient with endocrine therapy, or possibly assessed previously by some other means. The assessment of the "endocrine resistance status" of a patient directly is not a clinically routine test and so it is unlikely to have been carried out previously for the patient. Nevertheless, the status of patients who have previously received endocrine therapy may possibly be identified from previous clinical outcomes.

In addition to or as an alternative to assessing their history of unresponsiveness to endocrine therapy, the "endocrine resistance status" of a patient may be assessed by a variety of means, for example, the level of tamoxifen resistance, or resistance to a GnRH analogue or an aromatase inhibitor such as anastrazole, letrozole or exemestane demonstrated in a sample from said patient may be assessed. This may be carried out by an *in vitro* assay. The endocrine resistance status may further be implied from a history of unresponsiveness to endocrine therapy. Additionally, or alternatively, the level of ERα phosphorylation in a sample from the patient may be assessed by standard techniques known in the art. This may provide a measure of the likely responsiveness of cells in said sample to endocrine therapy. Further, the level of ERα nucleic acid, protein or activity may be assessed in a sample obtained from the patient to provide information on the "endocrine resistance status". This may provide a direct measure of the capability of cells in the sample to react to endocrine therapy. An absence of ERα, or phosphorylation of ERα, may indicate that the cells in the sample are, or may later develop into, endocrine resistant cells.

If (i) the level of LMTK3 nucleic acid, protein or activity in the sample is an elevated level; and (ii) the patient is assessed as having an elevated endocrine resistance status or being resistant to endocrine therapy, then typically the selected treatment regime may comprise treating the patient with an inhibitor of LMTK3 activity in combination with endocrine therapy, for example adjuvant hormonal treatment such as tamoxifen, a GnRH analogue or an aromatase inhibitor such as anastrazole, letrozole or exemestane.

The inventors have shown that inhibition of LMTK3 can overcome resistance to endocrine treatment, particularly Tamoxifen treatment: see the Examples. It is envisaged that in patients who are resistant to Tamoxifen, or any other endocrine therapy, then coadministration of an inhibitor of LMTK3 activity with Tamoxifen or other endocrine therapy as appropriate may restore sensitivity to the Tamoxifen or other endocrine therapy and lead to successful treatment of the cancer.

Thus, evaluating the LMTK3 status and endocrine resistance status of a patient with breast cancer will aid in informing the clinician how to arrive at an appropriate treatment regime for the patient. The LMTK3 status assessment may include assessing the level of activity of LMTK3 and assessing the genotype as explained above in relation to other aspects of the invention.

Further, it is not only those patients that are presently receiving endocrine therapy and who have developed resistance to said therapy that are considered to benefit from the invention of the preceding aspect. It is also envisaged that breast cancer patients who had previously received endocrine therapy and demonstrated endocrine resistance and who have now been assigned alternative treatments may benefit from the invention. A combination of LMTK3 inhibition and the endocrine therapy that they were previously resistant to may prove to be a more successful therapy than their current therapy. Further, breast cancer patients who previously demonstrated endocrine resistance and who have been in remission following alternative treatments and have now relapsed may benefit from the present invention. Indeed, patients in remission who did not demonstrate endocrine resistance but following relapse now demonstrate endocrine resistance are also considered likely to benefit. In all such patients, LMTK3 elevation and endocrine resistance typically suggests that they may benefit from therapy with LMTK3 inhibiting agents in combination with endocrine therapy.

The inhibitors of LMTK3 activity are as specified above in relation to other aspects of the invention. Examples of endocrine therapies intended to be included in this aspect include, but are not limited to, adjuvant hormonal treatment such as tamoxifen, a GnRH analogue and an aromatase inhibitor such as anastrazole, letrozole or exemestane. The LMTK3 inhibitor and endocrine therapy may be co-formulated or formulated separately as would be understood by the person skilled in the art. Such formulations would be prepared as appropriate according to standard pharmaceutical practice.

A further aspect of the invention provides a method for aiding in determining whether a patient with breast cancer has a relatively high or relatively low likelihood of disease free survival, the method comprising the step of assessing the level of LMTK3 nucleic acid or protein in a sample obtained from the patient and/or the step of assessing the patient's genotype for LMTK3 at position rs8108419 (in intron 2 of the LMTK3 gene) and/or position rs9989661 (in intron 15 of the LMTK3 gene). For example, a low LMTK3 level is considered to indicate a >90% chance of cure with standard therapy, and a high level is considered to indicate a <85% chance of cure. By "cure" is meant at least 10 year breast cancer free survival

A further aspect of the invention provides a method for treating a patient with breast cancer or for inhibiting breast cancer cell proliferation in a patient with breast cancer, the method comprising administering an inhibitor of LMTK3 activity to the patient. The inhibitor of LMTK3 activity may be, for example, an siRNA molecule or an antibody, antibody fragment or antibody-type molecule directed against LMTK3. The patient may be a patient in which the level of LMTK3 nucleic acid or protein in a sample from the patient has been determined to be elevated and/or the patient's genotype for LMTK3 has been determined to be LMTK3 rs8108419AA or LMTK3 rs9989661 CT or CC.

A further aspect of the invention provides an inhibitor of LMTK3 activity for use in treating a patient with breast cancer or for inhibiting breast cancer cell proliferation in a patient with breast cancer; optionally wherein the patient is a patient in which the level of LMTK3 nucleic acid or
protein in a sample from the patient has been determined to be elevated and/or the patient's genotype has been determined to be LMTK3 rs8108419AA or LMTK3 rs9989661 CT or CC.

A further aspect of the invention provides the use of an inhibitor of LMTK3 activity in the manufacture of a medicament for treating a patient with breast cancer or for inhibiting breast cancer cell proliferation in a patient with breast cancer; optionally wherein the patient is a patient in which the level of LMTK3 nucleic acid or protein in a sample from the patient has been determined to be elevated and/or the patient's genotype has been determined to be LMTK3 rs8108419AA or LMTK3 rs9989661 CT or CC.

The inhibitor of LMTK3 activity may be an inhibitor of LMTK3 expression, for example an anti LMTK3 siRNA molecule. Methods useful in designing, synthesising and delivering siRNA molecules are well know to those skilled in the art.

In a further embodiment, the patient may be administered an additional anti-cancer agent or treatment.

The additional anti-cancer agent or treatment, particularly when the cancer is breast cancer, may include, or further include, endocrine therapy, for example adjuvant hormonal treatment such as tamoxifen, a GnRH analogue or an aromatase inhibitor such as anastrazole, letrozole or exemestane. Administration of endocrine therapy in combination with an inhibitor of LMTK3 activity may increase the effectiveness of the endocrine therapy according to the present invention. Thus, a patient who was previously a poor responder to endocrine therapy or assessed as resistant to endocrine therapy may be treated effectively with the appropriate endocrine therapy following treatment with an inhibitor of LMTK3 activity. The inhibitors of LMTK3 activity may be as described herein in relation to other aspects of the invention.

Further, the additional anti-cancer agent or treatment may be any known anti-cancer agent or treatment, alone, or in combination with an endocrine therapy. Examples of known anti-cancer agents include: alkylating agents including nitrogen mustards such as mechlorethamine (HN₂), cyclophosphamide, ifosfamide, melphalan (L-sarcolysin) and chlorambucil; ethylenimines and methylmelamines such as hexamethylmelamine, thiotepa; alkyl sulphonates such as busulfan; nitrosoureas such as carmustine (BCNU), lomustine (CCNU), semustine (methyl-CCNU) and streptozocin (streptozotocin); and triazenes such as decarbazine (DTIC; dimethyltriazenoimidazole-carboxamide); Antimetabolites including folic acid analogues such as methotrexate (amethopterin); pyrimidine analogues such as fluorouracil (5-fluorouracil; 5-FU), floxuridine (fluorodeoxyuridine; FUdR) and cytarabine (cytosine arabinoside); and purine analogues and related inhibitors such as mercaptopurine (6-mercaptopurine; 6-MP), thioguanine (6-thioguanine; TG) and pentostatin (2-deoxycoformycin). Natural Products including vinca alkaloids such as vinblastine (VLB) and vincristine; epipodophyllotoxins such as etoposide and teniposide; antibiotics such as dactinomycin (actinomycin D), daunorubicin (daunomycin; rubidomycin), doxorubicin, bleomycin, plicamycin (mithramycin) and mitomycin (mitomycin C); enzymes such as L-asparaginase; and biological response modifiers such as interferon alphenomes. Miscellaneous agents including platinum coordination complexes such as cisplatin (*cis*-DDP) and carboplatin; anthracenedione such as mitoxantrone and anthracycline; substituted urea such as hydroxyurea; methyl hydrazine derivative such as procarbazine (N-methylhydrazine, MIH); and adrenocortical suppressant such as mitotane (*o,p*-DDD) and aminoglutethimide; taxol and analogues/derivatives; and hormone agonists/antagonists such as flutamide and tamoxifen.

A further aspect of the invention provides an inhibitor of LMTK3 activity for use in treating a breast cancer patient who is being treated with anti-oestrogen endocrine therapy.

A yet further aspect of the invention provides an anti-oestrogen endocrine therapeutic for use in treating a breast cancer patient who is being treated with an inhibitor of LMTK3 activity.

In an embodiment of the two immediately preceding aspects the patient may be additionally administered a further anti-cancer agent or treatment. The further anti-cancer agent or treatment may be any of those specified in relation to the other aspects of the invention.

A further aspect of the invention provides a screening method for identifying a compound likely to be useful in treating breast cancer, the method comprising the step of determining the effect of a test compound on LMTK3 nucleic acid, protein or activity level; and selecting a compound that reduces said level. Examples of assays suitable for
assessing LMTK3 nucleic acid or protein level will be well know to those skilled in the art. Such assays may, for example, make use of techniques for assessing LMTK3 nucleic acid or protein levels described herein in relation to assessing LMTK3 levels in a sample from a patient. Typically a nucleic acid that selectively hybridises to an LMTK3 RNA or a molecule (for example an antibody) that selectively binds to an LMTK3 polypeptide may be used. Examples of assays suitable for assessing LMTK3 activity will also be well known to those skilled in the art. For example LMTK3 activity may be assessed by using a reporter gene system based on the effect of LMTK3 on ERα, for example on ERα transcriptional activity on genes regulated by ERα, for example pS2, PgR and GREB1.

A further example of an assay that may be used to assess the level of LMTK3 activity or the effect of a test compound on LMTK3 activity includes assessing the kinase activity of LMTK3 on substrate ERα and/or FoxO3a and/or a peptide substrate such as the commercially available S1 substrate (see Example 7). For example, an assay such as that described in Example 4 may be used wherein recombinant LMTK3 kinase domain is used to transfer ³²P to the substrate ERα and/or FoxO3a under appropriate reaction conditions. This has been established using *in vitro* kinase assays using LMTK3 as a source of enzyme activity and ERα and/or FoxO3a as a source of substrate activity. Both GST- ERα and/or FoxO3a fusion proteins may be used, Phosphorylation of the substrate is detected by first separating the substrate on a polyacrylamide gel (SDS-PAGE) and transferring to a nitrocellulose membrane as would be understood by a skilled person, followed by detection of radio-labelled substrate by autoradiography. A test compound is identified as an inhibitor of LMTK3 activity if it reduces phosphorylation of the substrate when included in the assay at appropriate concentrations (i.e. where the effect may be considered to be due to specific action of the test compound and not non-specific action due to high concentrations of compound). Assays as described in Example 7 may also be used.

Suitable assay formats will be well know to those skilled in the art and may include HTRF (Homogeneous Time-Resolved Fluorescence) assays (for example as offered by Cisbio), Lanthascreen (Invitrogen) and KlnaseGLO (Promega). Screening assays which are capable of high throughput operation may be used. Focused kinase inhibitor compound libraries may be used to complete an initial compound screen. Thus, the test compound may be a compound already identified as a protein kinase inhibitor. These may be used to identify compound 'hits' (typically non-selective, low potency chemical start points). Available sequence knowledge, structural predictions and known kinase ligands may also be used to virtually screen commercially available compounds for novel compound hits. Hit compounds may be iteratively modified using classical medicinal chemistry strategies to derive a compound series which has optimal inhibitory potency against the target kinase, whilst limiting the effects on other important kinase families. This optimisation process is expected to lead to a candidate compound for testing in *in vivo* systems, for example BC xenograft studies in mice and hence as a potential pre-clinical candidate. Pre-clinical candidates will also require DMPK (Drug Metabolism and Pharmacokinetics), toxicology, safety and formulation studies prior to clinical testing, as will be well known to the skilled person.

Thus, the effect of the test compound may be determined *in vitro.* It may be determined in a cancer, for example breast cancer (or, for example, hepatocellular, bladder or gastric cancer)cell or cell line. A suitable cell line may be a breast cancer-derived cell line such as MCF-7. It may be determined in a primary cancer cell. By a "primary cancer cell" is meant a cancer cell derived directly from a patient, for example from a primary or secondary tumour. The term encompasses a cell of a short term culture (starting from a cell or cells removed from a patient), for example after culture for a limited period, for example of up to 20 weeks, but does not include a cell of an immortalised cell line. Alternatively it may be determined *in vivo* in a non-human test animal.

The invention is now described in more detail by reference to the following, non-limiting, Figures and Examples.

### Figure legends

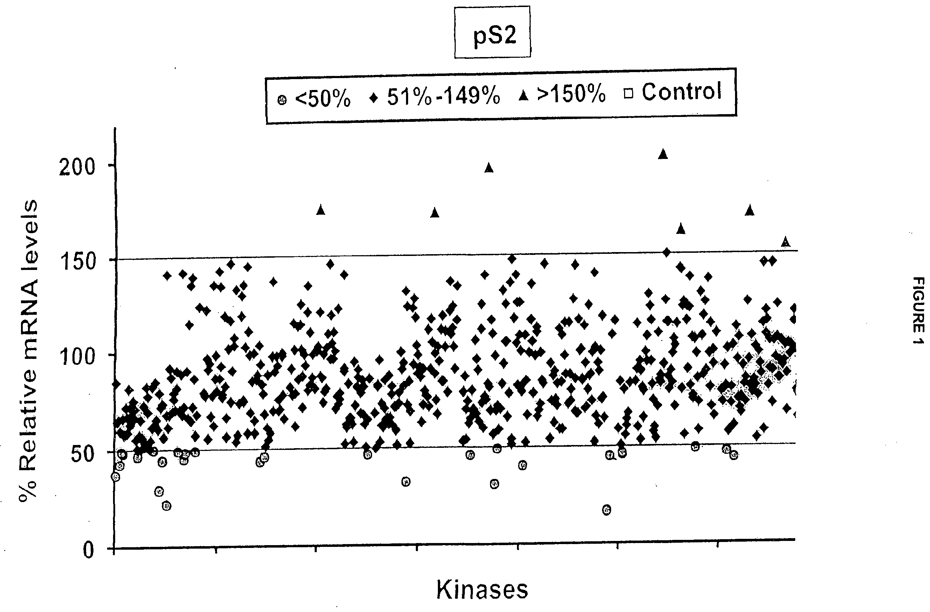

**Figure. 1** **High-throughput siRNA screen for kinases modulating ERα transcriptional activity**
   MCF7 cells plated in 24-well-plates were individually transfected for 48h with a pool of two different siRNAs per gene. Non-targeting siRNA controls were included in the screening (indicated in *yellow* squares). Gene expression of pS2 was measured by qRT-PCR after 24h of E2 treatment. The % relative mRNA levels of pS2 expression for each kinase is presented (100%= pS2 mRNA expression after E2 treatment alone). *Black triangles,* hits resulting in >50% up-regulation of pS2 expression after gene silencing; red *circles,* hits resulting in <50% down-regulation of pS2 expression after gene silencing; *blue diamonds,* hits resulting in 51-149% changes of pS2 expression after gene silencing. The screening has been done in duplicate (p < 0.05).

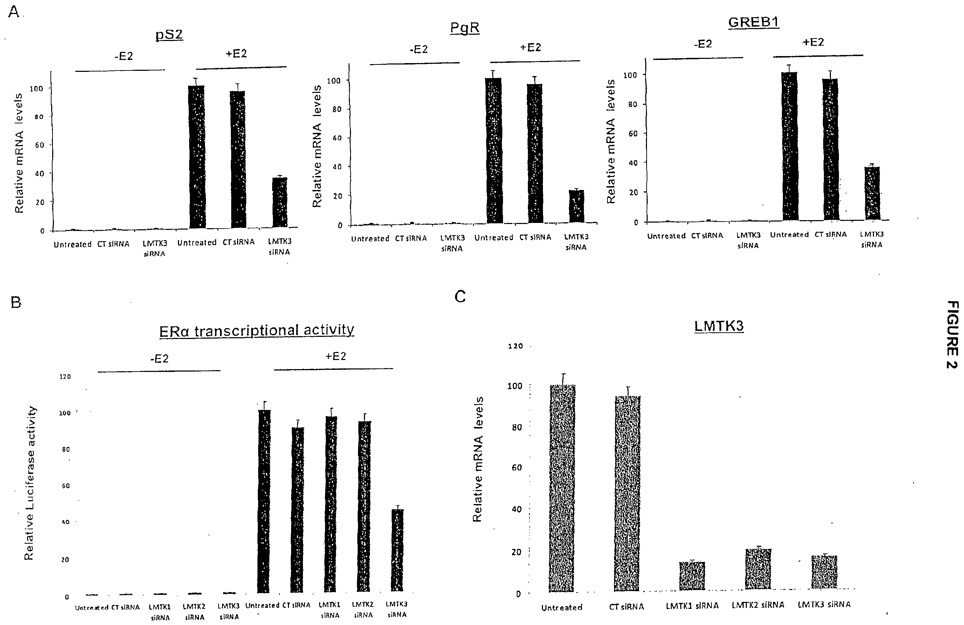

**Figure. 2** **LMTK3 silencing down-regulates E2-induced expression of pS2, PgR and GREB1 and decreases the transcriptional activity of ERα in MELN cells**
   **(A)** MCF7 cells (5 x 10⁴) were plated in 24-well plates and transfected with 20nM CT siRNA or LMTK3 siRNA for 48h. Following, cells were treated with 10nM of E2 (or ET-OH; vehicle) for 24h. Cells were then harvested and total RNA was extracted and used to synthesise cDNA by reverse transcription, as described in Materials and Methods. Gene expression of pS2, PgR and GREB1 was measured by qRT-PCR.
   **(B)** MELN cells (5 x 10⁴) were plated in 24-well plates. Following 48h transfection with 20nM of CT siRNA or LMTK3 siRNA, the cells were incubated in the presence or absence of 10nM E2 for 24h. ERE-dependent gene expression was quantified by measuring luciferase activity, given as fold of control.
   **(C)** Quantitative real time RT-PCR validation of down-regulation of LMTK1-3 mRNA levels after treatment with 20nM of siRNA.

   GAPDH was used for normalisation. Error bars represent SD of 2 experiments, each in triplicate.

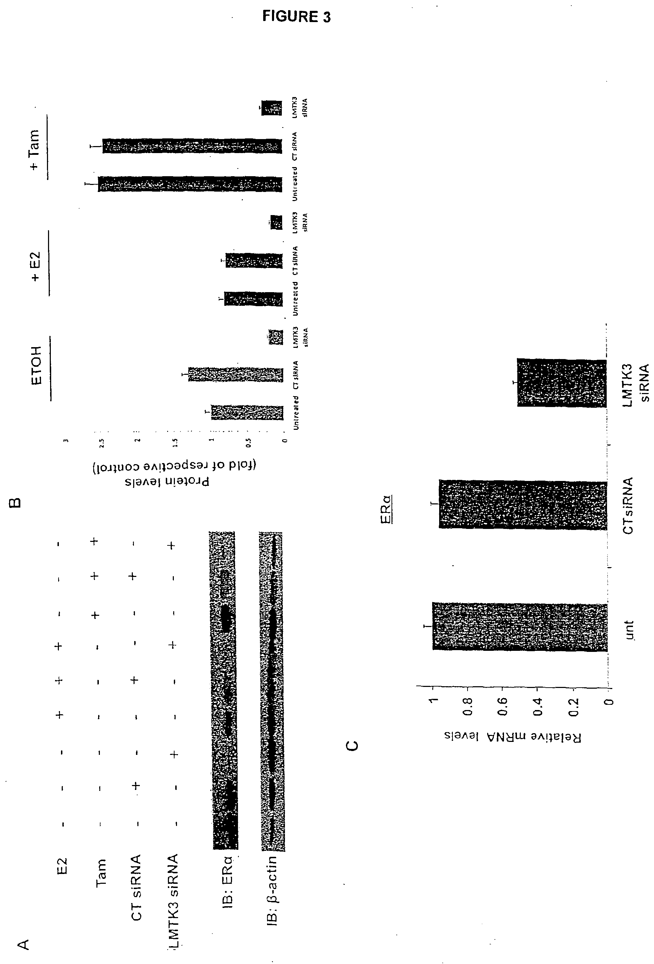

**Figure. 3** **Effects of LMTK3 silencing on ERα mRNA and protein levels in MCF7 cells**
   **(A)** MCF7 cells (1 x 10⁶) were plated in 10cm² plates in phenol red-free DMEM containing 10% DSS. The following day, cells were transfected with 20nM CT siRNA or LMTK3 siRNA for 72h and then treated or not (ET-OH) with E2 (10nM) for 24h. Cells were harvested, lysed and equal protein amounts were subjected to Western Blotting analysis using the indicated antibodies. β-actin was used as a control for sample loading.
   **(B)** Quantitative analysis of ERα and LMTK3 protein levels is given as fold of control. Error bars represent SD of 3 experiments (p < 0.05). (C) MCF7 cells (5 x 10⁴) were plated in 24-well plates and transfected with 20nM CT siRNA or LMTK3 siRNA for 48h.. Gene expression of ERα was measured by qRT-PCR. Error bars represent SD of 2 experiments each in triplicate (p < 0.05).

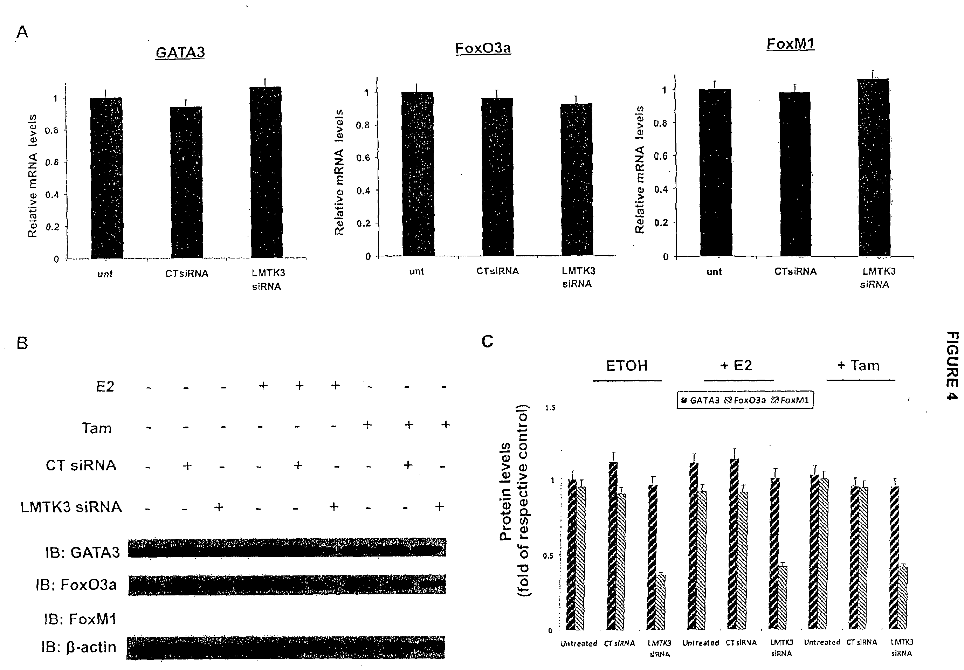

**Figure. 4** **Effects of LMTK3 silencing on transcriptional factors regulationg ERα**
   **(A)** MCF7 cells (5 x 10⁴) were plated in 24-well plates and transfected with 20nM CT siRNA or LMTK3 siRNA for 48h.. Gene expression of GATA3, Fox03a and FoxM1 were measured by qRT-PCR. Error bars represent SD of 2 experiments each in triplicate (p < 0.05). **(B)** MCF7 cells (1 x 10⁶) were plated in 10cm² plates in phenol red-free DMEM containing 10% DSS. The following day, cells were transfected with 20nM CT siRNA or LMTK3 siRNA for 72h and then treated or not (ET-OH) with E2 (10nM) for 24h. Cells were harvested, lysed and equal protein amounts were subjected to Western Blotting analysis using the indicated antibodies. β-actin was used as a control for sample loading. **(C)** Quantitative analysis of GATA3, Fox03a and FoxM1 protein levels is given as fold of control. Error bars represent SD of 3 experiments (p < 0.05).

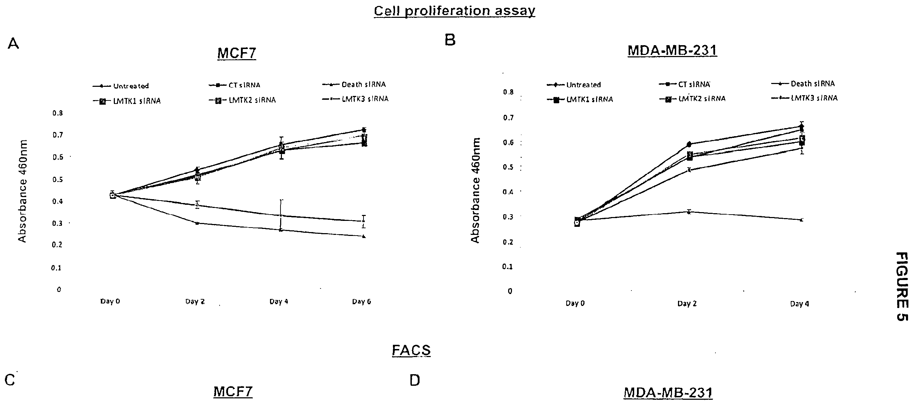

**Figure. 5** **Differential effects of silencing LMTK3 on the proliferation of ERα positive and negative breast cancer cell lines**
   BC cells were transfected with siRNA for the three LMTK isoforms (LMTK1, LMTK2 and LMTK3), CT siRNA and Death siRNA. Cells were subjected to the WST-1 cell proliferation assay, on the day of transfection (Day 0) and in consecutive 48 h intervals. Absorbance was measured at 460nm. Results represent the mean of three experiments per cell line ± SE. **(A)** Silencing of LMTK3 only inhibits proliferation of MCF7, ERα positive cells by > 50% (p < 0.001). **(B)** Silencing LMTK3 reduces proliferation of MDA-MB231, ERα negative cells by ∼15% (p = 0.08). Silencing of LMTK1 and LMTK2 isoforms did not affect proliferation of either BC cell lines.

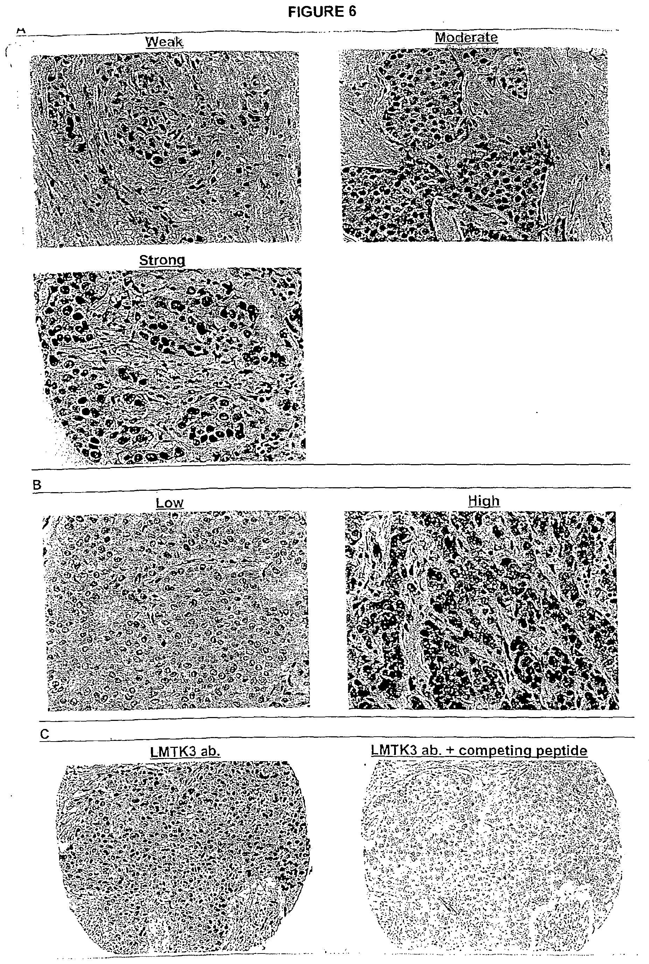

**Figure. 6** **Immunohistochemistry staining of LMTK3 in breast cancer tissue**
   Tissue microarrays (TMAs) containing 613 BC cases were immunostained and scored for LMTK3 expression. Representative images of different staining intensities are shown. **(A)** Nuclear LMTK3 staining: weak (H-score = 0-25); moderate (H-score = 26-134); strong (H-score = 135-300), **(B)** Cytoplasmic LMTK3 staining: low (Intensity score 0 & 1); High (Intensity score 2 & 3), **(C)** Specificity of the LMTK3 antibody was confirmed by the pre-incubation with the competing peptide. Magnification x200 was applied to all images.
**Figure. 7** **Nuclear LMTK3 expression in breast cancer tissue**
   Kaplan-Meier survival plots showing the association between nuclear LMTK3 expression and **(A)** Disease-free survival (DFS), **(B)** Breast cancer specific survival (BCSS), **(C)** Response to endocrine therapy, **(D)** Response to chemotherapy.
**Figure. 8** **Cytoplasmic LMTK3 expression in breast cancer tissue**
   Kaplan-Meier survival plots showing the association between cytoplasmic LMTK3 expression and **(A)** Disease-free survival (DFS), **(B)** Breast cancer specific survival (BCSS), (C) Response to endocrine therapy, (D) Response to chemotherapy.
**Figure 9** **Schematic representation of screening methodology**
**Figure 10** **Validation of screening and exclusion of off-target effects**
   **(A)** MCF7 cells (5 x 10⁴), plated in 24-well plates, were transfected (or not; mock) using 20nM of siRNA against MAPK and AKT as well as not targeting siRNA (CT siRNA). Following treatment with 10nM of E2 for 24h, the mRNA expression levels of pS2 gene were measured by qRT-PCR. Error bars represent SD of 2 experiments each in triplicate (p < 0.05). **(B)** Cell proliferation assay was performed in MCF7 cells transfected with 20nM of CT siRNA or 'positive cell death phenotype control' siRNA (Death siRNA) over the time course of the assay (3 days). Error bars represent SD of 2 experiments each in triplicate (p < 0.01).

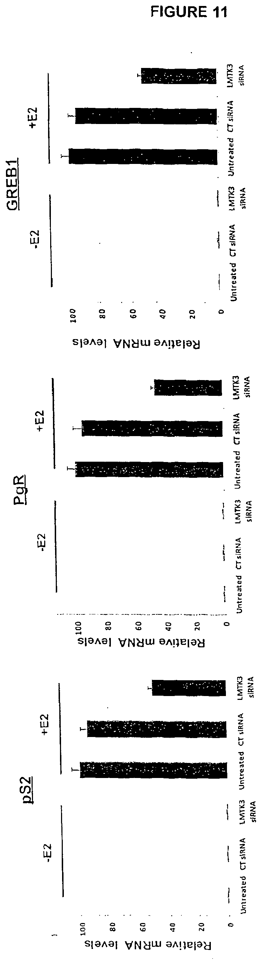

**Figure 11** **LMTK3 silencing down-regulates the expression of ERα-regulated genes in BT474 cells**
   **(A)** BT474 cells (5 x 10⁴) were plated in 24-well plates and transfected with 20nM CT siRNA or LMTK3 siRNA for 48h. Following, cells were treated with 10nM of E2 (or ET-OH; vehicle) for 24h. Cells were then harvested and total RNA was extracted and used to synthesise cDNA by reverse transcription, as described in Materials and Methods. Gene expression of pS2, PgR and GREB1 was measured by qRT-PCR. GAPDH was used for normalisation. Error bars represent SD of 2 experiments, each in triplicate.

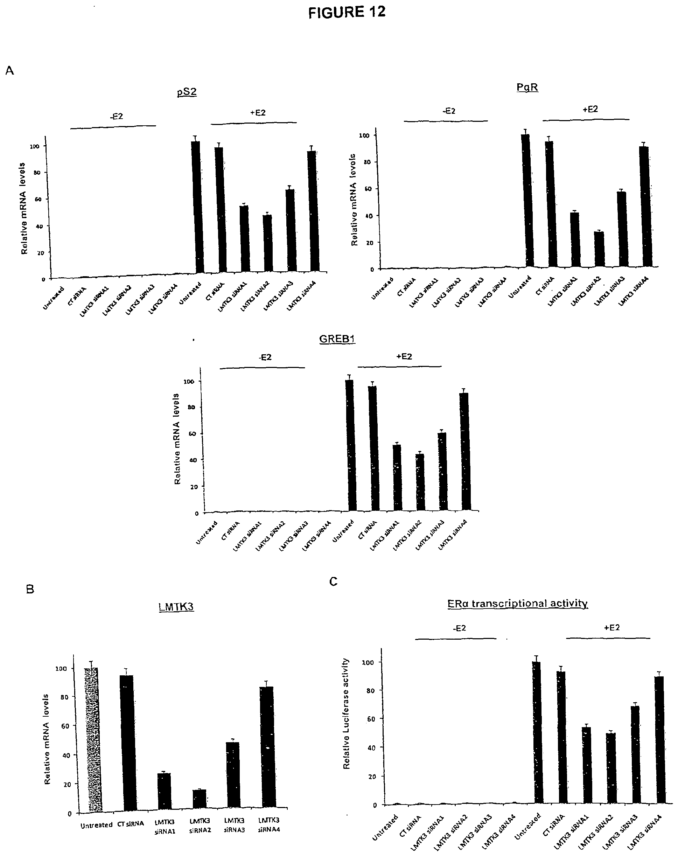

**Figure 12** **Validation of the effects of LMTK3 silencing on ERα transcriptional activity and the E2-induced expression of pS2, PgR and GREB1**
   **(A)** MCF7 cells (5 x 10⁴) were plated in 24-well plates and transfected with 20nM CT siRNA or 4 individual LMTK3 siRNAs for 48h. Following, cells were treated with 10nM of E2 (or ET-OH; vehicle) for 24h. Cells were then harvested and total RNA was extracted and used to synthesise cDNA by reverse transcription, as described in Materials and Methods. Gene expression of pS2, PgR and GREB1 was measured by qRT-PCR.
   **(B)** Quantitative real time RT-PCR validation of down-regulation of LMTK3 mRNA levels after treatment with 20nM of 4 individual LMTK3 siRNAs.
   **(C)** MELN cells (5 x 10⁴) were plated in 24-well plates. Following 48h transfection with 20nM of CT siRNA or 4 individual LMTK3 siRNAs, the cells were incubated in the presence or absence of 10nM E2 for 24h. ERE-dependent gene expression was quantified by measuring luciferase activity, given as fold of control. GAPDH was used for normalisation. Error bars represent SD of 2 experiments, each in triplicate.

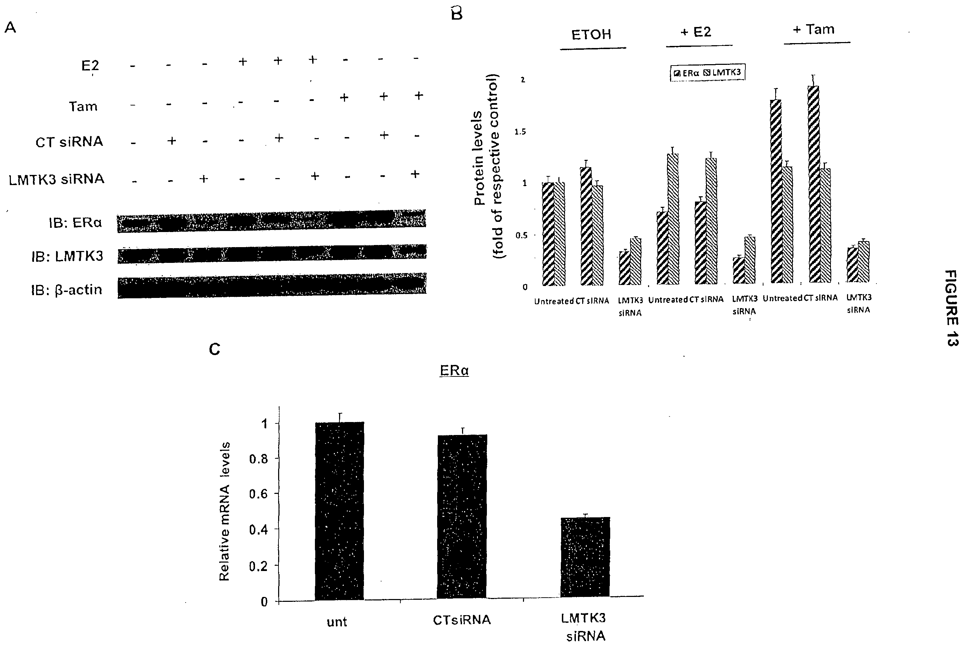

**Figure 13** **LMTK3 silencing down-regulates ERα mRNA and protein levels in BT474 cells**
   **(A)** BT474 cells (1 x 10⁶) were plated in 10cm² plates in phenol red-free DMEM containing 10% DSS. The following day, cells were transfected with 20nM CT siRNA or LMTK3 siRNA for 72h and then treated or not (ET-OH) with E2 (10nM) for 24h. Cells were harvested, lysed and equal protein amounts were subjected to Western Blotting analysis using the indicated antibodies. β-actin was used as a control for sample loading.
   **(B)** Quantitative analysis of ERα and LMTK3 protein levels is given as fold of control. Error bars represent SD of 3 experiments (p < 0.05). **(C)** BT474 cells (5 x 10⁴) were plated in 24-well plates and transfected with 20nM CT siRNA or LMTK3 siRNA for 48h.. Gene expression of ERα was measured by qRT-PCR. Error bars represent SD of 2 experiments each in triplicate (p < 0.05).

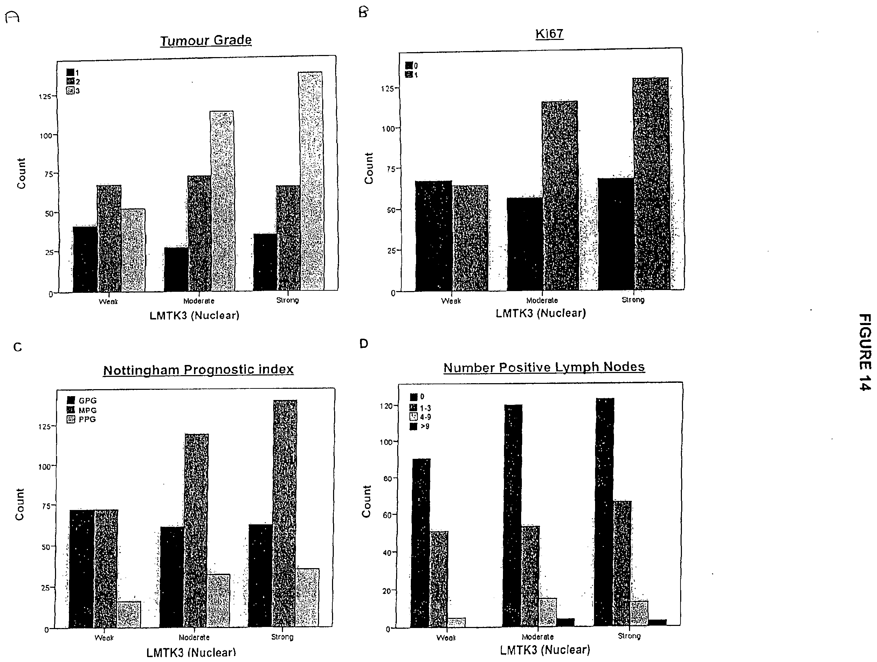

**Figure 14** **Correlation of the nuclear LMTK3 expression to relevant tumor characteristics.**
   **(A)** Tumor Grade (p< 0.0001), **(B)** Ki67 (p = 0.002), **(C)** Nottingham Prognostic Index (NPI) (p = 0.001), and **(D)** Positive Lymph Nodes (LN) (p = 0.308). Although the p value is non-significant, the presence of > 9 invaded LNs is only present in BCs expressing moderate and strong levels of nuclear LMTK3.
**Figure 15****: LMTK3 protein and nucleic acid sequence.**

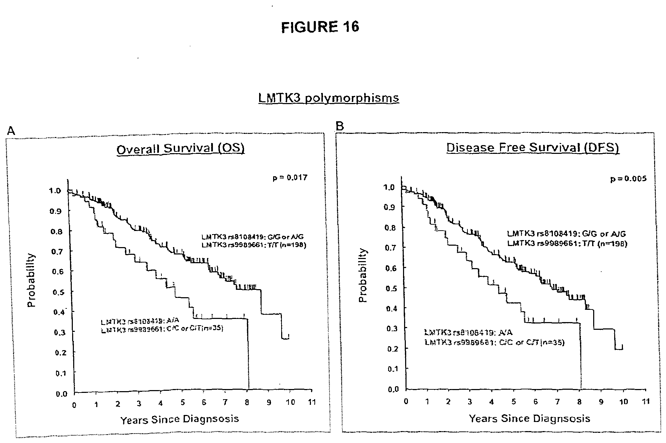

**Figure 16****: correlation of LMTK3 polymorphisms with overall survival and disease free survival.**

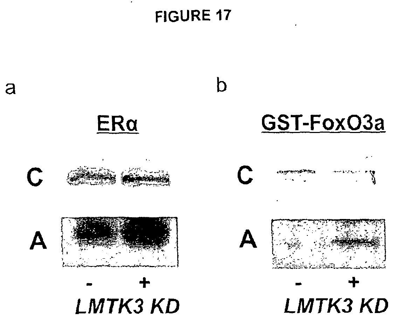

**Figure 17****. *In vitro* kinase assay demonstrating that LMTK3 kinase domain phosphorylates both the ERα and FoxO3a.**
   *In vitro* kinase assays were performed using recombinant LMTK3 kinase domain as sources of enzyme activity and a) full length recombinant human ERα, b) GST-recombinant human FoXO3a, as substrates. Proteins were separated by SDS-PAGE and the phosphorylated proteins were detected by autoradiography. C: Coomasie. A: Autoradiogram.

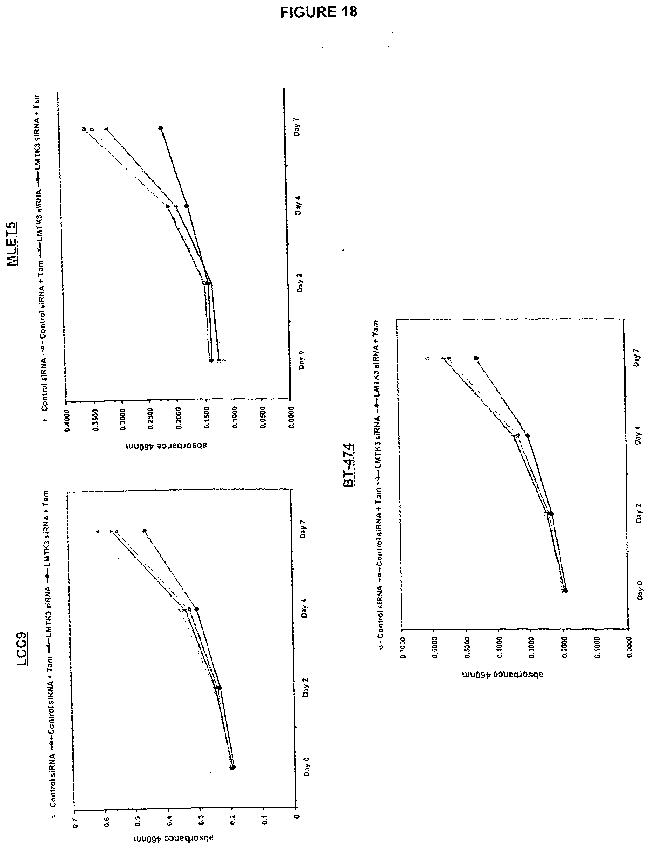

**Figure 18****. Inhibition of LMTK3 overcomes resistance to Tamoxifen treatment**
   Tamoxifen resistant cell lines (LCC9, MLET5, BT-474) were transfected with 20nM of control siRNA or LMTK3 siRNA in the presence or absence of Tamoxifen (100nM). Cells were subjected to the WST-1 cell proliferation assay, on the day of transfection (day 0) and then consecutive 48 h intervals. Absorbance was measured at 460 nm. Results represent the mean of three experiments per cell line ± SE. Silencing of LMTK3 partly reversed resistance of cell lines to Tamoxifen treatment.

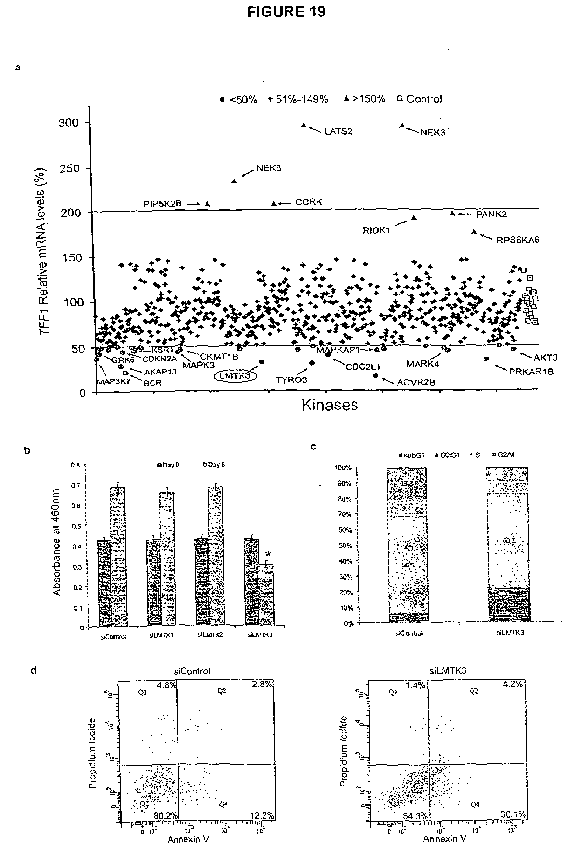

**Figure. 19** High-throughput siRNA screen identifies kinases modulating ERα transcriptional activity. **(a)** MCF7 cells were transfected with a pool of two different siRNAs per gene followed by E2 treatment. The percent relative mRNA levels of *TFF1* expression for each kinase is presented (100%= *TFF1* mRNA expression after E2 treatment alone). Hits resulting in: i) >50% up-regulation (*black triangles*), ii) <50% down-regulation (*red circles*) or iii) 51-149% changes (*blue diamonds*) of *TFF1* expression after individual kinase gene silencing. Non-targeting siRNA controls are indicated in *yellow* squares. (*p* < 0.05). **(b)** Silencing of LMTK3 in MCF7 cells inhibits proliferation by > 50% (*p* < 0.001 (Student's t test). **(c)** The proportion of MCF7 cells in subG₁ phase (apoptosis) was significantly higher after treatment with LMTK3 siRNA. **(d)** Representative cytograms and quantitation of apoptosis upon LMTK3 silencing.

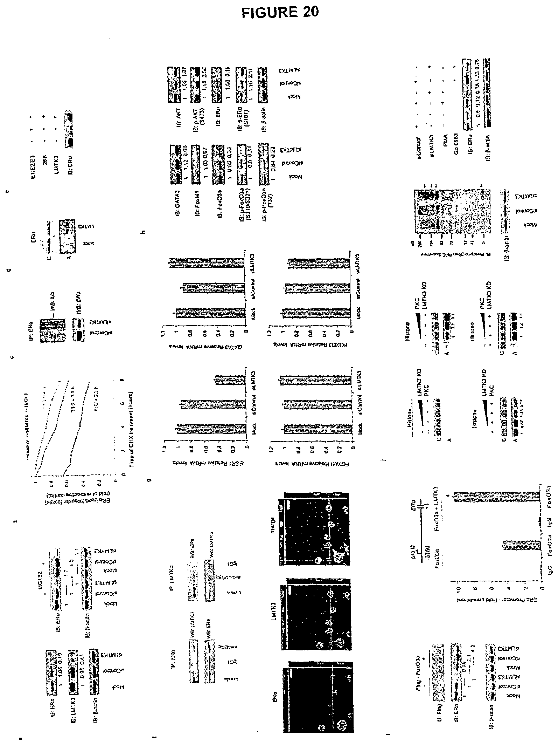

**Figure. 20** Mechanisms of LMTK3 action on ERα transcriptional and translational levels. **(a)** Silencing of LMTK3 decreased total ERα protein levels, while treatment with MG132 partly rescued ERα degradation. **(b)** Protein half-life of ERα after silencing and over-expressing LMTK3. **(c)** ERα ubiquitination assay after LMTK3 silencing. **(d)** ***In** vitro* phosphorylation of ERα by LMTK3. **(e)** *In vitro* degradation assay of ERα. **(f)** ERα and LMTK3 interact *in vivo* (Scale Bar, 50 µm). **(g)** Gene expression of *ERα, GATA3, FOXO3* and *FOXM1* after LMTK3 silencing **(h)** LMTK3 silencing increases FOXO3, p-AKT S473 and p-ERα S167 protein levels. **(i)** Over-expression of FOXO3 increases its binding to the ERα promoter rescuing ERα from LMTK3 siRNA-mediated degradation. **(j)** LMTK3 kinase domain (LMTK3 KD) inhibits the catalytic activity of PKC *in vitro.* LMTK3 silencing increased the ability of PKC to phosphorylate its substrates *in vivo.* LMTK3 silencing in the presence of a PKC activator (PMA) further degrades ERα, while inhibition of LMTK3 with a PKC inhibitor (Go 6983) partly rescues the LMTK3 siRNA-induced down-regulation of ERα.

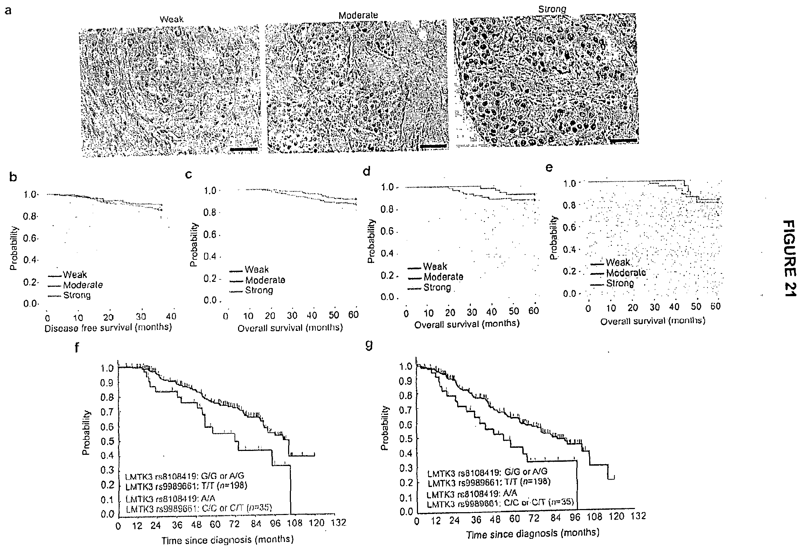

**Figure. 21** Association of LMTK3 expression and germline polymorphisms with clinical outcome. **(a)** Representative images of different staining intensities are shown. LMTK3 staining: weak (H-score = 0-25); moderate (H-score = 26-134); strong (H-score = 135-300). (Original magnification, 200x; Scale bar, 100µm). KM plots showing the association between LMTK3 expression and **(b)** Disease-free survival (*p* = 0.012), **(c)** Overall survival (*p* = 0.033), **(d)** Response to endocrine therapy (*p* = 0.039), **(e)** Response to chemotherapy (*p* = 0.184). KM plots demonstrating the association between two LMTK3 polymorphisms and **(f)** Overall survival (*p* = 0.017) and, **(g)** Disease-free survival (*p* = 0.02).

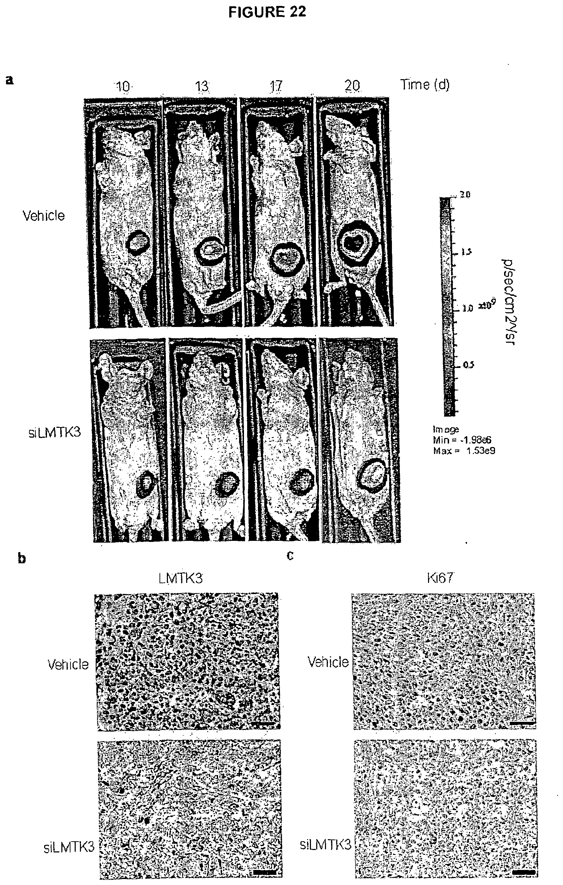

**Figure 22** Tumor growth inhibition in an orthotopic animal model by *in vivo* LMTK3 siRNA. **(a)** Bioluminescent images of a representative mouse for each group (n=8) (p<0.01). Histological analysis of **(b)** LMTK3 and, **(c)** Ki67 on representative tumor tissue sections demonstrate significant reduction of expression in LMTK3 siRNA vs vehicle treated tumors. (Original magnification, 200x; Scale bar, 100µm).

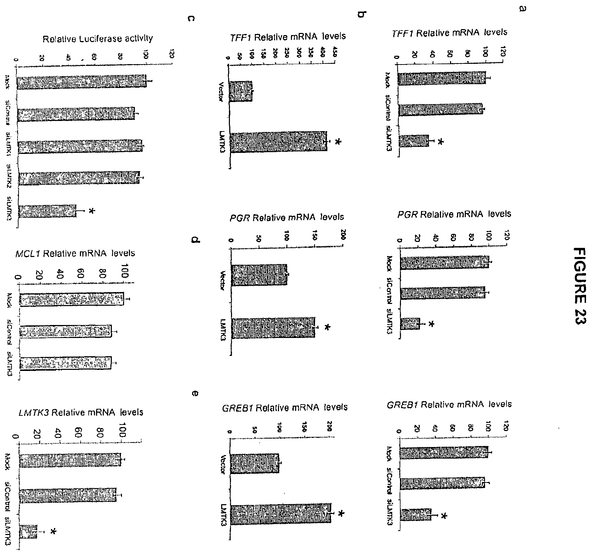

**Figure 23** Effects of *LMTK3* on ERα transcriptional activity and the expression of *TFF1, PGR, GREB1* and *MCL1* genes. **(a)** Transfection of MCF7 cells with *LMTK3* siRNA followed by treatment with E2, significantly inhibited the expression of estrogen-regulated genes (*TFF1, PGR* and *GREB1*) while **(b)** over-expression of *LMTK3* increased the **mRNA** levels of these genes. **(c)** ERE-dependent gene expression quantification after *LMTK3* silencing. **(d)** Silencing of *LMTK3* did not affect gene expression of *MCL1.* **(e)** RT-qPCR validation of down-regulation of *LMTK3* **mRNA** levels after treatment with 20 nM of siRNA. *GAPDH* was used for normalization. Error bars represent SD of 2 experiments, each in triplicate (* *p* < 0.05 compared to siControl (Student's t test)).

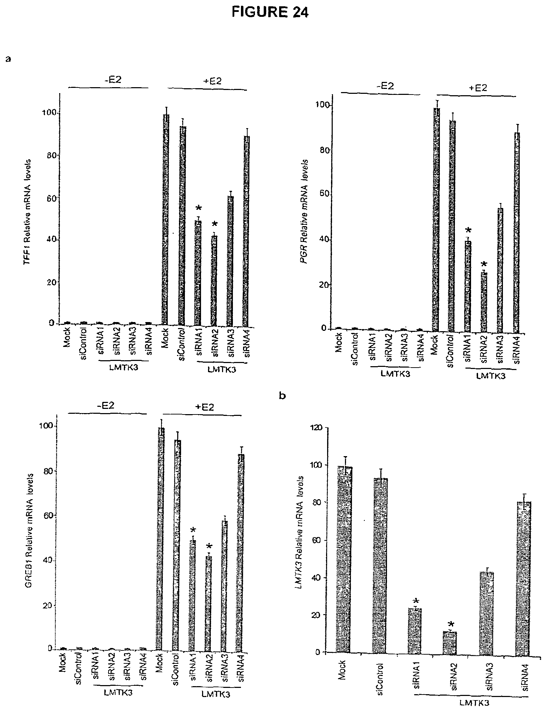

**Figure 24** Validation of the effects of *LMTK3* silencing on the E2-induced expression of *TFF1, PGR* and *GREB1* genes. **(a)** Gene expression of *TFF1, PGR* and *GREB1* in MCF7 cells transfected with 4 individual *LMTK3* siRNAs. **(b)** Validation of down-regulation of *LMTK3* **mRNA** levels after treatment with 4 individual *LMTK3* siRNAs (* *p <* 0.05 compared to siControl (Student's t test)).

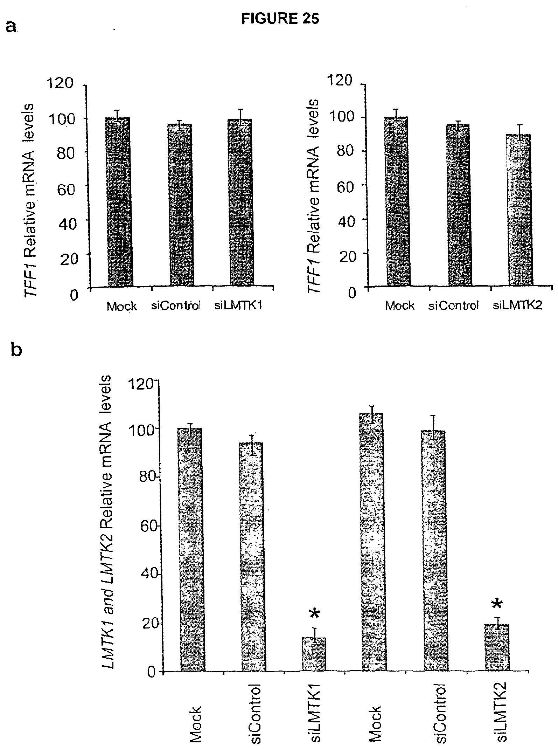

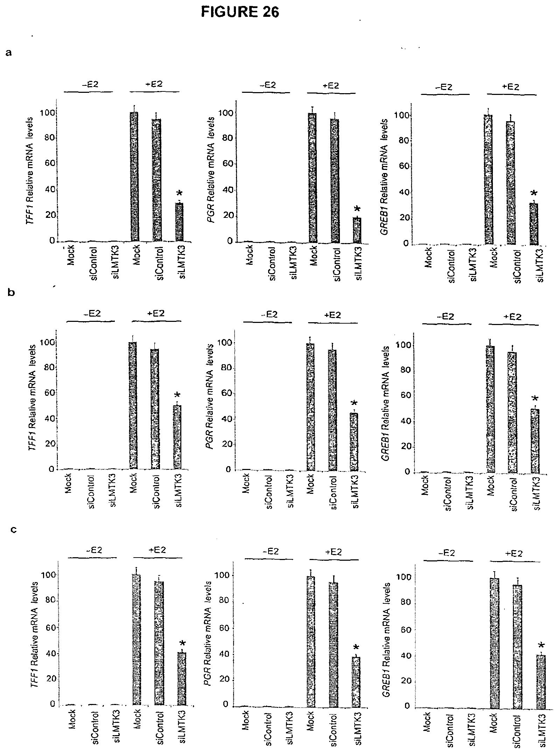

**Figure 25** Effects of *LMTK1* and *LMTK2* silencing on *TFF1* expression levels. **(a)** Gene expression of *TFF1* in *LMTK1* and *LMTK2* siRNA treated MCF7 cells. **(b)** RT-qPCR validation of down-regulation of *LMTK1* and *LMTK2* **mRNA** levels after treatment with 20 nM of siRNA. *GAPDH* was used for normalization. Error bars represent SD of 2 experiments, each in triplicate (* *p* < 0.05 compared to siControl (Student's t test)). **Figure 26** LMTK3 silencing down-regulates the expression of *TFF1,* PGR and *GREB1* genes in **(a)** MCF7, **(b)** BT-474 and **(c)** ZR-75-1 ERα+ cell lines (* *p* < 0.05 compared to siControl (Student's t test)).

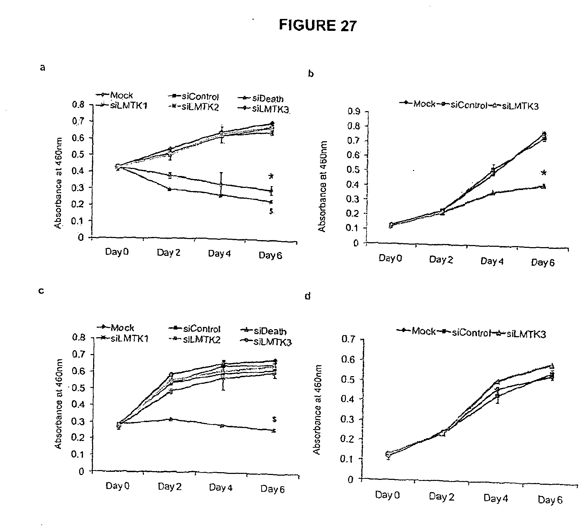

**Figure 27** LMTK3 silencing has differential effects on proliferation of ERα+ and ERα-cell lines. ERα+ cell lines: **(a)** MCF7, **(b)** ZR-75-1, and ERα- cell lines: **(c)** MDA-468 and **(d)** SKBR-3 were transfected with siRNA for the three *LMTK* isoforms (*LMTK1, LMTK2* and *LMTK3*). Silencing of *LMTK3* inhibits proliferation of ERα+ cells by > 50% and by ∼10% of ERα- cells. Results represent the mean of three experiments per cell line ± SE (*, $ *p* < 0.05 compared to siControl (Student's t test)).

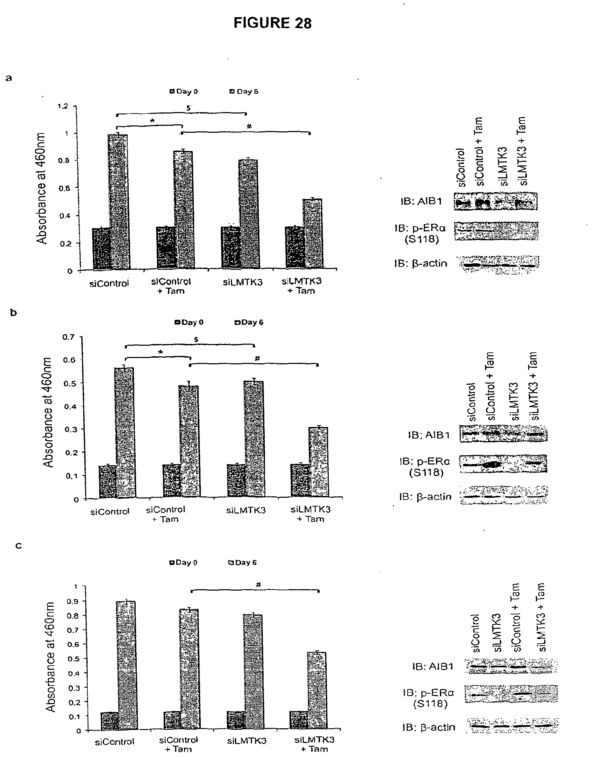

**Figure 28** Effects of LMTK3 silencing in tamoxifen-resistant cell lines. **(a)** BT-474, **(b)** MLET5 and, **(c)** LCC9 cells were transfected with LMTK3 siRNA in the presence or absence of tamoxifen (100nM). Comparisons are shown amongst: i) siControl-transfected and tamoxifen treated cells (*), ii) siControl- and siLMTK3-transfected cells ($) and iii) tamoxifen treated and siLMTK3-transfected cells (*p* < 0.05 - Student's t test). Western blotting of AIB1 and p-ERα S118 protein expression levels are shown.

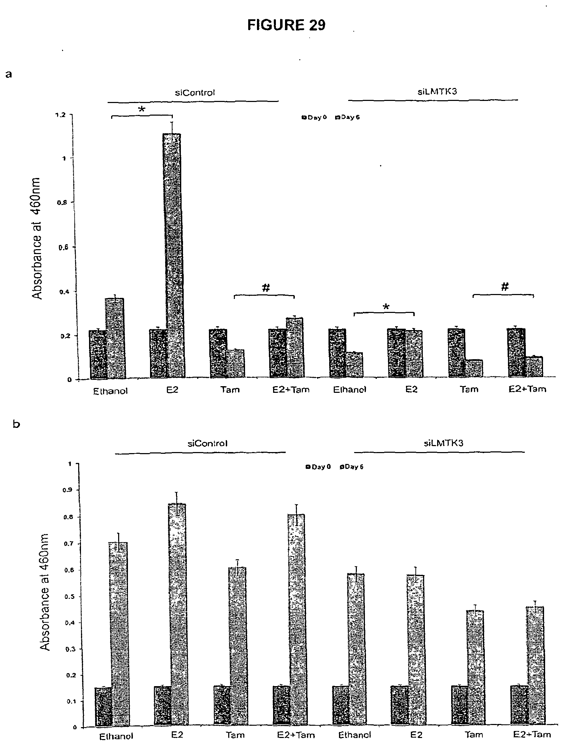

**Figure 29** LMTK3 is essential for E2-induced growth. **(a)** E2/tamoxifen-responsive (MCF7) and, **(b)** E2/tamoxifen-non responsive (LCC9) cells were transfected with LMTK3 siRNA and treated with ethanol (vehicle), E2 (10 nM), tamoxifen (100 nM) or E2+tamoxifen. Silencing of LMTK3 impeded E2- (and E2/tamoxifen-) induced proliferation of MCF7 while, it did not affect E2- (and E2/tamoxifen-) growth of MLET5. Comparisons are shown amongst siControl and siLMTK3-transfected cells between i) the ethanol and E2- induced growth (*) and ii) between the tamoxifen and tamoxifen+E2 induced growth (#). (p < 0.05 - Student's t test).

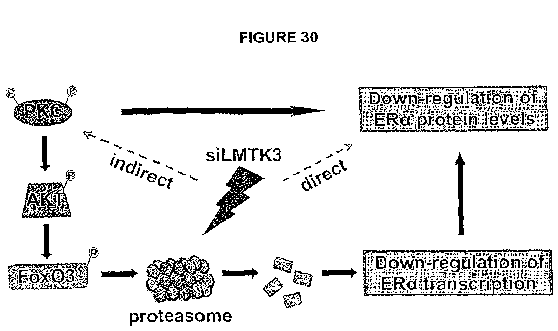

**Figure 30** Model of ERα regulation by LMTK3. LMTK3 interacts with and phosphorylates ERα stabilizing it and protecting it from proteasome-mediated degradation. Silencing of LMTK3 (staggered arrows) leads to activation of PKC resulting in: i) down-regulation of ERα protein levels and, ii) decreased ERα transcriptional activity by degradating FOXO3 via AKT phosphorylation.

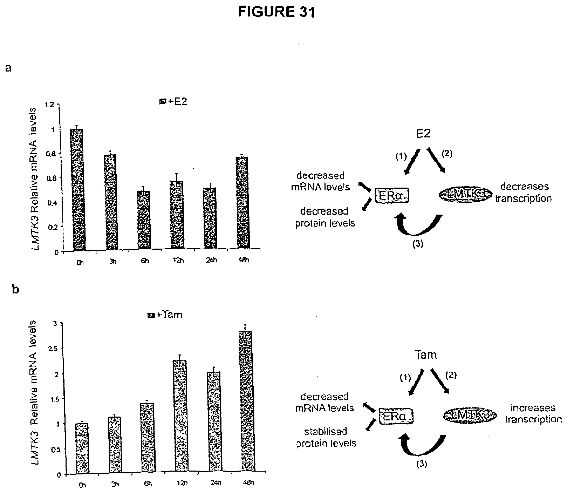

**Figure 31** Positive transcriptional feedback between ERα and LMTK3. **(a)** E2 down-regulates LMTK3 gene expression. Schematic: (1) E2 decreases ERα mRNA levels (transcription), which directly correlates with a decrease in ERα protein levels through the ubiquitin proteasome pathway. (2) In this study, we show that E2 decreases LMTK3 mRNA, leading to (3) decreased ERα mRNA and protein levels (as observed when using LMTK3 siRNA). **(b)** Tamoxifen up-regulates LMTK3 gene expression. Schematic: (1) Tamoxifen decreases ERα mRNA (transcription) while it stabilises ERα protein levels. (2) In this study, we show that tamoxifen increases LMTK3 mRNA, leading to (3) decreased ERα mRNA and stabilisation of ERα protein levels (as observed when over-expressing LMTK3).
**Figure 32.** (a) Low and high cytoplasmic (lower) and, (b) weak, moderate and strong nuclear staining (top) of LMTK3 in breast cancer tissues.
**Figure 33.** High baseline LMTK3 expression is associated with (a) poorer overall survival and, (b) poorer disease free survival in East Asian patients.
**Figure 34.** LMTK3 staining in control and tumor tissues from most major tumor sites.

### Example 1: Identification of LMTK3 as a new target in breast cancer

Therapies that target estrogenic signaling have transformed the treatment of breast cancer. Using a kinase RNAi screening, we identified the kinases that modulate the activity of the Estrogen Receptor-alpha (ERα). Small interfering RNA (siRNA) molecules represent an invaluable laboratory tool, since they can be used to investigate the downstream effects of silencing specific genes (27). A systematic high-throughput RNA interference screening using a large Human Kinase siRNA Library was performed to identify kinases able to phosphorylate and/or modulate ERα activity. Here, we demonstrate that LMTK3 activates ERα transcriptional activity and its inhibition leads to down-regulation of the well known E2-responsive genes, pS2, PgR and GREB1. Furthermore, we show that silencing of LMTK3 in breast cancer cell lines reduces the mRNA and protein levels of ERα and leads to increased cell death. Finally, we present evidence that LMTK3 is over-expressed in BC and represents both a prognostic and predictive factor in BC. We provide evidence that increased LMTK3 levels correlate with relapse in a large cohort of breast cancers, revealing LMTK3 as a new oncogenic target in BC.

### Materials and Methods

### Cell lines, reagents and antibodies

MCF7, BT474, MDA-MB-231 and MELN cells were maintained in DMEM supplemented with 10% FCS and 1% penicillin/ streptomycin / glutamine. All cells were incubated at 37°C in a humidified 5% CO₂ atmosphere. Estradiol (E2) was obtained from Sigma (Gillingham, UK) and dissolved in ethanol; Charcoal-dextran stripped serum (DSS) was obtained from Gemini (Bolnet, UK). The following antibodies were used: phospho (Cell Signaling, UK), (, Abcam, UK), phospho ERα (ser118) (2511, Santa Cruz (Heidelberg, Germany); Abcam (Cambridge, UK) and Millipore (Southampton, UK) respectively. Anti-β-actin mouse monoclonal antibody was obtained from Abcam (Cambridge, UK). Secondary HRP (horseradish peroxidase)-conjugated goat anti-rabbit IgG and goat anti-mouse IgG antibodies were from GE Healthcare (Slough, UK).

### High throughput siRNA screening

The Human Kinase siRNA Set Version 3.0 library (Qiagen, Crawley, UK) targeting 691 kinases and kinase-related genes has been used. The library was supplied in a 96-well format and contained a pool of two individual siRNAs per well targeting two different sequences for each gene. MCF7 cells were maintained in phenol red-free media with 10% charcoal-stripped serum (DSS) 48h before experimentation. Following, cells were plated in 24-well plates and transfected with siRNA (final concentration 20nM) using the Human Kinase siRNA Set Version 3.0 library and Hiperfect reagent according to the manufacturer's instructions (Qiagen). At 48h post-transfection, cells were treated with vehicle (ethanol) or E2 (10nM) for 24h and cells were harvested following RNA extraction and cDNA synthesis, as described below. Quantitative RT-PCR (qRT-PCR) analysis to examine the expression levels of pS2 and GAPDH (endogenous control) was performed for each well (kinase gene). Following, the pS2 gene expression, after silencing each kinase individually, was calculated in relation to GAPDH expression. The screening was performed in duplicate.

### RNA isolation and quantitative RT-PCR

Total RNA was isolated using the RNeasy kit (Qiagen). Reverse transcription was done using the high capacity cDNA reverse transcription kit (Applied Biosystems, USA). qRT-PCR analysis was performed on a 7900HT Thermocycler (Applied Biosystems) using TaqMan mastermix and primers for pS2, PgR, Greb1 and GAPDH cDNAs, purchased from Applied Biosystems.

### SDS-PAGE and Western blotting

Whole cell lysates were prepared using NP40 lysis buffer (50mM Tris-HCl, pH 8.0, 150mM NaCl, 10% (v/v) glycerol, 1% NP40, 5mM dithiothreitol (DTT), 1mM EDTA, 1mM EGTA, 50µM leupeptin and 30µg/ml aprotinin). These extracts were then clarified by centrifugation at 15000rpm for 15min at 4°C for western blotting. The bicinchoninic acid (BCA) protein assay (Pierce) was used to determine the protein concentration of the lysates. Lysates were incubated in 5x sodium dodecyl sulfate (SDS) sample buffer (5min, 95°C), subjected to 8% or 12% SDS-PAGE and blotted on a Hybond ECL super nitrocellulose membrane (GE Healthcare). The membranes were then blocked in TBS containing 0.1% (v/v) Tween20 and 5% (w/v) non-fat milk for 1 h and subsequently probed overnight with different antibodies following extensive washing with TBS/Tween and incubation with HRP-conjugated goat anti-rabbit IgG or goat anti-mouse IgG (1:1000 dilution) for 45mins. Enhanced chemiluminescence (ECL) detection then allowed detection of the immunocomplexes. The intensity of the bands were quantified using Image J software (NIH, Bethesda, MD).

### Firefly luciferase assay

MELN cells (0.5 x 10⁶) were plated in 24-well plates and starved for 24h in DMEM/10% DSS medium. The cells were then transfeted with xxxxx siRNA for 48h, treated with vehicle (ethanol) or E2 (10nM) for 24h and washed with PBS. Luciferase assays were performed using the firefly luciferase assay system (Promega) according to the manufacturer's instructions and measured with a Top Count NXT luminometer (Packard Biosciences, Beaconsfield, UK). All experiments were performed three times independently and the results were presented as the mean with standard error. Data are normalized to the untreated sample, which was given a reference value of 1.

### Cell proliferation assay

The colorimetric Rapid Cell Proliferation Kit (Calbiochem, UK) has been used. The assay is based on the incubation of cells with the tatrazolium salt WST-1, which is cleaved by mitochondrial dehydrogenases, functional only in viable cells. Therefore, the assay measurement corresponds to relative number or % of viable cells in proliferation after certain treatment. Briefly, cells were seeded at 3 x 10³ /well in a 96 well plate and allowed to adhere. The following day, cells were incubated with the WST-1 for 30 min at 37°C, according to manufacturer's instructions, and the absorbance read with the microplate reader at 460nm was considered to be Day 0. The same day cells were transfected with the CT siRNA, death siRNA and LMTK1-3 siRNAs. Readings were repeated consecutively in 48h intervals. Results for Day 0, Day 2, Day 4 and Day 6 were plotted ± SE.

### Clinical specimens and tissue microarrays

Tissue microarrays (TMAs) containing 614 primary operable BC cases from the Nottingham Tenovus Primary Breast Carcinoma Series (26) were employed. The cohort comprised women aged up to 70 years, who presented between 1986 and 1999. This well- characterized resource contains information on patients' clinical and pathological data including histological tumor type, primary tumor size, lymph node status, histological grade and data on other BC relevant biomarkers. These include, ERα, PgR, epidermal growth factor 2 receptor (Her2), cytokeratines (CKs; 5/6, 7/8, 18), the proliferation marker Ki67 and E-cadherin. Patients within the good prognosis group (Nottingham Prognostic Index (NPI) ≤ 3.4) did not receive adjuvant therapy (AT). Hormonal therapy (HT) was prescribed to patients with ERα positive tumours and NPI scores > 3.4 (moderate and poor prognostic groups). Pre-menopausal patients within the moderate and poor prognosis groups were candidates for chemotherapy. Conversely, postmenopausal patients with moderate or poor NPI and ERα positive were offered HT, while ERα negative patients received chemotherapy. Data collected included overall survival, breast cancer specific survival (BCSS), disease-free survival (DFS) and time to development of loco-regional and distant metastasis (DM). Clinical data were maintained on a prospective basis. Median follow-up was 124 months (range 1 to 233). Breast cancer specific survival (BCSS) was defined as the time (in months) from date of the primary surgical treatment to the time of death from BC, death being scored as an event, and patients who died as a result of other causes or were still alive were excluded at the time of last follow-up. DFS was defined as the interval (in months) from the date of the primary surgery to the first loco-regional or distant metastasis.

### Immunohistochemistry

Anti-LMTK3 mouse monoclonal antibody (Santa Cruz, UK) was optimized to a working concentration 2µg on full-face excisional BC tissue sections. Subsequently, BC TMA (n = 614) cases comprising 4 µm thick formalin fixed paraffin embedded tissue cores were immunostained with the optimised anti-LMTK3 McAb on the Leica BOND-MAX automated system using manufacturer's instructions. Hit-induced epitope retrieval was performed in citrate buffer (ER1) for 5 minutes. Detection was achieved using the Polymer Detection Kit (Leica Microsystems Inc., USA), These detection systems contain Peroxidase Block, Protein Block, Post Primary Block, Novolink Polymer, DAB Chromogen, Novolink DAB Substrate Buffer (Polymer) and Hematoxylin for subsequent counterstaining of the TMAs. Negative controls were performed by omission of the primary antibody. LMTK3 immunoreactivity was detected in the nucleus of breast epithelial cells, and in the cytoplasm to a variable degree. Nuclear staining was scored based on the nuclear H-score. Determination of the optimal cut-offs were performed using X-tile bioinformatics software; (Yale University, USA) (28) Cut-off values were as follows: weak (1-25); moderate (26-134); strong (135-300). Cytoplasmic staining was scored based on intensity ranging from 0 to +3; 0 = null, +1 = low, +2 = intermediate and +3 = high level of staining intensity. Scoring for each tissue core on the TMAs was done by two independent investigators. High resolution digital imaging (NanoZomer, Hamamatsu Photonics, Welwyn Garden City, UK) at 20x magnification with a web-based interface (Distiller, SlidePath Ltd., Dublin, Ireland) was used. All cases were scored without prior knowledge of the clinicopathological or outcome data. Standard cut-offs were used for established prognostic factors were: ERα, PgR and cytokeratins 5/6, 7/8 and 18, positive if ≥ 10%; Ecadherin positive if H-score ≥45. Ki67 negative if ≤10%; BRCA1 H-score was 0 = 0 1-100 = 1 and >100 = 2. Cut-off values for different biomarkers included in the study were chosen before statistical analysis.

### Statistical Analysis

Statistical analysis for TMAs was performed using SPSS 16.0 statistical software (SPSS Inc., Chicago, IL, USA). Analysis of categorical variables was performed with the appropriate statistical test. Survival curves were analyzed using the Kaplan-Meier method with significance determined by the Log Rank test. Multivariate analysis was performed by Cox hazard analysis. A *P* value ≤ 0.05 (two-sided) was considered significant.

Exploratory data analysis for *in vitro* data demonstrated that the distributions were often skewed with outliers. Shapiro-Wilks test was used to test for normality (data were not normally distributed). Between group comparisons were made using either the non parametric Mann Whitney U test.

### Results

### High-Throughput ERα-targeted siRNA kinase screening

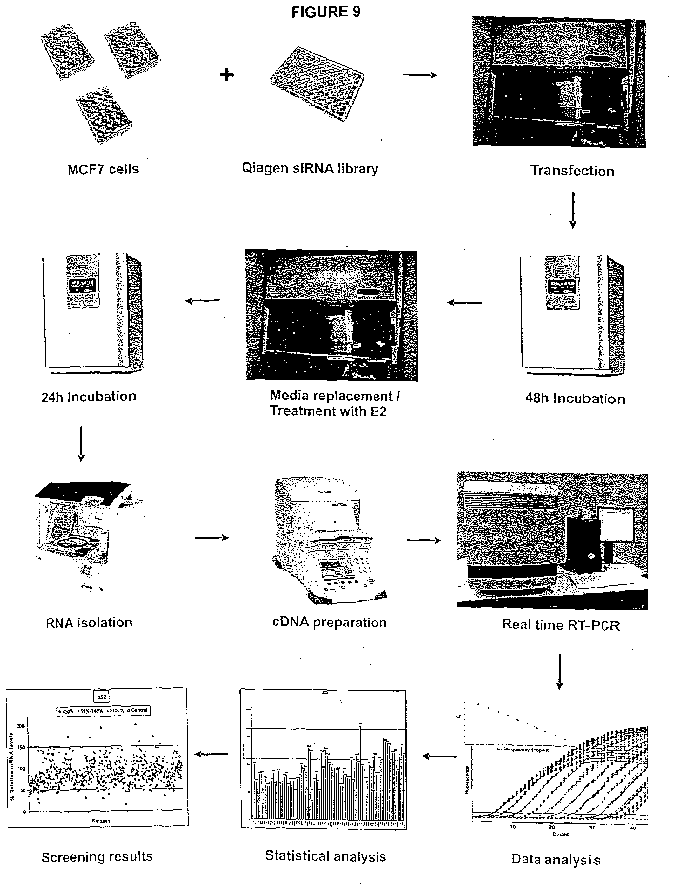

In order to identify novel kinases that are able to modulate the transcriptional activity of ERα, we performed a high-throughput RNAi screening targeting 691 kinase genes (Qiagen). An ERα positive breast cancer cell line (MCF7) has been used and transfected with a pool of 2 siRNAs per targeted gene in a 24-well format (total 1,382 siRNA duplexes). 48 hours after transfection, cells were treated with E2 or vehicle (ethanol) for 24 hours; subsequently, quantitative real time-PCR (qRT-PCR) analysis was performed to examine the expression levels of pS2, an ERα-regulated gene **(****Fig. 9****).** The entire screening has been repeated in duplicate and all data have been combined in the final analysis **(****Fig. 1****).** Based on our results, we discovered several kinases (among them some already identified that served as positive controls) that were able to significantly modulate the mRNA expression levels of pS2. We categorized the top 20 statistically significant hits into 2 groups as follows: a) those that after silencing they up-regulate ERα activity >50%, as demonstrated by the pS2 expression levels, comprising 7 kinases and, b) those that after silencing they down-regulate ERα activity <50% comprising 13 kinases **(Table 1).**

**Table 1. Results of human kinome ERα-regulated siRNA screening**

| **Group A (ERα up-regulation)** | | | |
|---|---|---|---|
| **siRNA pool** | **Gene name** | **pS2 (%)** | **p-value** |
| **LATS2** | large tumor suppressor, homolog 2 | 196.3 | |
| **NEK8** | NIMA (never in mitosis gene a)- related kinase 8 | 134.6 | |
| **PIPSK2B** | phosphatidylinositol-4-phosphate 5-kinase, type II, beta | 110.8 | |
| **CCRK** | cell cycle related kinase | 102.7 | |
| **NEK3** | NIMA (never in mitosis gene a)- related kinase 3 | 198.2 | <0.05 |
| **PANK2** | pantothenate kinase 2 | 90.9 | |
| **RIOK1** | RIO kinase 1 | 84.7 | |
| **RPS6KA6** | ribosomal protein S6 kinase, 90kDa, polypeptide 6 | 74.9 | |
| **BMPR1B** | bone morphogenetic protein receptor, type IB | 55.8 | |

| **Group B (ERα down-regulation)** | | | |
|---|---|---|---|
| **siRNA pool** | **Gene name** | **pS2 (%)** | **p-value** |
| **ACVR2B** | activin A receptor, type IIB | 78.5 | |
| **BCR** | breakpoint cluster region | 71.7 | |
| **AKAP13** | A-kinase anchor protein 13 | 69.9 | |
| **TYRO3** | TYRO3 protein tyrosine kinase | 69.5 | |
| **LMTK3** | lemur tyrosine kinase 3 | 66.2 | |
| **PRKAR18** | protein kinase, cAMP-dependent, regulatory, type I, beta | 65.1 | |
| **MAP3K7** | mitogen-activated protein kinase kinase kinase 7 | 60.1 | |
| **CDC2L1** | cell division cycle 2-like | 57.9 | <0.05 |
| **GRK6** | G protein-coupled receptor kinase 6 | 56.5 | |
| **CDKN2A** | cyclin-dependent kinase inhibitor 2A | 55.8 | |
| **MARK4** | MAP/microtubule affinity-regulating kinase 4 | 55.6 | |
| **CKMT1B** | creatine kinase, mitochondrial 1B | 55.1 | |
| **KSR1** | kinase suppressor of ras 1 | 54.4 | |
| **AKT3** | v-akt murine thymoma viral oncogene homolog 3 | 54.2 | |
| **MAPKAP1** | mitogen-activated protein kinase associated protein 1 | 53.1 | |

### Validation and specificity of screening

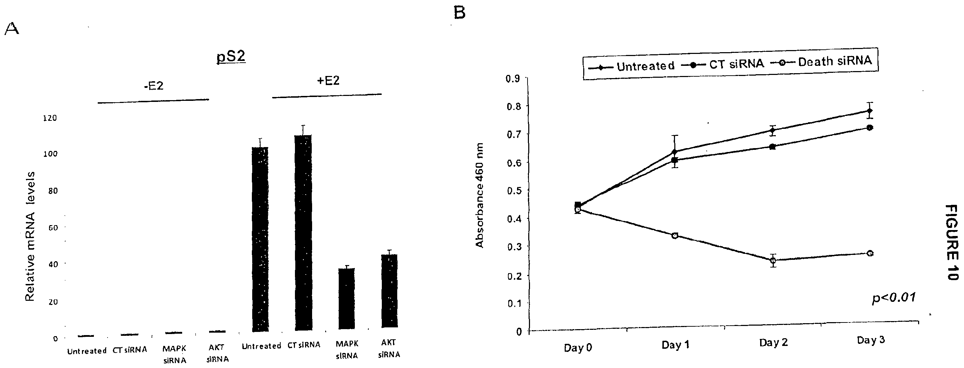

The performance of our transfection conditions and qRT-PCR reporter assay has been validated after performing the following experiments in MCF7 cells: (a) transfection with control siRNA (CT siRNA / non targeting siRNA) alter the relative mRNA expression levels of pS2 less than 10% compared to mock-transfected cells, after E2 treatment **(****Fig 10A****)** and (b) transfection with siRNAs targeting previously identified kinases that are able to phosphorylate and modulate the transcriptional activity of ERα, including MAPK and AKT (positive controls) resulted in a down-regulation of the pS2 relative mRNA expression levels >60% after E2 treatment, as expected (Fig 11A). In addition, in order to check the potential toxicity of our experimental conditions, we transfected MCF7 cells with CT siRNA and 'positive cell death phenotype control' siRNA (Death siRNA), which targets genes that are essential for cell survival. As presented in **Fig 10B****,** CT siRNA did not cause significant reduction in cell viability (<10%) as opposed to Death siRNA (∼80%). Our results further confirmed that these hits appear to have on-target effect.

### LMTK3 silencing causes significant decrease in E2-induced expression of ERα regulated genes

In order to decrease the number of the newly identified kinases and focus on the most important ones, we further examined the effects of the top 20 kinase-hits on the expression levels of two additional ERα regulated genes (PgR and GREB1), after individually silencing these kinases. We validated these data by using the pool siRNA as well as the 2 individual siRNAs/kinase in independent experiments, in order to exclude the possibility of non-specific effects. Our results demonstrated that: i) 2 of the 7 kinases that up-regulated ERα activity (according to pS2 expression) were also able to up-regulate the gene expression levels of PgR and GREB1 less than 50% (group A), and ii) 3 of the 13 kinases that down-regulated ERα activity (according to pS2 expression) were also able to down-regulate the gene expression levels of PgR and GREB1 >50% (group B) **(Table 2).**

**Supplementary Table 1. Results of human kinome ERα-regulated siRNA screening**

| **Group A (ERα up-regulation)** | | | | |
|---|---|---|---|---|
| **siRNA pool** | **PgR (%)** | **GREB1 (%)** | **pS2 (%)** | **p-value** |
| **CCRK** | 156.1 | 73.2 | 102.7 | <0.05 |
| **RPS6KA6** | 215.7 | 79.8 | 74.9 | |

| **Group B (ERα down-regulation)** | | | | |
|---|---|---|---|---|
| **siRNA pool** | **PgR (%)** | **GREB1 (%)** | **pS2 (%)** | **p-value** |
| **TYRO3** | 81.3 | 82.1 | 69.5 | |
| **LMTK3** | 78.1 | 66.4 | 66.2 | <0.05 |
| **KSR1** | 85.6 | 85.3 | 54.4 | |

Among the kinases that resulted in down-regulation of ERα activity, we identified Lemur Tyrosine Kinase 3 (LMTK3) to have highly significant effect in all three ERα-regulated genes (Fig. 2A). In addition, taken also into consideration the fact that so far no reports have linked LMTK3 with ERα, we decided to further investigate the mechanism of action of LMTK3 on ERα activity. LMTK3 silencing also resulted in a significant downregulation of pS2, PgR and GREB1 when tested in another ERα positive breast cancer cell line (BT474) **(****Fig 11****).**

### LMTK3 modulates ERα transcriptional activity in MELN cells

To examine the effects of LMTK3 in E2-dependent transcriptional activation of ERα, MELN cells were transfected with CT siRNA) or LMTK3 siRNA, treated with E2, and luciferase activities were measured. Upon LMTK3 silencing the E2-dependent luciferase activity was decreased by ∼50% compared to E2 treatment alone; no significant changes were observed after CT siRNA transfection. In addition, analysis of the other 2 members of the LMTK family (LMTK1 and LMTK2) did not have any effect on ERα activity **(****Fig. 2B****).These** data further demonstrate a specific association of LMTK3 isoform in E2-induced ERα activation. LMTK genes silencing was confirmed by qRT-PCR **(****Fig 2C****).** To further confirm the specificity of LMTK3 in modulating ERα activity and ERα-regulated gene expression, we repeated the experiments using 4 individual siRNAs for LMTK3 **(****Fig. 12****).**

### LMTK3 silencing decreases ERα protein and mRNA levels in breast cancer cell lines in a ligand-independent way

In order to investigate whether LMTK3 silencing influences ERα protein levels, we transfected MCF7 cells with siRNA targeting LMTK3 and examined the expression of ERα in the presence or absence of E2 and/or Tamoxifen (Tam). As previously described, treatment with E2 (10nM) or Tam (100nM) for 24h in untransfected or CT siRNA transfected MCF7 cells resulted in a ∼40% decrease and ∼50% increase of ERα respectively (Figs 3A and 3B). Interestingly, transfection with LMTK3 siRNA led to a more pronounced ERα down-regulation (∼80%), independent of treatment (Figs 3A and 3B). Following, we investigated whether the observed LMTK3-induced ERα protein down-regulation is due to a decrease of ERα gene transcription. Therefore, qRT-PCR analysis of ERα mRNA expression upon LMTK3 siRNA treatment was performed. Our data demonstrated a significant change of ERα mRNA after LMTK3 silencing (>60%), suggesting that the mechanism of action of LMTK3 on ERα may reside at the ERα transcriptional level (Fig 3C). Similar results were also obtained after inhibiting LMTK3 expression BT474 cells (Fig 14).

### LMTK3 silencing influences FOXO3a protein levels

Based on our previous results, we decided to examine the potential effects of LMTK3 on the mRNA expression levels of different transcriptional factors that regulate ERα transcription. MCF7 cells were transfected with LMTK3 siRNA for 48h before performing qRT-PCR analysis of 3 well characterized genes involved in ERα transcription including GATA3, FoxO3a and FoxM1. Inhibition of LMTK3 did not have any significant effects on the transcription of these genes (Fig 4A). Then, we investigated whether inhibition of LMTK3 is able to influence these transcriptional factors in protein level. MCF7 cells were transfected either with CT siRNA or LMTK3 siRNA in the presence or absence of E2 (10nM) or Tam (100nM), followed by immunoblotting using GATA3, FoxO3a and FoxM1 antibodies. Whereas in untreated, CT siRNA- and LMTK3 siRNA-treated cells the GATA3 and FoxM1 protein levels remained unaffected, treatment with LMTK3 siRNA (in the presence or absence of E2 or Tam) resulted in a significant decrease (∼60%) of FoxO3a protein levels (Figs 4B and 4C).

### LMTK3 silencing affects proliferation of ERα positive breast cancer cells

Based on our results which indicate the involvement of LMTK3 in ERα signaling, we investigated whether LMTK3 has any effects on the proliferation rate of ERα positive (MCF7) and ERα negative (MDA-MB-231) BC cells. To examine this MCF7 and MDA-MB-231 cells were transfected with LMTK3 siRNA and exposed to a WST-1 cell proliferation assay. Interestingly, LMTK3 silencing resulted in a profound reduction of cell proliferation (∼60%, p < 0.001) in MCF7 cells, while the effects inMDA-MB-231 cells were less pronounces (∼15%, p > 0.05) **(****Fig 5A****).** These data suggest that the inhibition of LMTK3 may confer a significant anti-tumor effect in BCs expressing ERα. (Fig 5B) FACS/Caspase assay

### LMTK3 expression in breast cancer tissue

To assess the clinical significance of our results, LMTK3 expression was examined on a panel of 613 BC TMAs. Immunostaining analysis revealed that LMTK3 was detected predominantly in the nucleus (LMTK3-nuc) with variable cytoplasmic staining. Nuclear staining was scored based on the H-score, as weak (1-25) in 26.4% of patients, moderate (26-134) in 34.7%, and strong (135-300) in 38.8% of analyzed samples. Cytoplasmic expression was scored based on intensity as being absent = 0, weak = 1, moderate = 2, and strong = 3. Scores were dichotomised into groups: low (scores 0 and 1), and high (scores 2 and 3). High cytoplasmic LMTK3 (LMTK3-cyto) was observed in 9.5% of cases, while, 87.3% had low LMTK3-cyto levels (20 cores = 3.2% did not have LMTK3-cyto score recorded). The specificity of the antibody was confirmed by the pre-incubation with the competing peptide **(****Figs 6A and 6B****).**

### Nuclear LMTK3 and correlation with relevant tumor biomarkers

Nuclear LMTK3 expression directly correlated with high tumor grade (p < 0.001) (Supplementary Fig 6). High LMTK3 expression was observed in 57.6% of grade 3 tumors, while grade 1 tumors had high LMTK3 levels in only 20% of the cases. Positive correlations were with two out of three components of the tumor grade, namely, pleomorphism and mitotic index (p < 0.001). LMTK3 expression directly correlated with the tumor proliferation marker Ki67 (p = 0.002) and NPI **(****Fig 14****).** Patients with moderate and strong LMTK3 expression belonged to the group with NPI of >3.4 (MPG and PPG) in 70% and 73% of cases, respectively (p < 0.001). Although we did not observe significant correlation with the number of positive lymph nodes (LNs) (p = 0.308), interestingly all patients with >9 invaded LNs had tumors expressing high levels of LMTK3-nuc **(****Fig 14****).**

LMTK3 overexpression was negatively associated with the expression of hormone receptors, ERα and PgR (p < 0.01). Cases with strong LMTK3-nuc had absence of ERα in 32.31%, vs. 17.76% of weak LMTK3-nuc. In the entire dataset, 49.6% of cases co-expressed LMTK3-nuc (moderate and strong expression combined) and ERα. Tumors over-expressing Her2 were more likely to be LMTK3 positive (p = 0.006). Loss of BRCA1 was more frequent in strong LMTK3-nuc BCs (p = 0.023). We did not observe significant correlation with lymphivascular invasion, patient age, CK5/6, CK7/8, CK18 or E-cadherin expression. Correlation of LMTK3 expression with tumor size was borderline significant (p = 0.052). However, there was a significant up-regulation of LMTK3 in invasive ductal carcinomas in the investigated cohort (p < 0.001), where 44.8% has strong, 35.2% moderate, and 20% weak LMTK3 levels. Patients' characteristics are summarized in **Table 3.**

**Table 3. Patients' characteristics and tumour biomarkers and correlation to LMTK3 nuclear expression**

| Variable/Biomarker | LMTK3 nuclear expression | | | p-value |
|---|---|---|---|---|
| | Weak (%) | Moderate (%) | Strong (%) | |
| *Grade* | | | | |
| 1 | 41 (25.6) | 27 (12.7) | 35 (14.8) | |
| 2 | 67 (41.8) | 72 (33.9) | 65 (27.4) | <0.001 |
| 3 | 52 (32.6) | 113 (53.4) | 13T (5T.8) | |

| *Tubules* | | | | |
|---|---|---|---|---|
| 1 | 11 (7.0) | 8 (3.8) | 12 (5.2) | |
| 2 | 61 (38.8) | 61 (29.3) | 75 (32.6) | 0.162 |
| 3 | 85 (54.2) | 139 (66.9) | 143 (62.2) | |

| *Mitosis* | | | | |
|---|---|---|---|---|
| 1 | 87 (55.4) | 59 (28.4) | 48 (20.9) | |
| 2 | 22 (14.0) | 48 (23.1) | 101 (43.9) | <0.001 |
| 3 | 48 (30.6) | 51 (48.5) | 116 (35.2) | |

| *Pleomorphism* | | | | |
|---|---|---|---|---|
| 1 | 7 (4.5) | 3 (1.4) | 3 (1.3) | |
| 2 | 84 (53.5) | 81 (38.9) | 82 (35.7) | <0.001 |
| 3 | 66 (42.0) | 124 (59.7) | 145 (63.0) | |

| *No. pos. LNs* | | | | |
|---|---|---|---|---|
| 0 | 90 (61.6) | 120 (62.5) | 123 (60.0) | |
| 1-3 | 51 (34.9) | 53 (27.6) | 66 (32.2) | 0.308 |
| 4-9 | 5 (3.4) | 15 (7.8) | 13 (6.3) | |
| >9 | 0 (0) | 4 (2.1) | 3 (1.5) | |

| *Stage* | | | | |
|---|---|---|---|---|
| 1 | 102 (63.3) | 132 (62.3) | 146 (61.3) | |
| 2 | 53 (32.9) | 55 (25.9) | 72 (30.2) | 0.067 |
| 3 | 6 (3.8) | 25 (11.8) | 20 (8.5) | |

| *Tumour type* | | | | |
|---|---|---|---|---|
| Invasive Ductal | 61 (16.6) | 141 (38.5) | 164 (44.9) | 0.002 |
| Other | 101 (40.9) | 72 (29.1) | 74 (30.0) | |

| *Tumor size* | | | | |
|---|---|---|---|---|
| < 2 cm | 108 (67.5) | 133 (62.8) | 132 (55.70 | 0.052 |
| ≥ 2 cm | 52 (32.5) | 79 (37.2) | 105 (44.3) | |

| *Age* | | | | |
|---|---|---|---|---|
| <50 | 52 (32.1) | 69 (32.4) | 88 (37.0) | 0.487 |
| ≥50 | 110 (67.9) | 144 (67.7) | 150 (63.0) | |

| *LVI* | | | | |
|---|---|---|---|---|
| Definite | 46 (28.9) | 79 (25.3) | 17 (24.6) | |
| Negative | 96 (60.4) | 111 (35.5) | 22 (31.9) | 0.312 |
| Probable | 17 (10.7) | 122 (39.2) | 30 (43.5) | |

| Variable/Biomarker | LMTK3 nuclear expression | | | p-value |
|---|---|---|---|---|
| | Weak (%) | Moderate (%) | Strong (%) | |
| *NPI* | | | | |
| GPG | 72 (45.0) | 61 (28.8) | 62 (26.2) | |
| MPG | 72 (45.0) | 119 (56.1) | 140 (59.0) | 0.001 |
| PPG | 16 (10.0) | 32 (15.1) | 35 (14.8) | |

| Ki67 | | | | |
|---|---|---|---|---|
| Negative | 67 (51.1) | 56 (32.7) | 67 (34.4) | 0.002 |
| Positive | 64 (48.9) | 115 (67.3) | 128 (65.6) | |

| *Estrogen receptor* | | | | |
|---|---|---|---|---|
| Negative | 27 (17.8) | 50 (25.1) | 74 (32.3) | 0.006 |
| Positive | 125 (82.2) | 149 (74.9) | 155 (67.7) | |

| *Progesterone receptor* | | | | |
|---|---|---|---|---|
| Negative | 48 (31.8) | 72 (36.4) | 110 (48.5) | 0.002 |
| Positive | 103 (68.2) | 149 (63.6) | 117 (51.5) | |

| *Her2 receptor* | | | | |
|---|---|---|---|---|
| Negative | 141 (93.4 | 186 (90.3) | 195 (83.3) | 0.006 |
| Positive | 10 (6.6) | 20 (9.7) | 39 (16.7) | |

| *Cytokeratin 5*/*6* | | | | |
|---|---|---|---|---|
| Negative | 132 (86.3) | 173 (85.2) | 191 (83.0) | 0.664 |
| Positive | 21 (13.7) | 30 (14.8) | 39 (17.0) | |

| *Cytokeratin 7*/*8* | | | | |
|---|---|---|---|---|
| Negative | 2 (1.3) | 4 (2.0) | 1 (0.5) | 0.342 |
| Positive | 152 (98.7) | 200 (98.0) | 228 (99.5) | |

| *Cytokeratin 18* | | | | |
|---|---|---|---|---|
| Negative | 15 (10.6) | 16 (8.4) | 24 (11.2) | |
| 0.629 | | | | |
| Positive | 126 (89.4) | 174 (91.6) | 190 (88.8) | |

| *E-cadherin* | | | | |
|---|---|---|---|---|
| Negative | 37 (24.3) | 32 (16.0) | 38 (19.7) | 0.085 |
| Positive | 115 (75.6) | 168 (84.0) | 193 (80.3) | |

| *BRCA1* | | | | |
|---|---|---|---|---|
| 0 | T9 (62.7) | 103 (60.2) | 133 (66.2) | |
| 1 | 34 (27.0) | 57 (33.3) | 64 (31.8) | 0.023 |
| 2 | 13 (10.3) | 11 (6.5) | 4 (2.0) | |

### Predictive and prognostic value of nuclear LMTK3 in breast cancer

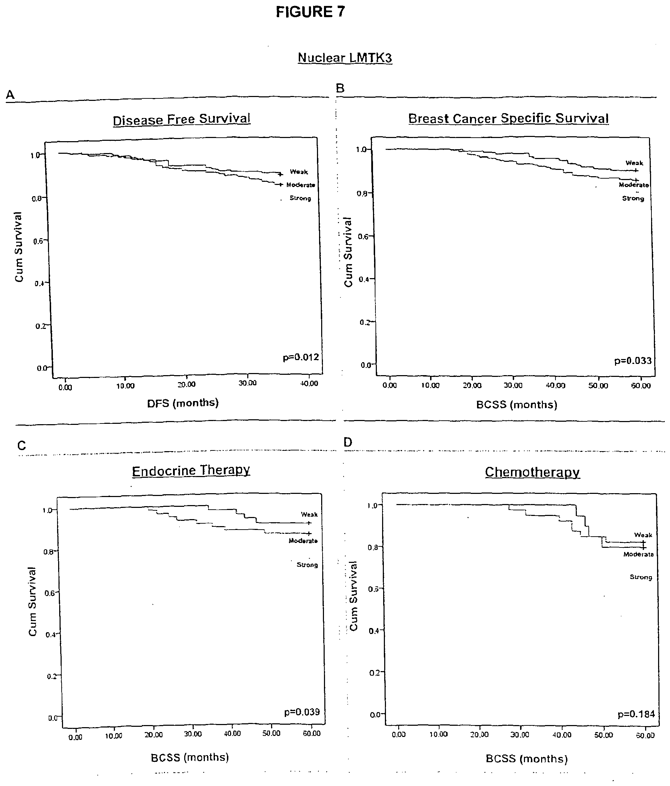

Univariate analysis showed that patients with high LMTK3-nuc expression had shorter DFS (p = 0.012), and shorter BCSS (p = 0.033) **(****Figs 7A and 7B****).** Strong LMTK3-nuc cases showed a 76.8% 3-year DFS, compared to 88.5% in the LMTK3-nuc weak tumors. Patients with strong LMTK3-nuc showed a 72.5% 5-year BCSS, compared to 80.1 % in weak nuclear LMTK3 cases. Cox proportional hazards analysis revealed that LMTK3-nuc was not a predictor of shorter DFS (p = 0.523) or BCSS (p = 0.097), independent of tumor grade, stage and size. Importantly, we found that LMTK3-nuc was able to predict outcome to adjuvant hormonal treatment (p = 0.039). Patients with strong nuclear LMTK3 tumors receiving hormonal therapy had 74.6% BCSS at 5 years, compared to 91.5% 5 year BCSS in LMTK3-nuc weak cases. LMTK3-nuc did not predict response to adjuvant chemotherapy (p = 0.184) **(****Figs 7C and 7D****).**

### Cytoplasmic LMTK3 expression in breast cancer and correlation with relevant tumor biomarkers

Upon correlations with relevant biomarkers, we found that high LMTK3-cyto correlated directly with high tumor grade (p < 0.001). High LMTK3-cyto tumors were grade 3 in 84% of cases vs. 46.9% in the low LMTK3-cyto group. All three components of grade correlated significantly with high LMTK3-cyto namely, tubular formation (p = 0.012), pleomorphism and mitotic index (p < 0.001). Tumors with high LMTK3-cyto were more likely to be ERα, PgR negative (p < 0.001) and negative for the luminal marker, CK18 (p = 0.007). Conversely, basal-like tumors expressing high CK5/6, also had high cytoplasmic expression of LMTK3 (p = 0.031). No significance was rendered when we correlated LMTK3cyto with tumor stage and number of positive LNs, patient age, tumor size, lymphovascular invasion, Ki67, Her2 or E-cadherin (p > 0.05). Patients' characteristics are summarized in **Table 4.**

**Table 4. Patients' characteristics and tumour biomarkers and correlation to LMTK3 cytoplasmic expression**

| Variable/Biomarker | LMTK3 cytoplasmic Low (%) | expression High (%) | p-value |
|---|---|---|---|
| *Grade* | | | |
| 1 | 95 (17.9) | 4 (6.9) | |
| 2 | 187 (35.2) | 9 (15.5) | <0.001 |
| 3 | 249 (46.9) | 45 (77.6) | |

| *Tubules* | | | |
|---|---|---|---|
| 1 | 29 (5.6) | 0 (0.0) | |
| 2 | 178 (34.3) | 12 (21.0) | <0.001 |
| 3 | 312 (60.1) | 45 (79.0) | |

| *Mitosis* | | | |
|---|---|---|---|
| 1 | 193 (37.2) | 10 (17.5) | |
| 2 | 111 (21.4) | 6 (10.5) | <0.001 |
| 3 | 215 (41.4) | 41 (72.0) | |

| *Pleomorphism* | | | |
|---|---|---|---|
| 1 | 11 (2.1) | 1 (1.8) | |
| 2 | 228 (43.9) | 8 (14.0) | <0.001 |
| 3 | 280 (54.0) | 48 (84.2) | |

| *No. pos. LNs* | | | |
|---|---|---|---|
| 0 | 290 (60.9) | 36 (67.9) | |
| 1-3 | 153 (32.1) | 12 (22.6) | 0.535 |
| 4-9 | 27 (5.7) | 4 (7.5) | |
| >9 | 6 (1.3) | 1 (1.9) | |

| *Stage* | | | |
|---|---|---|---|
| 1 | 333 (62.5) | 36 (62.1) | |
| 2 | 160 (30.0) | 14 (24.1) | 0.206 |
| 3 | 40 (7.5) | 8 (13.8) | |

| *Tumour type* | | | |
|---|---|---|---|
| Invasive Ductal | 301 (56.3) | 50 (86.2) | 0.001 |
| Other | 234 (43.7) | 8 (13.8) | |

| *Tumour size* | | | |
|---|---|---|---|
| < 2 cm | 326 (61.4) | 37(63.8) | 0.418 |
| ≥ 2 cm | 205 (38.6) | 21 (36.2) | |

| *Age* | | | |
|---|---|---|---|
| <50 | 177 (33.1) | 25 (43.1) | 0.145 |
| ≥50 | 358 (66.9) | 33 (56.9) | |

| *LVI* | | | |
|---|---|---|---|
| Definite | 182 (34.3) | 19 (33.3) | |
| Negative | 285 (53.T) | 35 (61.4) | 0.357 |
| Probable | 64 (12.0) | 3 (5.3) | |

| Variable/Biomarker | LMTK3 cytoplasmic expression | | p-value |
|---|---|---|---|
| | Low (%) | High (%) | |
| *NPI* | | | |
| GPG | 180 (33.9) | 11 (18.9) | |
| MPG | 284 (53.5) | 34 (58.6) | 0.023 |
| PPG | 67 (12.6) | 13 (22.5) | |

| *Ki67* | | | |
|---|---|---|---|
| Negative | 174 (39.8) | 11 (26.8) | 0.069 |
| Positive | 263 (60.2) | 30 (73.2) | |

| *Estrogen receptor* | | | |
|---|---|---|---|
| Negative | 115 (22.9) | 30 (52.6) | <0.001 |
| Positive | 388 (77.1) | 27 (47.4) | |

| *Progesterone receptor* | | | |
|---|---|---|---|
| Negative | 182 (36.5) | 39 (68.4) | <0.001 |
| Positive | 317 (63.5) | 18 (31.6) | |

| *Her2 receptor* | | | |
|---|---|---|---|
| Negative | 457 (88.9) | 47 (81.0) | 0.067 |
| Positive | 57 (11.1) | 11 (19.0) | |

| *Cytokeratin 5*/*6* | | | |
|---|---|---|---|
| Negative | 438 (86.1) | 43 (74.1) | 0.018 |
| Positive | 71 (13.9) | 15 (25.9) | |

| *Cytokeratin 7*/*8* | | | |
|---|---|---|---|
| Negative | 6 (1.2) | 1 (1.7) | 0.531 |
| Positive | 504 (98.8) | 57 (98.3) | |

| *Cytokeratin 18* | | | |
|---|---|---|---|
| Negative | 41 (8.7) | 12 (21.8) | 0.005 |
| Positive | 432 (91.3) | 43 (78.2) | |

| *E-cadherin* | | | |
|---|---|---|---|
| Negative | 92 (18.2) | 12 (20.7) | 0.376 |
| Positive | 414 (81.8) | 46 (79.3) | |

| *BRCA1* | | | |
|---|---|---|---|
| 0 | 278 (64.7) | 30 (58.8) | |
| 1 | 127 (29.5) | 20 (39.2) | 0.235 |
| 2 | 25 (5.8) | 1 (2.0) | |

### Predictive and prognostic value of cytoplasmic LMTK3 in breast cancer

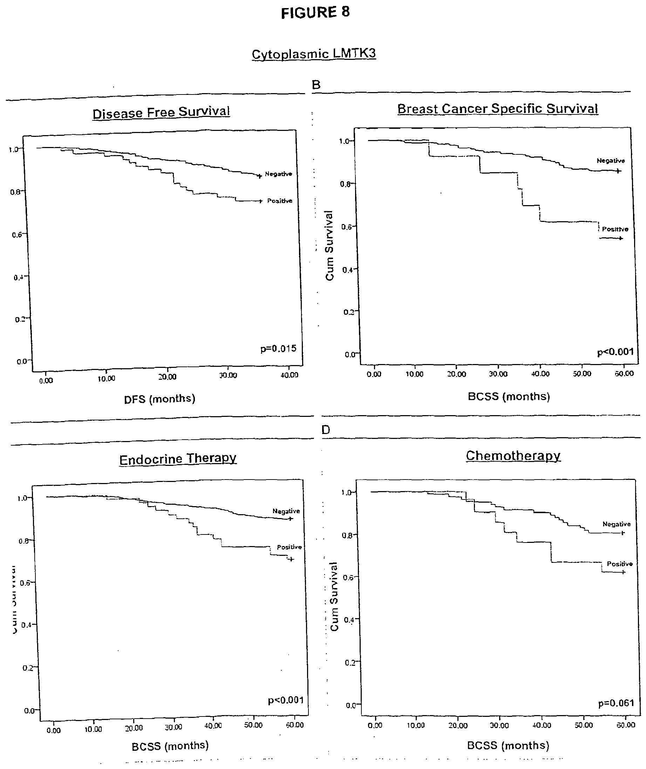

Kaplan-Meier survival analysis indicated that high LMTK3-cyto was a predictor of worse DFS (p = 0.015) and BCSS (p < 0.001) **(****Figs 8A and 8B****).** In the high LMTK3-cyto cohort, DFS at 3 years was 72.4% compared to 84.2% in the LMTK3-cyto low group. Similarly, BCSS at 5 years was 68% in patients with high LMTK3-cyto expression, while 87.3% of women were alive at 5 years if their tumors expressed low LMTK3-cyto levels. In a multivariate model of LMTK3-cyto with tumor grade, stage and size, the impact on DFS (p = 0.622) or BCSS (p =0.076) was not preserved. However, LMTK3-cyto, like its nuclear counterpart, was able to predict outcome to hormonal adjuvant treatment. In the patient cohort eligible for and receiving hormonal therapy, only 53.8% were alive at 5 years if their tumors exhibited high LMTK3-cyto, vs. a much better outcome and survival of 85.5% at 5 years, in LMTK3-cyto low cases (p < 0.001). LMTK3-cyto did not predict outcome to chemotherapy (p = 0.061) **(****Figs 8C and 8D****).**

### Discussion

As previously described, ERα is expressed in ∼70% of BC patients and is correlated with a better overall and disease free survival (29). Even though traditional endocrine therapies have been very effective, resistance to these therapies and recurrence still retain BC deaths in high rates among all cancer deaths worldwide (30). Therefore, understanding and elucidating the molecular mechanisms and intracellular signaling involved in ERα regulation is considered imperative in order to develop more successful strategies for the treatment of BC.

As protein kinases have been involved in the progression of different types of cancer including BC, it is not surprising that they represent an important therapeutic target for drug development (31). Moreover, phosphorylation of ERα, is a well-described and essential post-translational modification event that regulates ERα (32). In the present study, we performed a high-throughput kinome-siRNA screening to discover new kinases that modulate ERα transcriptional activity, followed by protein expression analysis of targeted kinases in BC clinical samples to examine their clinical significance.

We have identified LMTK3 as a novel kinase that is implicated in ERα transcriptional activity and in the expression of ERα-regulated genes. In addition, we demonstrated that LMTK3 is involved in the regulation of ERα mRNA and protein levels via signaling through FoxO3a. Furthermore, we showed that suppression of LMTK3 in ERα positive BC cell line (MCF7) results in cell death while does not have any effects in the ERα negative BC cell line MDA-MB-231. Finally, we report the expression pattern of LMTK3 in BC and correlate it with various tumour biomarkers and clinical outcome, demonstrating the significance of our findings.

We examined the effects of all human kinases on the expression level of the ERα-regulated pS2 gene, after silencing each kinase individually using RNAi technique. Among the newly discovered hits, we identified LMTK3 to have the most significant effects in different E2-responsive genes including PgR and GREB1; in addition, silencing of LMTK3 resulted in a ∼50% reduction in the E2 response of an integrated ERE reporter gene in MELN cells. Furthermore, treatment of two different breast cancer cell lines (MCF7 and BT474) with LMTK3 siRNA resulted in a decrease of both ERα mRNA and protein levels, independently of ligand treatment (E2 or Tam). As it is already known that the transcription of ERα is regulated by various transcriptional factors (i.e. GATA3, FoXO3a, FoXM1) we examined the effects of LMTK3 inhibition on these factors. We discovered that FoXO3a protein, but not mRNA levels, are also down-regulated upon treatment with LMTK3 siRNA, implying the involvement of FoXO3a.

Biological and histological markers of BC are used to assess disease aggressiveness and the likelihood of response to available treatments, thereby driving both clinical decision-making and research. Leading biological markers for BC are ERα, PgR and Her2. Further elucidation of molecular profiles and novel biomarkers is paramount for the development of targeted, personalized therapies.

Our work reports the pattern and clinical implications of LMTK3 expression in BC. Immunostaining of a large, well-characterized cohort of BC patients revealed over-expression of LMTK3 in BC, in the nucleus and to a variable degree in the cytoplasm. Elevated levels of this protein in either of the cellular compartments yielded significant correlations with common tumor biomarkers and patho-histological parameters.

Both LMTK3 counterparts, nuclear and cytoplasmic, correlated significantly with high tumor grade and a high mitotic index (33). Furthermore, strong LMTK3-nuc correlated with increased Ki67 levels (34) and moderate or poor prognosis according to the NPI (35). Our results also demonstrate that strong LMTK3-nuc levels are coordinately regulated with Her2 (36) over-expression and loss of BRCA1 (37), both markers of aggressive disease. In the analysed dataset (n = 614), 49.6% of the cases co-expressed LMTK3-nuc and ERα, even though overall correlation of both LMTK3-nuc and LMTK3-cyto with ERα was inverse. Interestingly, LMTK3-cyto was found to be a marker inherent to basal-like tumors, as it co-expressed with basal cytokeratins 5/6 and inversely correlated with the luminal cytokeratin 18. Collectively, our data indicate that LMTK3 is over-expressed in BC with invasive properties and high propensity for the development of metastatic disease.

Most importantly, we observed that over-expression of both LMTK3-nuc and LMTK3-cyto was highly indicative of worse patient survival, both disease-free and overall in the general dataset. Kaplan-Meier survival analysis of the subsets of patients receiving hormonal therapy, further indicated that patients with tumors over-expressing LMTK3 are less likely to benefit from hormonal treatment compared to BC patients with LMTK3 weak tumors, as judged by shorter breast cancer specific survival. This finding, once again, emphasizes the need for understanding molecular profiles underlying clinical behavior of BC in order to design more tailored and effective therapies.

Collectively, our results indicate that strong expression of both nuclear and cytoplasmic LMTK3 are found predominantly in BCs exhibiting biomarkers of poor prognosis. Strong LMTK3-nuc and high LMTK3-cyto confer unfavorable response to adjuvant hormonal treatment, and most importantly, carry a prognostic value and indicate propensity for early disease relapse and worse overall survival. Taking into account our *in vitro* data demonstrating the anti-proliferative effects of silencing LMTK3 in BC cells together with these *in vivo* observations, we conclude that LMTK3 is not only a novel prognostic and a predictive marker in BC but most importantly, a novel therapeutic target.

### Example 2: assay formats suitable for compound screening

Protein kinase screening assay formats known in the art may be used, adapted in view of the identification of LMTK3 as an activator of ERα transcriptional activity.

Examples of assays suitable for assessing LMTK3 nucleic acid or protein level will be well know to those skilled in the art. Such assays may, for example, make use of techniques for assessing LMTK3 nucleic acid or protein levels described herein in relation to assessing LMTK3 levels in a sample from a patient. Typically a nucleic acid that selectively hybridises to an LMTK3 RNA or a molecule (for example an antibody) that selectively binds to an LMTK3 polypeptide may be used. Examples of assays suitable for assessing LMTK3 activity will also be well known to those skilled in the art. For example LMTK3 activity may be assessed by using a reporter gene system based on the effect of LMTK3 on ERα, for example on ERα transcriptional activity on genes regulated by ERα, for example pS2, PgR and GREB1.

Suitable assay formats may include HTRF (Homogeneous Time-Resolved Fluorescence) assays (for example as offered by Cisbio), Lanthascreen (Invitrogen) and KlnaseGLO (Promega). Screening assays which are capable of high throughput operation may be used. Focused kinase inhibitor compound libraries may be used to complete an initial compound screen. Thus, the test compound may be a compound already identified as a protein kinase inhibitor. These may be used to identify compound 'hits' (typically non-selective, low potency chemical start points). Available sequence knowledge, structural predictions and known kinase ligands may also be used to virtually screen commercially available compounds for novel compound hits. Hit compounds may be iteratively modified using classical medicinal chemistry strategies to derive a compound series which has optimal inhibitory potency against the target kinase, whilst limiting the effects on other important kinase families. This optimisation process is expected to lead to a candidate compound for testing in *in vivo* systems, for example BC xenograft studies in mice and hence as a potential pre-clinical candidate. Pre-clinical candidates will also require DMPK (Drug Metabolism and Pharmacokinetics), toxicology, safety and formulation studies prior to clinical testing.

### Comparative Example 1: LMTK3 polymorphisms

### Results

### LMTK3 rs8108419 polymorphism and DFS, OS in breast cancer patients

Genotyping for LMTK3 rs8108419 was successful in 234 (99%) of 237 cases. In the other 3 patients (1%) genotyping was not successful, because of limited quantity and quality of extracted genomic DNA. Sixty-six percent (155/234) of patients were homozygous for LMTK3 rs8108419 GG genotype, 27% (64/234) were heterozygous (GA), and 7% (15/234) were homozygous for the AA genotype. The LMTK3 rs8108419 polymorphism showed a significant association with TTR (Time To Relapse) adjusted by tumor sizes, lymph nodes, and c-erbB2 status. Patients with the LMTK3 rs8108419 AA genotype had a median TTR of 4.8 years (95% CI: 3.0 to 8.1 years), compared to 7.5 years (95% CI: 6.3-9.7 years) in patients with combined heterozygous GA and homozygous GG genotypes (p = 0.038, two- side Wald test, **Table 5**). There is no statistically significant association between this polymorphism and OS (p = 0.31, two-side Wald test).

### LMTK3 rs9989661 polymorphism and DFS, OS in breast cancer patients

Genotyping for LMTK3 rs9989661 was successful in 235 (99%) of 237 cases. In the other 2 patient (1%) genotyping was not successful, because of limited quantity and quality of extracted genomic DNA. Ninety-two percent (215/235) of patients were homozygous for the LMTK3 rs9989661 A/A genotype, 7% (17/235) were heterozygous (A/G), and 1% (3/235) was homozygous for the LMTK3 rs9989661 G/G genotype. The LMTK3 rs9989661 polymorphism showed a significant association with TTR. Patients with the LMTK3 rs9989661 TT genotype had a median TTR of 7.6 years (95% CI: 6.3 to 9.7 years), compared to 5.4 years (95% CI: 2.1 to 8+ years) for those with heterozygous CT and homozygous CC genotypes. (p = 0.042, two-side Wald test, **Table 5**). Patients with LMTK3 rs9989661 polymorphism were also significantly associated with overall survival. Patients with the LMTK3 rs9989661 TT genotype had a median OS of 8.4 years (95% CI: 7.4 to 10.0+ years), compared to 4.4 years (95% CI: 3.0 to 8.8 years) for those with heterozygous CT and homozygous CC genotypes. (p = 0.039, two-side Wald test, **Table 5**).

### Multivariable analysis of LMTK3 rs8108419 and LMTK3 rs9989661 with DFS and OS

In a combined analysis, there was a statistically significant relationship between the two polymorphisms and TTR. Patients harbouring favourable alleles of these 2 polymorphisms (LMTK3 rs8108419 GG or AG and LMTK3 rs9989661 TT) were at lowest risk to develop tumor recurrence (RR = 1; reference) compared to patients carrying unfavourable alleles (LMTK3 rs8108419 AA and LMTK3 rs9989661 CT or CC), who were at greater risk to develop tumor recurrence (RR=2.44; CI: 1.40-4.25) (adjusted p = 0.002; **Table 5**). OS was also significantly associated with combined analysis of these 2 polymorphisms and stratified by tumor size, lymph nodes, and c-erbB2 status. Patients with favourable alleles of these 2 polymorphisms (LMTK3 rs8108419 GG or AG and LMTK3 rs9989661 TT) were at lowest risk of time to death (RR = 1, reference), compared to patients carrying unfavourable alleles (LMTK3 rs8108419 AA and LMTK3 rs9989661 CT or CC), who were at greater risk to death (RR=2.06; CI: 1.14-3.73) (p = 0.017; **Table 5**) **(****Figs 16A and 16B****).**

### Materials and Methods

### Candidate polymorphisms

Candidate LMTK3 polymorphisms were chosen with the assistance of the Ensembl program using two main criteria: 1) That the polymorphism has some degree of likelihood to alter the function of the gene in a biological relevant manner. Rs8108419 polymorphism located in intron 2 of the LMTK3 gene, rs9989661 polymorphism located in intron 15 of the LMTK3 gene. Intron polymorphisms can change gene transcription levels by alternative splicing or by affecting binding of a transcription factor. 2) That the frequency of the polymorphism is sufficient enough that its impact in clinical outcome would be meaningful on a population level (above 10% allele frequency).

### LMTK3 rs8108419 and rs9989661 genotyping

Tissue specimens from primary breast cancer tumors were collected and genomic DNA was extracted using the QIAamp kit (Qiagen). LMTK3 polymorphisms (rs8108419 and rs9989661) were tested using polymerase chain reaction restriction fragment length polymorphism (PCR-RFLP) technique. Briefly, forward primer 5'-ATTCCACCACTCCCTCCAG-3' and reverse primer 5'-GACCCTGCAGTGCCTC AC-3' for rs8108419 and forward primer 5'-GGGCCTTCCCAAGTGGTT-3' and reverse primer 5'-ATCCAAGCCTGGGGTGAG-3' for rs9989661 were used for PCR amplification, PCR products were digested by restriction enzyme BsrD1(rs8108419) or Btsc1(rs9989661)( (New England Biolab, Massachusetts, USA), and alleles were separated on 4% NuSieve ethidium bromide-stained agarose gel.

### Statistical analysis for polymorphisms

DFS and OS were the primary endpoints in the analysis. DFS was defined as the period from the date of initial diagnosis to the date of the first documented relapse or death, and OS was defined as the time from the initial diagnosis to death. DFS time was censored at the date of last follow-up if patients were still relapse-free and alive, and OS was censored at the time when patients were alive. The associations between 2 LMTK polymorphisms (rs8108419 and rs9989661) and DFS and OS were examined using Kaplan-Meier curves and Cox proportional hazards regression model. A forward stepwise Cox regression model was conducted to select baseline patient demographic and tumor characteristics to be included in the multivariate analyses of 2 LMTK polymorphisms and clinical outcome. Chi-square tests were used to examine the associations between baseline tumor characteristics and 2 LMTK polymorphisms. All testes were 2-sided at a 0.05 significance level and performed using the SAS statistical package version 9.2.

**Table 5. LMTK3 polymorphisms and time to recurrence and overall survival in patients with breast cancer**

| | | Disease Free Survival | | | | Overall survival | |
|---|---|---|---|---|---|---|---|
| | *N* | Median time to relapse, yrs (95% CI) | HR (95% CI) | *P* value t | Median time to relapse, yrs (95% CI) | HR (95% CI) | *P* value † |
| **LMTK3 rs8108419** | | | | 0.038 | | | 0.31 |
| G/G* | 155 | 7.5 (6.3, 9.7) | 1 (Reference) | | 8.4 (7.4, 8.8) | 1 (Reference) | |
| A/G* | 64 | | | | | | |
| A/A | 15 | 4.8 (3.0, 8.1) | 2.221 (1.043, 4.728) | | 6.1 (1.7, 8.5+) | 1.563 (0.663, 3.685) | |
| **LMTK3 rs9989661** | | | | 0.042 | | | 0.039 |
| T/T | 215 | 7.6 (6.3, 9.7) | 1 (Reference) | | 8.4 (7.4, 10.0+) | 1 (Reference) | |
| C/T* | 17 | 5.4 (2.1, 8.0+) | 2.036 (1.028, 4.033) | | 4.4 (3.0, 8.8) | 2.095 (1.039, 4.221) | |
| C/C* | 3 | | | | | | |
| **Combined 2 polymorphisms** | | | | 0.002 | | | 0.017 |
| G/G or AG and TT | 198 | 7.6 (6.4, 9.7) | 1 (Reference) | | 8.4 (7.4, 10.0+) | 1 (Reference) | |
| AA, C/T or C/C | 35 | 4.8 (3.0, 8.1) | 2.435 (1.396, 4.247) | | 5.9 (4.3, 8.8) | 2.062 (1.140, 3.730) | |

### References

1. Garcia, M., Jemal, A., Ward, E., Center, M., Hao, Y., Siegel, R. and Thun, M. (2007). American Cancer Society, Atlanta.
2. Stierer, M., Rosen, H., Weber, R., Hanak, H., Spona, J. and Tuchler, H. (1993) Immunohistochemical and biochemical measurement of estrogen and progesterone receptors in primary breast cancer. Correlation of histopathology and prognostic factors. Annals of surgery, 218, 13-21.
3. Cordera, F. and Jordan, V.C. (2006) Steroid receptors and their role in the biology and control of breast cancer growth. Semin Oncol, 33, 631-641.
4. Holst, F., Stahl, P.R., Ruiz, C., Hellwinkel, O., Jehan, Z., Wendland, M., Lebeau, A., Terracciano, L., Al-Kuraya, K., Janicke, F. et al. (2007) Estrogen receptor alpha (ESR1) gene amplification is frequent in breast cancer. Nature genetics, 39, 655-660.
5. Khan, S.A., Rogers, M.A., Khurana, K.K., Meguid, M.M. and Numann, P.J. (1998) Estrogen receptor expression in benign breast epithelium and breast cancer risk. J Natl Cancer Inst, 90, 37-42.
6. Frech, M.S., Halama, E.D., Tilli, M.T., Singh, B., Gunther, E.J., Chodosh, L.A., Flaws, J.A. and Furth, P.A. (2005) Deregulated estrogen receptor alpha expression in mammary epithelial cells of transgenic mice results in the development of ductal carcinoma in situ. Cancer research, 65, 681-685.
7. Frasor, J., Weaver, A., Pradhan, M., Dai, Y., Miller, L.D., Lin, C.Y. and Stanculescu, A. (2009) Positive cross-talk between estrogen receptor and NF-kappaB in breast cancer. Cancer research, 69, 8918-8925.
8. Fowler, A.M. and Alarid, E.T. (2007) Amping up estrogen receptors in breast cancer. Breast Cancer Res, 9, 305.
9. Ali, S., Metzger, D., Bornert, J.M. and Chambon, P. (1993) Modulation of transcriptional activation by ligand-dependent phosphorylation of the human oestrogen receptor A/B region. The EMBO journal, 12, 1153-1160.
10. Williams, C.C., Basu, A., El-Gharbawy, A., Carrier, L.M., Smith, C.L. and Rowan, B.G. (2009) Identification of four novel phosphorylation sites in estrogen receptor alpha: impact on receptor-dependent gene expression and phosphorylation by protein kinase CK2. BMC biochemistry, 10, 36.
11. Yi, P., Feng, Q., Amazit, L., Lonard, D.M., Tsai, S.Y., Tsai, M.J. and O'Malley, B.W. (2008) Atypical protein kinase C regulates dual pathways for degradation of the oncogenic coactivator SRC-3/AIB1. Molecular cell, 29, 465-476.
12. Grisouard, J., Medunjanin, S., Hermani, A., Shukla, A. and Mayer, D. (2007) Glycogen synthase kinase-3 protects estrogen receptor alpha from proteasomal degradation and is required for full transcriptional activity of the receptor. Molecular endocrinology (Baltimore, Md, 21, 2427-2439.
13. Giamas, G., Castellano, L., Feng, Q., Knippschild, U., Jacob, J., Thomas, R.S., Coombes, R.C., Smith, C.L., Jiao, L.R. and Stebbing, J. (2009) CK1delta modulates the transcriptional activity of ERalpha via AlB1 in an estrogen-dependent manner and regulates ERalpha-AlB1 interactions. Nucleic acids research, 37, 3110-3123.
14. Skliris, G.P., Rowan, B.G., AI-Dhaheri, M., Williams, C., Troup, S., Begic, S., Parisien, M., Watson, P.H. and Murphy, L.C. (2009) Immunohistochemical validation of multiple phospho-specific epitopes for estrogen receptor alpha (ERalpha) in tissue microarrays of ERalpha positive human breast carcinomas. Breast cancer research and treatment, 118, 443-453.
15. Heldring, N., Pike, A., Andersson, S., Matthews, J., Cheng, G., Hartman, J., Tujague, M., Strom, A., Treuter, E., Warner, M. et al. (2007) Estrogen receptors: how do they signal and what are their targets. Physiological reviews, 87, 905-931.
16. Eeckhoute, J., Keeton, E.K., Lupien, M., Krum, S.A., Carroll, J.S. and Brown, M. (2007) Positive cross-regulatory loop ties GATA-3 to estrogen receptor alpha expression in breast cancer. Cancer research, 67, 6477-6483.
17. Madureira, P.A., Varshochi, R., Constantinidou, D., Francis, R.E., Coombes, R.C., Yao, K.M. and Lam, E.W. (2006) The Forkhead box M1 protein regulates the transcription of the estrogen receptor alpha in breast cancer cells. The Journal of biological chemistry, 281, 25167-25176.
18. Guo, S. and Sonenshein, G.E. (2004) Forkhead box transcription factor FOXO3a regulates estrogen receptor alpha expression and is repressed by the Her-2/neu/phosphatidylinositol 3-kinase/Akt signaling pathway. Molecular and cellular biology, 24, 8681-8690.
19. Morelli, C., Lanzino, M., Garofalo, C., Maris, P., Brunelli, E., Casaburi, I., Catalano, S., Bruno, R., Sisci, D. and Ando, S. Akt2 inhibition enables the forkhead transcription factor FoxO3a to have a repressive role in estrogen receptor alpha transcriptional activity in breast cancer cells. Molecular and cellular biology, 30, 857-870.
20. Zou, Y., Tsai, W.B., Cheng, C.J., Hsu, C., Chung, Y.M., Li, P.C., Lin, S.H. and Hu, M.C. (2008) Forkhead box transcription factor FOXO3a suppresses estrogen-dependent breast cancer cell proliferation and tumorigenesis. Breast Cancer Res, 10, R21.
21. Robinson, D.R., Wu, Y.M. and Lin, S.F. (2000) The protein tyrosine kinase family of the human genome. Oncogene, 19, 5548-5557.
22. Tomomura, M., Morita, N., Yoshikawa, F., Konishi, A., Akiyama, H., Furuichi, T. and Kamiguchi, H. (2007) Structural and functional analysis of the apoptosis-associated tyrosine kinase (AATYK) family. Neuroscience, 148, 510-521.
23. Inoue, T., Kon, T., Ohkura, R., Yamakawa, H., Ohara, O., Yokota, J. and Sutoh, K. (2008) BREK/LMTK2 is a myosin VI-binding protein involved in endosomal membrane trafficking. Genes Cells, 13, 483-495.
24. Kawa, S., Ito, C., Toyama, Y., Maekawa, M., Tezuka, T., Nakamura, T., Nakazawa, T., Yokoyama, K., Yoshida, N., Toshimori, K. et al. (2006) Azoospermia in mice with targeted disruption of the Brek/Lmtk2 (brain-enriched kinase/lemur tyrosine kinase 2) gene. Proceedings of the National Academy of Sciences of the United States of America, 103, 19344-19349.
25. Tyner, J.W., Deininger, M.W., Loriaux, M.M., Chang, B.H., Gotlib, J.R., Willis, S.G., Erickson, H., Kovacsovics, T., O'Hare, T., Heinrich, M.C. et al. (2009) RNAi screen for rapid therapeutic target identification in leukemia patients. Proceedings of the National Academy of Sciences of the United States of America, 106, 8695-8700.
26. Naik, S., Dothager, R.S., Marasa, J., Lewis, C.L. and Piwnica-Worms, D. (2009) Vascular Endothelial Growth Factor Receptor-1 Is Synthetic Lethal to Aberrant {beta}-Catenin Activation in Colon Cancer. Clin Cancer Res, 15, 7529-7537.
27. lorns, E., Lord, C.J., Turner, N. and Ashworth, A. (2007) Utilizing RNA interference to enhance cancer drug discovery. Nature reviews, 6, 556-568.
28. Camp, R.L., Dolled-Filhart, M. and Rimm, D.L. (2004) X-tile: a new bio-informatics tool for biomarker assessment and outcome-based cut-point optimization. Clin Cancer Res, 10, 7252-7259.
29. Carey, L.A., Perou, C.M., Livasy, C.A., Dressler, L.G., Cowan, D., Conway, K., Karaca, G., Troester, M.A., Tse, C.K., Edmiston, S. et al. (2006) Race, breast cancer subtypes, and survival in the Carolina Breast Cancer Study. JAMA, 295, 2492-2502.
30. Musgrove, E.A. and Sutherland, R.L. (2009) Biological determinants of endocrine resistance in breast cancer. Nat Rev Cancer, 9, 631-643.
31. Giamas, G., Man, Y.L., Hirner, H., Bischof, J., Kramer, K., Khan, K., Lavina Ahmed, S.S., Stebbing, J. and Knippschild, U. Kinases as targets in the treatment of solid tumors. Cell Signal.
32. Lannigan, D.A. (2003) Estrogen receptor phosphorylation. Steroids, 68, 1-9.
33. Ignatiadis, M. and Sotiriou, C. (2008) Understanding the molecular basis of histologic grade. Pathobiology, 75, 104-111.
34. Yerushalmi, R., Woods, R., Ravdin, P.M., Hayes, M.M. and Gelmon, K.A. Ki67 in breast cancer: prognostic and predictive potential. Lancet Oncol, 11, 174-183.
35. Galea, M.H., Blamey, R.W., Elston, C.E. and Ellis, I.O. (1992) The Nottingham Prognostic Index in primary breast cancer. Breast cancer research and treatment, 22, 207-219.
36. Lohrisch, C. and Piccart, M. (2001) HER2/neu as a predictive factor in breast cancer. Clin Breast Cancer, 2, 129-135; discussion 136-127.
37. Kennedy, R.D., Quinn, J.E., Johnston, P.G. and Harkin, D.P. (2002) BRCA1: mechanisms of inactivation and implications for management of patients. Lancet, 360, 1007-1014.

### Example 3. LMTK3 directly phosphorylates ERα and FoxO3a; and knockdown of LMTK3 expression overcomes Tamoxifen resistance in cancer cell lines.

Data from *in vitro* kinase assays shows that LMTK3 directly phosphorylates the estrogen receptor (ERα) and FoxO3a (see Figure 17).

We have also established that inhibition of LMTK3 can lead to reversal of what is termed 'endocrine resistance' (see Figure 18). Here, the proliferation rate of MLET5 and BTK474 cells, known to be tamoxifen resistant, was decreased on addition of tamoxifen plus siRNA to LMTK3, compared to either alone. Therefore, knockdown of LMTK3 may be able to restore sensitivity to important cancer drugs. This means that for women with relapsed disease (estrogen receptor positive women who relapse) we are developing the first targeted therapy that focuses exclusively on this large group of women. The aim here is to reverse endocrine resistance.

### Example 4: Kinome Screening for Regulators of the Estrogen Receptor Identifies LMTK3 as a New Therapeutic Target in Breast Cancer

Therapies targeting the estrogen receptor alpha (ERα) have transformed the treatment of breast cancer. However, large numbers of women relapse highlighting the need for the discovery of new regulatory targets modulating ERα pathways¹⁻⁶. A short interfering RNA (siRNA) screen identified kinases whose silencing alters the estrogen response including those previously implicated in regulating ERα activity (MAPK, AKT). Amongst the most potent regulators was LMTK3, for which a role has not previously been assigned. In contrast to other modulators of ERα activity we found evidence of Darwinian positive selection of LMTK3, an intriguing result given the unique susceptibility of humans to ERα⁺ breast cancer. LMTK3 acts by inhibiting PKC and activating AKT thereby increasing binding of FOXO3 to the ERα promoter. LMTK3 phosphorylates ERα protecting it from proteasomal degradation. Silencing of LMTK3 reduced tumor volume and bioluminescence in an orthotopic mouse model and abrogated proliferation of ERα⁺ but not ERα⁻ cells, indicative of its importance for ERα activity. In human cancers, LMTK3 levels and intronic polymorphisms were significantly associated with disease-free and overall survival and predicted response to endocrine therapies. These findings yield insights into the natural history of breast cancer in humans and reveals LMTK3 as a new target.

More than two-thirds of breast tumors express ERα³ and patients with ERα⁺ disease respond to anti-estrogens (tamoxifen), estrogen withdrawal (aromatase inhibitors) and direct targeting of the receptor (fulvestrant)¹. The introduction of these treatments has a profound impact on patient survival². However, resistance to these therapies is common. *In vitro* evidence points to the importance of ERα phosphorylation⁴, in the development of endocrine resistance^{5,6}. To identify kinases that regulate ERα activity, we performed an siRNA screen using expression of the estrogen-responsive *TFF1* gene as a readout for altered ERα activity (Figure 9). We identified 5 genes whose knockdown stimulated *TFF1* >100% and 16 genes whose knockdown reduced *TFF1* <50% (Figure 19). Two further independent replicate screenings confirmed these findings. The identified kinases MAPK3 and AKT, which phosphorylate ERα at S118 and S167 respectively⁷⁻¹¹, confirmed the screen could successfully identify regulators of estrogen-responsive gene expression.

Subsequent measurement of the expression of two other ERα regulated genes (*PGR* and *GREB1*) and two control genes (*GAPDH* and *MCL1*) was also performed. In aggregate: i) 2 of the 5 genes, whose down-regulation increased *TFF1,* also up-regulated *PGR* or *GREB1* >100% (group A) and, ii) 3 of the 13 kinases that down-regulated ERα activity were also able to down-regulate the expression levels of both *PGR* and *GREB1* >50% (group B) (Table 5.1), whereas the expression levels of *GAPDH* and *MCL1* did not change, indicating effects were E2 treatment-dependent.

To prioritize amongst these kinases, we asked whether any of the candidate proteins demonstrated evidence of positive selection. It is well established that humans and the great apes (especially so for chimpanzees [*Pan troglodytes*]*,* our closest living relatives), differ in susceptibility to epithelial neoplasms including breast cancer¹²⁻¹⁶, possibly resulting from recent evolutionary events reflected in the adaptive profile of genes that play a regulatory role here. Strikingly, of those proteins revealed to regulate ERα, only LMTK3 has been subject to recent Darwinian positive selection compared to its chimpanzee orthologue (Table 5.2). Further, LMTK3 silencing consistently inhibited the expression of estrogen-regulated genes most potently (Fig. 23 and Fig. 26), while over-expression of LMTK3 resulted in opposite effects (Fig. 23b).

LMTK (Lemur Tyrosine Kinases) represents a family of phospho-Ser/Thr/Tyr kinases¹⁷⁻¹⁹. A function has not been ascribed to LMTK3, although screens suggest a putative role in the β-catenin pathway²⁰ and leukemic cell survival²¹. We found that LMTK3 knockdown inhibited the activity of an estrogen-regulated luciferase reporter gene, which taken together with the fact that LMTK3 did not alter *GAPDH* or *MCL1* expression indicates it as a specific regulator of ERα activity (Fig. 23c-e and Figs. 24 and 25). In addition, knockdown of LMTK3 inhibited MCF-7 cell growth (Fig. 19b), accompanied by accumulation of cells in the sub-G1 phase (Fig. 19c,d). Similar results were obtained in other ERα⁺ cell lines with no effects in ERα⁻ cells (Fig. 27).

To establish the mechanisms of LMTK3 action in MCF7 cells, we next examined ERα expression. ERα protein levels were reduced 80% by LMTK3 knockdown (Fig. 20a). ERα levels were higher in the presence of proteasome inhibition (Fig. 20a) and its half-life reduced following LMTK3 knockdown whereas LMTK3 over-expression stabilized ERα (Fig. 20b). ERα levels showed an increase in ERα ubiquitination following LMTK3 knockdown (Fig. 20c). Moreover, phosphorylation of ERα by LMTK3 (Fig. 20d) rescued ERα from *in vitro* proteasome-mediated degradation (Fig. 20e), while the interaction of LMTK3 with ERα was demonstrated *in vivo* (Fig. 20f). Together, these data demonstrate that LMTK3 regulates ERα by phosphorylating and protecting it from proteasome mediated degradation.

We then observed that LMTK3 knockdown reduced *ESR1* mRNA (Fig. 20g). ERα expression is regulated by GATA3²², FOXO3²³⁻²⁵ and by FOXM1²⁶ and also by its own expression²⁷. LMTK3 siRNA did not affect mRNA levels of these genes (Fig 20g), however FOXO3 protein was reduced 70%, while FOXO3 phosphorylation sites were reduced relative to total FOXO3 levels (Fig. 20h). Over-expression of FOXO3 rescued LMTK3 siRNA-mediated ERα reduction (Fig. 20i), while ChIP confirmed that over-expression of LMTK3 increased binding of FOXO3 to the ERα promoter (Fig. 20i). AKT phosphorylates and inhibits Fox03a by promoting its degradation²⁸. No change in total AKT was observed but phosphorylated AKT increased upon LMTK3 silencing, suggesting that LMTK3 siRNA-induced FOXO3 down-regulation is regulated via AKT (Fig. 20h). Activated AKT also increased phosphorylation of ERα at S167⁷ despite decreased total ERα levels (Fig. 20h). As PKC activity has been implicated in ERα protein degradation²⁹ and in decreased synthesis of ERα gene via activation of AKT and inhibition of FOXO3³⁰, we examined effects of LMTK3 on PKC. *In vitro* kinase assays demonstrated that LMTK3 inhibits the ability of PKC to phosphorylate histone (Fig. 20j). Use of a specific phospho-(Ser) PKC substrate antibody demonstrated that LMTK3 silencing increased the ability of PKC to phosphorylate a number of substrates (Fig. 20j). In addition, inhibition of PKC partly rescued the down-regulation of ERα induced by LMTK3 silencing, while co-treatment with a PKC activator and LMTK3 siRNA resulted in further degradation of ERα (Fig. 20j). These data further imply that the effects of LMTK3 on ERα mRNA and protein levels are directly and indirectly mediated via PKC signaling.

Our findings indicate that LMTK3 plays an important role in regulating ERα activity. To confirm these data in primary breast cancer, LMTK3 immunohistochemistry (Fig. 21 a and Fig. 6c), was undertaken in 613 breast cancer samples. High nuclear LMTK3 were associated with a significantly shorter disease-free survival (DFS, *p* = 0.01) and overall survival (OS, *p* = 0.03) (Fig. 3b, c). LMTK3 levels were also predictive for response to endocrine therapy (*p* = 0.04) (Fig. 3d) but did not predict response to adjuvant chemotherapy (*p* = 0.18) (Fig. 21e). To further investigate potential involvement of LMTK3 in the development of tamoxifen-resistance, we analyzed effects of LMTK3 silencing in tamoxifen-resistant cell lines. Tamoxifen alone slightly affected basal cell growth, whereas addition of LMTK3 siRNA increased the growth inhibitory effects of tamoxifen and predicted elevated levels of phosphorylated ERα and AIB1 were decreased (Fig. 28). In addition LMTK3 was also essential for E2-induced growth, as silencing of LMTK3 impeded cell proliferation in the presence of E2 (Fig. 29).

We also tested whether methylation may play a role in transcription or translation of LMTK3. We found only 5 out of 227 patients with a methylated LMTK3 gene suggesting that methylation is not operational here (Table 5.3). Since DNA variation impacts transcription and/or translation affecting clinical outcome, we demonstrated that two intronic polymorphisms were independently associated with DFS and OS suggesting functionally relevant polymorphisms. Patients harboring the *LMTK3* rs8108419 GG or AG and the LMTK3 rs9989661 TT alleles were at a lower risk of developing tumor recurrence (*RR* = 1; reference) compared to patients carrying the *LMTK3* rs8108419 AA and *LMTK3 rs9989661* CT or CC alleles (*RR*=2.44; *Cl*: 1.40-4.25) (*p* = 0.002; Table 5.4). Overall survival was associated with combined analyses of these 2 polymorphisms (*p* = 0.02; Fig. 3f,g and Table 5.4). In multivariate analyses LMTK3 polymorphisms were an independent biomarker for both DFS and OS.

Next, to investigate the effects of LMTK3 knockdown on breast tumor xenograft growth, we injected LMTK3 siRNA into pre-established human MCF7 breast carcinoma tumors grown in nude mice. *In vivo* bioluminescence imaging and volume measurements of the xenografted tumors demonstrated that loss of LMTK3 expression leads to a significant decrease in tumor growth (Fig. 22, *p* < 0.01).

The majority of human breast tumors express ERα and patients with ERα⁺ disease usually respond to endocrine therapies. Endocrine resistance is a major problem highlighting a need for understanding the mechanisms of ERα action and the development of new therapeutic agents. By performing a kinome siRNA screen to identify new proteins modulating ERα, combined with evolutionary and mechanistic analyses, we have established a role for LMTK3 in regulating ERα in breast cancer. We propose a model where inhibition of LMTK3 regulates the stability and activity of ERα at: i) the mRNA level, via activation of PKC resulting in degradation of FOXO3 via AKT phosphorylation, in turn leading to decreased ERα transcriptional activity and, ii) the protein level, by directly promoting proteasomal degradation of ERα and indirectly by increasing PKC activity that leads to down-regulation of ERα protein levels (Fig. 30). Interestingly, LMTK3 expression was down-regulated by E2 and up-regulated in response to tamoxifen thereby revealing a positive feedback loop between LMTK3 and ERα (Fig. 31).

The demonstration that expression and polymorphisms of LMTK3 are associated with clinical outcome and response to endocrine therapy in breast cancer in combination with our *in vivo* studies suggests clinical and translational relevance. While presumably all proteins must have been positively selected for their biochemical functions at some time in the past, only very few show evidence of such adaptive evolution³¹. It is relevant that LMTK1 and LMTK2 isoforms are not positively selected between humans and chimpanzees; they are well-conserved. Here, positive selection has been operational on human LMTK3 (in a region containing no recognized conserved kinase domains); this may have altered substrate binding characteristics of human *vs*. chimpanzee LMTK3. While the selective pressure that drove this adaptive event is at present unclear, an evolutionary trade-off may have led to increased human susceptibility to this disease. Most humans we examined have the 'protective' TT allele, while the less-susceptible NHPs lack the protective TT allele, as a result of selective pressure to counter possible deleterious effects of sequence changes to human LMTK3 (Table 5.5). Further investigation of chimpanzee LMTK3 may yield insights into the natural history of breast cancer in humans vs. chimpanzees. In aggregate, these data reveal LMTK3 as a biomarker of response to endocrine therapy in breast cancer and highlight its potential as a therapeutic target.

### Methods

### High throughput siRNA screening

The human kinase siRNA Set Version 3.0 library (Qiagen) targeting 691 kinases and kinase-related genes was used. The library was supplied in a 96-well format and contained a pool of two individual siRNAs per well targeting two different sequences for each gene. MCF7 cells were maintained in phenol red-free media with 10% charcoal-stripped serum (DSS) 48 h before experimentation. Cells were plated in 24-well plates and transfected with siRNA (final concentration 20 nM) using the Human Kinase siRNA Set Version 3.0 library and Hiperfect reagent according to the manufacturer's instructions (Qiagen). At 48 h post-transfection, cells were treated with vehicle (ethanol) or E2 (10 nM) for 24 h and cells were harvested following RNA extraction and cDNA synthesis. Quantitative RT-PCR (RT-qPCR) analysis to examine the expression levels of *TFF1* and *GAPDH* (endogenous control) was performed for each well (kinase gene). Next, the *TFF1* gene expression after silencing each kinase individually was calculated in relation to *GAPDH* expression; screening was performed in duplicate.

### Evolutionary analysis

Positive selection on the protein-coding regions of LMTK3 was detected by use of molecular evolution algorithms that characterize the relative proportion of nonsynonymous (replacement) nucleotide substitutions as compared to synonymous (silent) nucleotide substitutions in the kinase coding sequences. (Note that the LMTK3 rs8108419 GG or AG and the LMTK3 rs9989661 TT alleles were not examined in this manner, as these regions are exclusively intronic.) All kinases shown in our screen to modulate ERα, as well the isoforms LMTK1 and LMTK2, were examined for evidence of sequence level positive selection between human and chimpanzee orthologues using the Li93 software. Both whole coding sequence and subsection sliding windows were examined. Only LMTK3 showed evidence of positive selection (*p* < 0.005).

### Candidate polymorphisms and genotyping

Candidate LMTK3 polymorphisms were chosen with the assistance of the Ensembl program using two main criteria: first, that the polymorphism has some degree of likelihood to alter the function of the gene in a biological relevant manner. Rs8108419 polymorphism located in intron 2 of the *LMTK3* gene, rs9989661 polymorphism located in intron 15 of the *LMTK3* gene. Intron polymorphisms can change gene transcription levels by alternative splicing or by affecting binding of a transcription factor. Second, that the frequency of the polymorphism is sufficient enough that its impact in clinical outcome would be meaningful on a population level (above 10% allele frequency). Genomic DNA was extracted from micro-dissected tissue specimens using the QIAamp kit. *LMTK3* polymorphisms (rs8108419 and rs9989661) were tested using polymerase chain reaction restriction fragment length polymorphism (PCR-RFLP) technique. Briefly, forward primer 5'-ATTCCACCACTCCCTC CAG-3' and reverse primer 5'-GACCCTGCAGTGCCTCAC-3' for rs8108419 and forward primer 5'-GGGCCTTCCCAAGTGGTT-3' and reverse primer 5'-ATCCAAGCCTGGGGTG AG-3' for rs9989661 were used for PCR amplification, PCR products were digested by restriction enzyme BsrD1 (rs8108419) or Btsc1 (rs9989661), and alleles were separated on 4% NuSieve ethidium bromide-stained agarose gel. Appropriate ethics committee approval was obtained.

### In vivo tumorigenicity assay in nude mice bearing orthotopic breast cancer xenografts

Bioluminescent MCF-7 breast cancer cell lines were injected into the mammary fat pad of nude mice. When tumors reached an approximate area of 100-200 mm³ (day 15), mice were randomly assigned to different groups (*n* = 8, each group) to receive intra-tumoral injections of 10 µg *in vivo* modified LMTK3 siRNA or control siRNA (Qiagen). Three intra-tumoral injections were repeated every 3 days and mice were terminated 3 days after the last injection. Tumor growth was monitored using calliper measurements and bioluminescent imaging was performed 24 hours prior to dosing, and 72 hours after dosing. At termination, primary tumors were excised, weighed and formalin fixed. Samples were paraffin embedded, cut at 3 µm and H&E stained for histological evaluation of target proteins expression.

### References

1. Ali, S. & Coombes, R.C. Endocrine-responsive breast cancer and strategies for combating resistance. Nat Rev Cancer 2, 101-112 (2002).
2. Cordera, F. & Jordan, V.C. Steroid receptors and their role in the biology and control of breast cancer growth. Semin Oncol 33, 631-641 (2006).
3. Hammond, M.E., et al. American Society of Clinical Oncology/College Of American Pathologists guideline recommendations for immunohistochemical testing of estrogen and progesterone receptors in breast cancer. J Clin Oncol 28, 2784-2795.
4. Lannigan, D.A. Estrogen receptor phosphorylation. Steroids 68, 1-9 (2003).
5. Li, C., et al. Essential phosphatases and a phospho-degron are critical for regulation of SRC-3/AIB1 coactivator function and turnover. Molecular cell 31, 835-849 (2008).
6. Wu, R.C., et al. Selective phosphorylations of the SRC-3/AIB1 coactivator integrate genomic reponses to multiple cellular signaling pathways. Molecular cell 15, 937-949 (2004).
7. Campbell, R.A., et al. Phosphatidylinositol 3-kinase/AKT-mediated activation of estrogen receptor alpha: a new model for anti-estrogen resistance. The Journal of biological chemistry 276, 9817-9824 (2001).
8. Chen, D., et al. Phosphorylation of human estrogen receptor alpha at serine 118 by two distinct signal transduction pathways revealed by phosphorylation-specific antisera. Oncogene 21, 4921-4931 (2002).
9. Jiang, J., et al. Phosphorylation of estrogen receptor-alpha at Ser167 is indicative of longer disease-free and overall survival in breast cancer patients. Clin Cancer Res 13, 5769-5776 (2007).
10. Kato, S., et al. Activation of the estrogen receptor through phosphorylation by mitogen-activated protein kinase. Science 270, 1491-1494 (1995).
11. Sarwar, N., et al. Phosphorylation of ERalpha at serine 118 in primary breast cancer and in tamoxifen-resistant tumours is indicative of a complex role for ERalpha phosphorylation in breast cancer progression. Endocrine-related cancer 13, 851-861 (2006).
12. Beniashvili, D.S. An overview of the world literature on spontaneous tumors in nonhuman primates. J Med Primatol 18, 423-437 (1989).
13. McClure, H.M. Tumors in nonhuman primates: observations during a six-year period in the Yerkes primate center colony. Am J Phys Anthropol 38, 425-429 (1973).
14. Puente, X.S., et al. Comparative analysis of cancer genes in the human and chimpanzee genomes. BMC Genomics 7, 15 (2006).
15. Seibold, H.R. & Wolf, R.H. Neoplasms and proliferative lesions in 1065 nonhuman primate necropsies. Lab Anim Sci 23, 533-539 (1973).
16. Waters, D.J., et al. Workgroup 4: spontaneous prostate carcinoma in dogs and nonhuman primates. Prostate 36, 64-67 (1998).
17. Inoue, T., et al. BREK/LMTK2 is a myosin VI-binding protein involved in endosomal membrane trafficking. Genes Cells 13, 483-495 (2008).
18. Robinson, D.R., Wu, Y.M. & Lin, S.F. The protein tyrosine kinase family of the human genome. Oncogene 19, 5548-5557 (2000).
19. Tomomura, M., et al. Structural and functional analysis of the apoptosis-associated tyrosine kinase (AATYK) family. Neuroscience 148, 510-521 (2007).
20. Naik, S., Dothager, R.S., Marasa, J., Lewis, C.L. & Piwnica-Worms, D. Vascular Endothelial Growth Factor Receptor-1 Is Synthetic Lethal to Aberrant {beta}-Catenin Activation in Colon Cancer. Clin Cancer Res 15, 7529-7537 (2009).
21. Tyner, J.W., et al. RNAi screen for rapid therapeutic target identification in leukemia patients. Proceedings of the National Academy of Sciences of the United States of America 106, 8695-8700 (2009).
22. Eeckhoute, J., et al. Positive cross-regulatory loop ties GATA-3 to estrogen receptor alpha expression in breast cancer. Cancer research 67, 6477-6483 (2007).
23. Guo, S. & Sonenshein, G.E. Forkhead box transcription factor FOXO3a regulates estrogen receptor alpha expression and is repressed by the Her-2/neu/phosphatidylinositol 3-kinase/Akt signaling pathway. Molecular and cellular biology 24, 8681-8690 (2004).
24. Morelli, C., et al. Akt2 inhibition enables the forkhead transcription factor FoxO3a to have a repressive role in estrogen receptor alpha transcriptional activity in breast cancer cells. Molecular and cellular biology 30, 857-870.
25. Zou, Y., et al. Forkhead box transcription factor FOXO3a suppresses estrogen-dependent breast cancer cell proliferation and tumorigenesis. Breast Cancer Res 10, R21 (2008).
26. Madureira, P.A., et al. The Forkhead box M1 protein regulates the transcription of the estrogen receptor alpha in breast cancer cells. The Journal of biological chemistry 281, 25167-25176 (2006).
27. Castles, C.G., Oesterreich, S., Hansen, R. & Fuqua, S.A. Auto-regulation of the estrogen receptor promoter. J Steroid Biochem Mol Biol 62, 155-163 (1997).
28. Brunet, A., et al. Akt promotes cell survival by phosphorylating and inhibiting a Forkhead transcription factor. Cell 96, 857-868 (1999).
29. Marsaud, V., Gougelet, A., Maillard, S. & Renoir, J.M. Various phosphorylation pathways, depending on agonist and antagonist binding to endogenous estrogen receptor alpha (ERalpha), differentially affect ERalpha extractability, proteasome-mediated stability, and transcriptional activity in human breast cancer cells. Molecular endocrinology (Baltimore, Md 17, 2013-2027 (2003).
30. Belguise, K. & Sonenshein, G.E. PKCtheta promotes c-Rel-driven mammary tumorigenesis in mice and humans by repressing estrogen receptor alpha synthesis. J Clin Invest 117, 4009-4021 (2007).
31. Messier, W. & Stewart, C.B. Episodic adaptive evolution of primate lysozymes. Nature 385, 151-154 (1997).

### Supplementary Information

### Full Methods

### Cell lines, reagents, antibodies and plasmids

MCF7, ZR-75-1, SKBR3, MDA-468, But-474¹, LCC9², MLET5³ and MELN cells were maintained in DMEM supplemented with 10% FCS and 1% penicillin / streptomycin / glutamine. All cells were incubated at 37 °C in humidified 5% CO₂. Estradiol (E2) and Tamoxifen (Tam) were obtained from Sigma and dissolved in 100% ethanol; Cyclohexamide (CHX), Go6983 and PMA were purchased from Sigma; charcoal-dextran stripped serum (DSS) was obtained from Gemini. The following antibodies were used: ERα mouse monoclonal (Abcam), ERα-S167 rabbit monoclonal (Abcam), β-actin mouse monoclonal (Abcam), AKT rabbit polyclonal (Abcam), LMTK3 mouse monoclonal (Santa Cruz), AKT-S473 (Santa Cruz), PKC rabbit polyclonal (Santa Cruz), FOXO3 rabbit monoclonal (Cell Signaling), phospho-FOXO3-S318/321 rabbit polyclonal (Cell Signaling), phospho-FOXO3-T32 (a kind gift of Prof Eric Lam), FOXM1 rabbit polyclonal (Cell Signaling) and Phospho-(Ser) PKC Substrate Antibody (Cell Signaling). Secondary HRP (horseradish peroxidase)-conjugated goat anti-rabbit IgG and goat anti-mouse IgG antibodies were from GE Healthcare. The expression plasmid pTriEx-1.1-4F encoding for LMTK3 kinase domain (aa 133-411) was purchased by Geneart. The expression plasmid pSG5-ERα was generated as described⁴.

### RNA isolation and quantitative RT-PCR

Total RNA was isolated using the RNeasy kit (Qiagen). Reverse transcription was performed using high capacity cDNA reverse transcription kit (Applied Biosystems). RT-qPCR analysis was performed on a 7900HT Thermocycler (Applied Biosystems) using TaqMan mastermix and primers for *TFF1, PGR, GREB1, FOXO3, FOXM1, LMTK1, LMTK2, LMTK3, MCL1* and *GAPDH* cDNAs, purchased from Applied Biosystems.

### SDS-PAGE and Western blotting

Whole cell lysates were prepared using NP40 lysis buffer (50 mM Tris-HCl, pH 8.0, 150 mM NaCl, 10% (v/v) glycerol, 1% NP40, 5 mM dithiothreitol (DTT), 1 mM EDTA, 1 mM EGTA, 50 µM leupeptin and 30 µg/ml aprotinin). These extracts were then clarified by centrifugation at 15000 rpm for 15 min at 4 °C for western blotting. The bicinchoninic acid (BCA) protein assay (Pierce) was used to determine the protein concentration of the lysates. Lysates were incubated in 5x sodium dodecyl sulfate (SDS) sample buffer (5 min, 95 °C), subjected to 8% or 12% SDS-PAGE and blotted on a Hybond ECL super nitrocellulose membrane (GE Healthcare). The membranes were then blocked in TBS containing 0.1% (v/v) Tween20 and 5% (w/v) non-fat milk for 1 h and subsequently probed overnight with different antibodies following extensive washing with TBS/Tween and incubation with HRP-conjugated goat anti-rabbit IgG or goat anti-mouse IgG (1:1000 dilution) for 45 min. Enhanced chemiluminescence (ECL) detection then allowed detection of the immuno-complexes. The intensity of the bands was quantified using Image J software (NIH, Bethesda, MD).

### In vitro kinase assay

To examine whether LMTK3 can phosphorylate ERα {or affect PKC catalytic activity}, recombinant LMTK3 kinase domain (Genscript) was incubated with full length recombinant human ERα protein (Invitrogen) {or recombinant PKC (Promega)}in kinase buffer (containing 50 mM HEPES pH 7.5, 10 mM MgCl₂, 1 mM EGTA, 1 mM DTT, 0.2 mM ortho-vanadate, 0.25 mM ATP and 0.3 µCi/µl [γ-³²P]ATP) for 30 min at 30 °C. Samples were resolved by SDS-PAGE, stained with Coomassie, dried and subjected to autoradiography.

### In vitro ERα degradation assay

ERα degradation assays were performed using the recombinant human ERα, 26S proteasome fraction, GST-ubiquitin-activating enzyme (E1), GST-ubiquitin-conjugating enzyme UbcH7 (E2), MCF-7 lysate (50 µg) as E3 source, and an energy regenerating solution (Boston Biochem) for 60 min at 37°C, as previously described⁵. Prior to degradation assays, where indicated, recombinant ERα was phosphorylated *in vitro* with LMTK3 kinase domain for 30 min at 30°C. ERα was precipitated, and assayed by Western blotting.

### Chromatin immunoprecipitation assays (ChIP)

Cross-linked chromatin was prepared from MCF-7 cells as described previously⁶. For immunoprecipitation, aliquots of 20 µg were incubated overnight with 2 µg of FOXO3 antibody or without (mock controls) in a total volume of 1 ml. Triplicate samples of 5 µl of immunoprecipitated genomic DNA were amplified by real time PCR. Primers used for Chip analysis (Promoter D): 5'-TTTAATCTGGGTGGCTGGAG-3' and 5'-CTCAACTTCCCCG TGTCTGT-3'. Values are expressed as fold of enrichment with respect to input DNA.

### Cell proliferation assay

The colorimetric Rapid Cell Proliferation kit (Calbiochem, UK) was used. The assay is based on the incubation of cells with the tatrazolium salt WST-1, which is cleaved by mitochondrial dehydrogenases, functional only in viable cells. Therefore, the assay measurement corresponds to relative number or % of viable cells in proliferation after certain treatment. Briefly, cells were seeded at 3 x 10³/well in a 96 well plate and allowed to adhere. The following day, cells were incubated with the WST-1 for 30 min at 37 °C, according to manufacturer's instructions, and the absorbance read with the microplate reader at 460 nm was considered to be Day 0. The same day cells were transfected with the CT siRNA, death siRNA and LMTK1-3 siRNAs. Readings were repeated consecutively in 48 h intervals. Results represent the mean of three experiments per cell line ± SE.

### Flow cytometry (FACS)

To examine the effect of LMTK3 silencing on cell cycle progression and apoptosis/necrosis, MCF7 cells were treated with CT siRNA or LMTK3 siRNA for different time points (24 h, 48 h and 72 h). Following, 1 × 10⁵ cells were collected and stained with propidium iodide (PI) using the BD Cycletest™ Plus DNA Reagent Kit, according to the manufacturer's instructions (BD Biosciences). Cells were analyzed using a LSR II flow cytometer (BD Biosciences). The percentage of cells in subG₁ G₀/G₁, S and G₂/M phases were determined from >10,000 ungated cells using the FACSDiva 6.0 software (BD Biosciences).

### Immunofluorescence

MCF-7 cells grown on poly-D-lysine-coated glass coverslips were fixed in 4% w/v paraformaldehyde at 37 °C for 15 min, permeabilized with 0.1% Triton-X for 10 min and incubated in immune-fluorescent blocking buffer (10% AB-serum in PBS) for 1h, followed by incubation with the following primary antibodies: LMTK3 anti-mouse antibody (1/100), FOXO3 anti-rabbit antibody (1/200) and ERα anti-mouse antibody (1/200). After washing with PBS, coverslips were incubated for 45 min at 37°C with anti-rabbit or anti-mouse-IgG Alexa Fluor®-488 or -555 antibodies (Invitrogen). DNA was visualized by DAPI staining. Cells were examined on an Axiovert-200 laser scanning inverted microscope (Zeiss) equipped with a confocal imaging system. Image composites of ∼20 × 0.5 µm z-stacks were obtained using Axiovision LE software (Zeiss). Photoshop 8.0 (Adobe Software) was used for post-acquisition editing of images.

### Annexin V apoptosis assay

For detection and quantification of apoptosis by flow cytometry, the annexin V-fluorescein isothiocyanate (FITC) labeled Apoptosis Detection Kit I (BD Biosciences) was used according to the manufacturer's instructions. Briefly, MCF7 cells (1 × 10⁶) were platted in 100-mm Petri-dish and treated with 20nM of CT siRNA or LMTK3 siRNA for 72 h. Subsequently, cells were labelled with annexin V-FITC (10 µg/mL), PI (10 µg/mL) and analyzed using a flow cytometer as described above.

### Immunohistochemistry

Anti-LMTK3 mouse monoclonal antibody (Santa Cruz) was optimized to a working concentration 4 µg/ml on full-face excisional tissue sections. Subsequently, BC TMA (n = 614) cases comprising 4 µm thick formalin fixed paraffin embedded tissue cores were immuno-stained with the optimised anti-LMTK3 McAb on the Leica BOND-MAX automated system using manufacturer's instructions. Hit-induced epitope retrieval was performed in citrate buffer (ER1) for 5mins. Detection was achieved using the Polymer Detection kit (Leica Microsystems Inc.), These detection systems contain Peroxidase Block, Protein Block, Post Primary Block, Novolink Polymer, DAB Chromogen, Novolink DAB Substrate Buffer (Polymer) and Haematoxylin for subsequent counterstaining of the TMAs. Negative controls were performed by omission of the primary antibody. LMTK3 immunoreactivity was detected in the nucleus of breast epithelial cells, and in the cytoplasm to a variable degree. Nuclear staining was scored based on the nuclear H-score. Determination of the optimal cut-offs were performed using X-tile bioinformatics software; (Yale University, USA). Cut-off values were as follows: weak (1-25); moderate (26-134); strong (135-300). Scoring for each tissue core on the TMAs was performed by two independent investigators (AF and JS). High resolution digital imaging (NanoZomer, Hamamatsu Photonics) at 20x magnification with a web-based interface (Distiller, SlidePath Ltd.) was used. All cases were scored without knowledge of the clinicopathological or outcome data.

### Clinical specimens and tissue microarrays

Tissue microarrays (TMAs) containing 614 primary operable breast cancer cases from the previously validated Nottingham Tenovus Primary Breast Carcinoma Series⁷ were employed. The cohort comprised women aged up to 70 years, who presented between 1986 and 1999. Patients within the good prognosis group (Nottingham Prognostic Index (NPI) ≤ 3.4) did not receive adjuvant therapy. Hormonal therapy was prescribed to patients with ERα positive tumors and NPI scores > 3.4 (moderate and poor prognostic groups). Pre-menopausal patients within the moderate and poor prognosis groups were candidates for chemotherapy. Conversely, postmenopausal patients with moderate or poor NPI and ERα⁺ were offered hormonal therapy, while ERα- patients received chemotherapy. Data collected included overall survival (OS) and disease-free survival (DFS). Appropriate ethics committee approval was obtained.

### Bisulfite modification and MSP

Genomic DNA was extracted from formalin-fixed paraffin embedded breast cancer tissues via microdissection as previously described⁸. Genomic DNA was then bisulfite converted using the Zymo EZ DNA methylation kit (Zymo Research) according to the manufacturer's specifications. MethyLight analysis of the *LMTK3* promoter region was performed as previously described⁹. Percent of Methylated Reference (PMR) values were calculated using a control reaction based on ALU repeats for normalization as previously described⁹. The primer and probe sequences for *LMTK3* are as follows: *forward: 5'*-TGT AGATATATTCGGAAGCGCG-3'; *reverse*: 5'-ACGCCTCGCAAATACTCACG-3'; *probe*: 6FAM-ATCCGCGCCCAAAATA-MGBNFQ.

### Statistical analysis

Statistical analysis for TMAs was performed using SPSS 16.0 statistical software (SPSS Inc., Chicago, IL, USA). Analysis of categorical variables was performed with the appropriate statistical test. Survival curves were analyzed using the Kaplan-Meier method with significance determined by the Log rank test; multivariate analysis was performed by Cox hazards regression analyses. Exploratory data analysis for *in vitro* data demonstrated that the distributions were often skewed with outliers. Shapiro-Wilks test was used to test for normality (data were not normally distributed). Between the groups, comparisons were made using the non parametric Mann Whitney U test. DFS was defined as the period from the date of initial diagnosis to the date of the first documented relapse and OS was defined as the time from the initial diagnosis to breast cancer related death. DFS time was censored at the date of last follow-up if patients were still relapse-free and alive, and OS was censored at the time when patients were alive. A forward stepwise Cox regression model was conducted to select baseline patient demographic and tumor characteristics to be included in the multivariate analyses of 2 LMTK3 polymorphisms and clinical outcome. Chi-square tests were used to examine the associations between tumor characteristics and LMTK3 polymorphisms. All tests were 2-sided at a 0.05 significance level and performed using the SAS statistical package version 9.2.

### References for Supplementary Information

1. Wang, L.H., et al. Disruption of estrogen receptor DNA-binding domain and related intramolecular communication restores tamoxifen sensitivity in resistant breast cancer. Cancer cell 10, 487-499 (2006).
2. Brunner, N., et al. MCF7/LCC9: an antiestrogen-resistant MCF-7 variant in which acquired resistance to the steroidal antiestrogen ICI 182,780 confers an early cross-resistance to the nonsteroidal antiestrogen tamoxifen. Cancer research 57, 3486-3493 (1997).
3. Tolhurst, R.S., et al. Transient over-expression of estrogen receptor-alpha in breast cancer cells promotes cell survival and estrogen-independent growth. Breast cancer research and treatment*.*
4. Tora, L., et al. The human estrogen receptor has two independent nonacidic transcriptional activation functions. Cell 59, 477-487 (1989).
5. Chu, I., et al. Src promotes estrogen-dependent estrogen receptor alpha proteolysis in human breast cancer. J Clin Invest 117, 2205-2215 (2007).
6. Shang, Y., Hu, X., DiRenzo, J., Lazar, M.A. & Brown, M. Cofactor dynamics and sufficiency in estrogen receptor-regulated transcription. Cell 103, 843-852 (2000).
7. Habashy, H.O., et al. Forkhead-box A1 (FOXA1) expression in breast cancer and its prognostic significance. Eur J Cancer 44, 1541-1551 (2008).
8. Woodson, K., et al. Gene-specific methylation and subsequent risk of colorectal adenomas among participants of the polyp prevention trial. Cancer Epidemiol Biomarkers Prev 14, 1219-1223 (2005).
9. Weisenberger, D.J., et al. CpG island methylator phenotype underlies sporadic microsatellite instability and is tightly associated with BRAF mutation in colorectal cancer. Nature genetics 38, 787-793 (2006).

**Table 5.1 Results of human kinome ERα-regulated siRNA screening (p < 0.05)**

| **Group A (up-regulation)** | | | ***FOLD CHANGE*** | |
|---|---|---|---|---|
| **siRNA pool** | **Gene name** | ***TFF1*** | ***PGR*** | ***GREB1*** |
| **LATS2** | large tumor suppressor, homolog 2 | 2.98 | 1.34 | 2.05 |
| **NEK3** | NIMA (never in mitosis gene a)- related kinase 3 | 2.96 | - | 1.14 |
| **NEK8** | NIMA (never in mitosis gene a)- related kinase 8 | 2.35 | 1.19 | 1.59 |
| **PIP5K2B** | phosphatidylinositol-4-phosphate 5-kinase, type II, beta | 2.11 | 1.80 | 1.40 |
| **CCRK** | cell cycle related kinase | 2.03 | 2.56 | 1.73 |

| **Group B (down-regulation)** | | | ***FOLD CHANGE*** | |
|---|---|---|---|---|
| **siRNA pool** | **Gene name** | ***TFF1*** | ***PGR*** | |
| ***GREB1*** | | | | |
| **ACVR2B** | activin A receptor, type IIB | 4.65 | 2.81 | 1.53 |
| **BCR** | breakpoint cluster region | 3.53 | - | - |
| **AKAP13** | A-kinase anchor protein 13 | 3.33 | 3.22 | 1.76 |
| **TYRO3** | TYRO3 protein tyrosine kinase | 3.28 | 5.35 | 5.59 |
| **LMTK3** | lemur tyrosine kinase 3 | 2.96 | 4.57 | 2.98 |
| **PRKAR1B** | protein kinase, cAMP-dependent, regulatory, type I, beta | 2.87 | - | - |
| **MAP3K7** | mitogen-activated protein kinase kinase kinase 7 | 2.51 | 1.24 | 1.71 |
| **CDC2L1** | cell division cycle 2-like | 2.38 | 1.65 | 1.45 |
| **GRK6** | G protein-coupled receptor kinase 6 | 2.30 | +1.82 | +1.67 |
| **CDKN2A** | cyclin-dependent kinase inhibitor 2A | 2.26 | +1.66 | 1.17 |
| **MARK4** | MAP/microtubule affinity-regulating kinase 4 | 2.25 | +1.44 | 1.10 |
| **MAPK3** | mitogen-activated protein kinase 3 | 2.25 | - | - |
| **CKMT1B** | creatine kinase, mitochondrial 1B | 2.22 | 1.09 | 1.87 |
| **KSR1** | kinase suppressor of ras 1 | 2.19 | 6.94 | 6.80 |
| **AKT3** | v-akt murine thymoma viral oncogene homolog 3 | 2.18 | - | - |
| **MAPKAP1** | mitogen-activated protein kinase associated protein 1 | 2.13 | - | - |

| | | | | |
|---|---|---|---|---|
| - = *not measured* | | | | |

**Table 5.2 Kinase Ka/Ks values**

| Kinase | Positively selected | Ka/Ks value | Substitutions |
|---|---|---|---|
| *Group A (up-regulation)* | | | |
| LATS2 | No | 0.12 | R 6, S 21 |
| NEK3 | No | 0.00 | R0, S2 |
| NEK8 | No | 0.00/0.00 (Sequences are identical) | No substitutions |
| PIP5K2B | No | 0.00 | R 0, S 8 |
| CCRK | No | 0.16 | R 5, S 7 (partial seq) |
| RIOK1 | No | 0.43 | R 6, S 7 |
| PANK2 | No | 0.10 | R 2, S 6 (partial seq) |
| RPS6KA6 | No | 0.50 | R3, S3 |

| *Group B (down-regulation)* | | | |
|---|---|---|---|
| ACVR2B | No | 0.00 | R 0, S 2 |
| BCR seq) | No | 0.28 | R 2, S 3 (partial seq) |
| AKAP13 | No | 0.25 | R 3, S 6 (partial seq) |
| TYRO3 | No | 0.00 | R 0, S 3 (partial seq) |
| LMTK3 | Yes, p < 0.005 | Div/0 (no silent substitutions) | R 5, S 0 |
| PRKAR1B | No | 0.51 | R 1, S 1 (partial seq) |
| MAP3K7 | No | 0.00 | R 0, S 5 |
| CDC2L1 | No | 0.00 | R 0, S 6 |
| GRK6 | No | 0.56 | R 5, S 5 |
| CDKN2A | No | 0.00 | R 0, S 1 |
| MARK4 | No | 0.00/0.00 (Sequences are identical) | No substitutions |
| MAPK3 | No | 0.00/0.00 (Sequences are identical) | No substitutions |
| CKMT1B | No | 0.11 | R 3, S 12 |
| KSR1 | No | 0.14 | R 3, S 10 |
| AKT3 | No | 0.00 | R 0, S 3 |
| MAPKAP1 | No | 0.00 | R 0, S 1 (partial seq) |

| *Other LMTK isoforms* | | | |
|---|---|---|---|
| LMTK1 R 17, S 20 | No | 0.62 | |
| LMTK2 | No | 0.26 | |
| R11,S18 | | | |

| Overall Patients | Methylator Phenotype (PMR>0) | No Methylation (PMR=0) |
|---|---|---|
| *n* = 227 | *n* = 5 | *n* = 222. |
| Percentage | 2.2% | 97.8% |
| Overall Survival (median months, 95% Cl) | 5.8 (4.4, 8.2+) | 8.4 (7.4, 10.0+) |
| | Log-rank p > 0.05 | |
| Progression-free Survival (PFS) (median months, 95% Cl) | 5.8 (2.1, 8.2+) | 6.4 (5.2, 8.4) |
| | Log-rank *p* > 0.05 | |

All kinases listed were identified in our screen, with the exception of the isoforms LMTK1 and LMTK2. These isoforms of LMTK3 were examined to rule out the possibility that the LMTK protein family has been positively selected as a whole. The positively selected region of LMTK3 contains no synonymous nucleotide substitutions between the human and chimpanzee orthologues. In contrast, several of the pairwise comparisons listed here show ONLY synonymous nucleotide substitutions. Two pairs of human/chimpanzee orthologues were identical, base for base. The number of replacement (labelled R) and silent substitutions (labelled S) is shown for each comparison. In a few cases, only partial sequence was used; these are noted.

### Table 5.3 CIMP status and clinical outcome

Methylation of LMTK3 in 227 patients. Only 5 patients had PMR value greater than 0. The comparisons on OS and PFS between Methylator Phenotype + and lack of methylation may not be valid due to the small number of patients with Methylator Phenotype.

**Table 5.4 LMTK3 polymorphisms and time to recurrence and overall survival in patients with breast cancer**

| | *n* | Median time to relapse, yrs (95% CI) | HR (95% CI) | *ρ* value | Median time to relapse, yrs (95% CI) | HR (95% CI) | *ρ* v |
|---|---|---|---|---|---|---|---|
| | | | Disease Free Survival | | | Overall survival | |
| **ITK3 rs8108419** | | | | 0.038 | | | 0.C |
| G/G* | 155 | 7.5 (6.3, 9.7) | 1 (Reference) | | 8.4 (7.4, 8.8) | 1 (Reference) | |
| A/G* | 64 | | | | | | |
| A/A | 15 | 4.8 (3.0, 8.1) | 2.221 (1.043, 4.728) | | 6.1 (1.7, 8.5+) | 1.563 (0.663, 3.685) | |
| **LMTK3 rs9989661** | | | | 0.042 | | | 0.039 |
| T/T | 215 | 7.6 (6.3, 9.7) | 1 (Reference) | | 8.4 (7.4, 10.0+) | 1 (Reference) | |
| C/T* | 17 | 5.4 (2.1, 8.0+) | 2.036 (1.028, 4.033) | | 4.4 (3.0, 8.8) | 2.095 (1.039, 4.221) | |
| C/C* | 3 | | | | | | |
| **Combined 2 polymorphisms** | | | | 0.002 | | | 0.017 |
| G/G or AG and TT | 198 | 7.6 (6.4, 9.7) | 1 (Reference) | | 8.4 (7.4, 10.0+) | 1 (Reference) | |
| AA, C/T or C/C | 35 | 4.8 (3.0, 8.1) | 2.435 (1.396, 4.247) | | 5.9 (4.3, 8.8) | 2.062 (1.140, 3.730) | |

**Table 5.5 LMTK3 polymorphisms between human and non-human primates**

| **LMTK3 rs9989661 Genotype** | **Human (*n* = 235)** | **Nonhuman Primates (NHP) (*n*=12)** |
|---|---|---|
| **TT** | 215(91.5%) | 1(8.3%) |
| **C/T** | 17 (7.2%) | 0(0%) |
| **C/C** | 3 (1.3%) | 11(91.7%) |
| ***p* value** | < 0.001 | |

We sequenced DNA directly, with overlapping reads in both directions. As shown above, the TT allele occurred in 91.5% of human individuals sampled (n = 235) while in NHPs the TT allele occurred in only 8.3% of individuals examined (p < 0.001, Fischer's exact test). Human samples were those described in the main text. NHPs included several species, as we wished to interrogate a wide phylogenetic diversity within the NHPs: 4 common chimpanzees (*Pan troglodytes*)*,* 2 bonobos (*Pan paniscus*)*,* 2 gorillas (*Gorilla gorilla*), 1 Sumatran orangutan (*Pongo abelii*)*,* 1 lar gibbon (*Hylobates lar*)*,* 1 baboon (*Papio cynocephalus*)*,* and a capuchin (*Cebus albifrons*). All NHP individuals are unrelated.

In effect we are postulating two selective pressures/events: one on the protein sequence/structure and another on the frequency of the TT SNP in the LMTK3 intron. The second event was thus likely compensatory for changes that occurred in the first event, which altered the protein itself. We acknowledge that it is of course possible that the positively selected amino acid replacements in human LMTK3 are not responsible for the documented human susceptibility to breast cancer; it is still the case however, that the TT allele our data suggests to be protective in humans is present at far lower frequencies in the less-susceptible NHPs, suggesting that the difference in allelic frequency compensates at least in part for greater human susceptibility to breast cancer. The allelic differences in LMTK3 intron are likely to impact gene expression/regulation of LMTK3 or nearby genes.

### Example 5: LMTK3 expression in breast cancer: association with tumor phenotype and clinical outcome.

Interactions between kinases and the estrogen receptor α (ERα) are thought to be a critical signaling pathway in the majority of human breast cancers. We have recently identified a previously uncharacterized molecule, lemur tyrosine kinase-3 (LMTK3) as a prognostic and predictive oncogenic ERα regulator with a critical role in endocrine resistance. Unusually this protein has undergone Darwinian positive selection between Chimpanzees and humans, suggesting it may contribute to human susceptibility to ERα-positive tumors. Using over 600 primary breast cancer cases, we wished to establish tumor characteristics associated with both cytoplasmic and nuclear LMTK3 expression, and then validate our previously observed European clinical outcomes with an Asian group of patients receiving chemotherapy. Both nuclear and cytoplasmic expression correlated with tumor grade (p<0.001) and in the Asian cohort, independent blinded analyses demonstrated that high basal LMTK3 expression was associated with advanced stage of primary breast cancers and decreased overall (p=0.03) and disease free survival (p=0.006). In summary, higher LMTK3 expression is associated with more aggressive cancers. These data validate our previous findings and suggest LMTK3 expression may be a reliable new biomarker in breast cancer.

### Introduction

As shown in the preceding examples, LMTK3 represents a master modulator of estrogenic signaling, a prognostic and predictive factor and a new target in breast cancer.

Tumor pathology provides useful insights into the relevance of potential new biomarkers and associations reveal insights into the role of novel markers in breast cancer biology. Based on our findings, we wished to establish the tumor characteristics associated with LTMK3 over-expression, and validate our clinical outcomes in an independent cohort. As ethnic differences in breast cancer are thought to have an central role in incidence, presentation, outcome and pattern of disease^{15,16}, we used a published cohort of Asian patients from Singapore¹⁷ to validate the findings from our large European cohort.

### Methods

### Immunohistochemistry

Anti-LMTK3 mouse monoclonal antibody (Santa Cruz, Heidelberg, Germany) was optimized to a working concentration 2µg on full-face excisional tissue sections. Subsequently, breast cancer tissue microarrays (TMAs) (n = 614) cases comprising 4 µm thick formalin fixed paraffin embedded tissue cores were immuno-stained with the optimised anti-LMTK3 McAb on the Leica BOND-MAX automated system using manufacturer's instructions. Hit-induced epitope retrieval was performed in citrate buffer (ER1) for 5mins. Detection was achieved using the Polymer Detection kit (Leica Microsystems Inc., USA), These detection systems contain Peroxidase Block, Protein Block, Post Primary Block, Novolink Polymer, DAB Chromogen, Novolink DAB Substrate Buffer (Polymer) and Hematoxylin for subsequent counterstaining of the TMAs. Negative controls were performed by omission of the primary antibody. LMTK3 immuno-reactivity was detected in the nucleus of breast epithelial cells, and in the cytoplasm to a variable degree. Nuclear staining was scored based on the nuclear H-score. Determination of the optimal cut-offs were performed using X-tile bioinformatics software (Yale University, USA). Cut-off values were as follows: weak (1-25); moderate (26-134); strong (135-300). Cytoplasmic staining was scored based on intensity ranging from 0 to +3; 0 = null, +1 = low, +2 = intermediate and +3 = high level of staining intensity. Scoring for each tissue core on the TMAs was performed by two independent investigators (AF and JS). High resolution digital imaging (NanoZomer, Hamamatsu Photonics, Welwyn Garden City, UK) at 20x magnification with a web-based interface (Distiller, SlidePath Ltd., Dublin, Ireland) was used. All cases were scored without knowledge of the clinicopathological or outcome data. Standard cut-offs were used for established prognostic factors: ERα, PgR and cytokeratins 5/6, 7/8 and 18, positive if ≥ 10%; Ecadherin positive if H-score ≥45. Ki67 negative if ≤10%; BRCA1 H-score was 0 = 0, 1-100 = 1 and >100 = 2. Cut-off values for different biomarkers included in the study were chosen before statistical analysis, and followed standard guidelines^{3,18,19}.

### UK clinical specimens and tissue microarrays

Primary operable breast cancer cases (n = 614) from the previously validated Nottingham Tenovus Primary Breast Carcinoma Series were employed^{20,21}. The cohort comprised women aged up to 70 years, who presented between 1986 and 1999 and has been used to develop prognostic indicators^{22,23}. This well-characterized resource contains information on patients' clinical and pathological data including histological tumor type, primary tumor size, lymph node status, histological grade and data on other breast cancer relevant biomarkers. These include ERα, PgR, HER2, cytokeratins (CKs; 5/6, 7/8, 18), Ki67 and E-cadherin. Patients within the good prognosis group (Nottingham Prognostic Index (NPI) ≤ 3.4 did not receive adjuvant therapy. Hormonal therapy was prescribed to patients with ERα positive tumors and NPI scores > 3.4 (moderate and poor prognostic groups). Pre-menopausal patients within the moderate and poor prognosis groups were candidates for chemotherapy. Conversely, postmenopausal patients with moderate or poor NPI and ERα positive were offered hormonal therapy, while ERα negative patients received chemotherapy. Data collected is presented here in line with the REMARK criteria. Clinical data were maintained on a prospective basis with a median follow-up was 124 months (range 1 to 233).

### Singapore cohort

The recently published cohort of East Asian females (n = 100) was used with staining performed on core biopsies taken from the primary breast tumor at diagnosis with appropriate ethical approval¹⁷. During and post- neo-adjuvant chemotherapy (doxorubicin-docetaxel), biopsy samples were also obtained, and stained for LMTK3. The investigators in Singapore (WT and LSC) were blinded to the identity of the antibody/protein and scored the analysis in a blinded manner. The protocol followed was identical to the one used in the UK.

### Statistics

Statistical analysis for TMAs was performed using SPSS 16.0 statistical software (SPSS Inc., Chicago, IL, USA). Analysis of categorical variables was performed with the appropriate statistical test; tumor characteristics from the Singapore cohort were analyzed using standard Chi-squared testing. Survival curves were analyzed using the Kaplan-Meier method with significance determined by the Log rank test; multivariate analysis was performed by Cox hazards regression analyses. DFS was defined as the interval from the date of the primary surgery to the first loco-regional or distant metastasis. OS was defined as the time from the date of the primary surgical treatment to the time of death from breast cancer, death being scored as an event, and patients who died as a result of other causes or were still alive were excluded at the time of last follow-up.

### Results

### LMTK3 expression in breast cancer tissue

Immunostaining analysis in the British cohort revealed that LMTK3 was detected predominantly in the nucleus (LMTK3-nuc) with variable cytoplasmic staining. Nuclear staining was scored based on the H-score, as weak (1-25) in 26.4% of patients, moderate (26-134) in 34.7%, and strong (135-300) in 38.8% of analyzed samples (Fig 32a). Cytoplasmic expression (LMTK3-cyto) was scored based on intensity as being absent = 0, weak = 1, moderate = 2, and strong = 3. Cytoplasmic scores were dichotomised into groups: low (scores 0 and 1), and high (scores 2 and 3) as shown (Fig 32a). High LMTK3-cyto was observed in 9.5% of cases, while, 87.3% had low LMTK3-cyto levels (20 cores = 3.2% did not have LMTK3-cyto score recorded) (Fig 32b). The staining pattern observed in the Asian cohort was predominantly nuclear, as in the British cohort. Due to the smaller sample size, cut-off values used for statistical analysis in the Asian cohort were weak (H-score ≤ 25) and strong (H-score > 25).

### Nuclear LMTK3 and correlation with relevant tumor biomarkers

Nuclear LMTK3 expression directly correlated with high tumor grade (p < 0.001). High LMTK3 expression was observed in 57.6% of grade 3 tumors, while grade 1 tumors had high LMTK3 levels in only 20% of the cases. Positive correlations were with two out of three components of the tumor grade, namely, pleomorphism and mitotic index (p < 0.001). LMTK3 expression directly correlated with the tumor proliferation marker Ki67 (p = 0.002) and the Nottingham Prognostic Index (NPI). Patients with moderate and strong LMTK3 expression belonged to the group with NPI of >3.4 (MPG and PPG) in 70% and 73% of cases, respectively (p < 0.001). Although we did not observe significant correlation with the number of positive lymph nodes (LNs) (p = 0.308), interestingly all patients with >9 invaded LNs had tumors expressing high levels of LMTK3-nuc.

LMTK3 overexpression was negatively associated with the expression of hormone receptors, ERα and PgR (p < 0.01). Cases with strong LMTK3-nuc had absence of ERα in 32.31%, vs. 17.76% of weak LMTK3-nuc. In the entire dataset, 49.6% of cases co-expressed LMTK3-nuc (moderate and strong expression combined) and ERα. Tumors over-expressing Her2 were more likely to be LMTK3 positive (p = 0.006). Loss of BRCA1 was more frequent in strong LMTK3-nuc BCs (p = 0.023). We did not observe significant correlation with lymphovascular invasion, patient age, CK5/6, CK7/8, CK18 or E-cadherin expression. Correlation of LMTK3 expression with tumor size was of borderline significance (p = 0.052). However, there was a significant up-regulation of LMTK3 in invasive ductal carcinomas in the investigated cohort (p < 0.001), where 44.8% has strong, 35.2% moderate, and 20% weak LMTK3 levels. Patients' characteristics are summarized in Table 6.1.

### Cytoplasmic LMTK3 expression in breast cancer and correlation with relevant tumor biomarkers

Upon correlations with relevant biomarkers, we found that high LMTK3-cyto correlated directly with high tumor grade (p < 0.001). High LMTK3-cyto tumors were grade 3 in 84% of cases vs. 46.9% in the low LMTK3-cyto group. All three components of grade correlated significantly with high LMTK3-cyto namely, tubular formation (p = 0.012), pleomorphism and mitotic index (p < 0.001). Tumors with high LMTK3-cyto were more likely to be ERα, PgR negative (p < 0.001) and negative for the luminal marker, CK18 (p = 0.007). Conversely, basal-like tumors expressing high CK5/6, also had high cytoplasmic expression of LMTK3 (p = 0.031). No significance was rendered when we correlated LMTK3-cyto with tumor stage and number of positive LNs, patient age, tumor size, lymphovascular invasion, Ki67, Her2 or E-cadherin (p > 0.05). Patients' characteristics are summarized in Table 6.2.

### LMTK3 expression and clinical outcomes in Asian patients

As per our data from the European/British cohort, high baseline LMTK3 expression was associated with a decreased overall survival. Although numbers are small results were statistically significant (p=0.034). Similarly significant associations were observed for disease free survival (p=0.006).

Changes in LMTK3 expression during neo-adjuvant chemotherapy with doxorubicin and docetaxel containing therapy were not prognostic or predictive. However, LMTK3 expression levels demonstrated a tendency to increase with ongoing neo-adjuvant chemotherapy. Interestingly, this rise in LMTK3 levels appears to be an early response to chemotherapy in ERα negative patients (after the 1^{st} cycle) and a late response in ERα positive sub-population (after sixth cycle). At baseline, LMTK3 expression was correlated with T4 tumors (median size 13.5 cm), as shown in Table 6.3.

### LMTK3 expression in various cancer tissues

We investigated LMTK3 expression in a panel of human tissues, both normal and their malignant counterpart. Expression of LMTK3 was absent or low in normal tissues while cases of hepatocellular, bladder and gastric cancer had low to moderate expression of LMTK3. For other organ specific tumors (brain, ovarian, colon, prostate, skin, liver, lung, lymphoma, testis, thyroid, esophagus, head & neck, cervix, renal and pancreatic), expression of LMTK3 matched the normal tissue counterpart.

### Discussion

We have used a siRNA screen targeting all known kinases with expression of estrogen-regulated genes as a read-out, to identify LMTK3 as a new oncogenic regulator of ERα function¹⁴ (see preceding Examples). We now demonstrate that both nuclear and cytoplasmic expression of LMTK3 is associated with several parameters of more aggressive breast cancers. Independently, we use as validation an Asian cohort and demonstrate that even with a relatively smaller sample size, high levels correlate with shorter survival parameters. These data validate our previous findings.

Collectively, our results strongly suggest that as for ERα expression, scoring of LMTK3 should be nuclear; several assays we have used demonstrate physical and functional co-localization of these two proteins in the nucleus. High cytoplasmic staining was observed in 10%, whereas high nuclear staining in 39% of patients, using our pre-specified cut-offs. Larger tumors were observed to be LMTK3 positive in both the European and Asian cohorts and basal-like cancers were more common with LMTK3 positivity. Of all the tumor types tested, breast cancer appears to be unique in having consistently high LMTK3 staining, with other cancers demonstrating moderate staining at best (Fig 34). We are conscious that despite years of research and hundreds of reports on tumor markers in oncology, the number of markers that have emerged as clinically useful is pitifully small. Often initially reported studies of a marker show great promise, but subsequent studies on the same or related markers yield inconsistent conclusions or stand in direct contradiction to the initial promising results, as per the REMARK guidelines which were followed here^{24,25}. The independent validation here is limited by its small sample size. However, we showed many of the similar characteristics observed in the much larger European group of patients, and these data reinforce the proposition that 'Western' data merits validation with Asian cohorts, and vice versa. Both cohorts failed to demonstrate an association with ERα levels, and we suggest that their functional interaction as opposed to absolute levels is most relevant here, although this may also reflect the wide variety of methods available to measure ERα levels.

In our description of LMTK3, we suggest that the exonic sequence changes between our closest living relatives, Chimpanzees, and humans, may contribute to the unique susceptibility of humans to ERα positive breast cancer. While it may be suggested that dietary and environmental factors also contribute, the consistency of our data especially overall survival, between British and Asian patients further reinforces the role of this intriguing new kinase, which further suggest it has the potential to be a druggable target. The majority of breast tumors, in humans at least, express ERα and patients with ERα⁺ disease usually respond to endocrine therapies. Endocrine resistance is a major problem in breast cancer treatment, highlighting a need for understanding the mechanisms of ERα action and the development of new therapeutic agents many of which are destined to be kinase inhibitors^{26,27}. We now demonstrate that the new kinase LMTK3 is associated with a number of parameters typically associated with aggressive tumors such as size and grade, and validate our clinical outcomes with an independent patient cohort. Prospective validation and mechanistic interpretation of the role of LMTK3 levels in tamoxifen-resistance, as well as of the significance of chemotherapy induced increase of LMTK3 levels, will be required to strengthen these data.

### References

1. Stebbing J, Crane J, Gaya A. Breast cancer (metastatic). Clin Evid 2006(15):2331-59.
2. Stebbing J, Delaney G, Thompson A. Breast cancer (non-metastatic). Clin Evid (Online) 2007;2559-1602.
3. Hammond ME, Hayes DF, Dowsett M, et al. American Society of Clinical Oncology/College Of American Pathologists guideline recommendations for immunohistochemical testing of estrogen and progesterone receptors in breast cancer. J Clin Oncol 2010;28(16):2784-95*.*
4. Kato S, Endoh H, Masuhiro Y, et al. Activation of the estrogen receptor through phosphorylation by mitogen-activated protein kinase. Science 1995;270(5241):1491-4.
5. Ali S, Coombes RC. Endocrine-responsive breast cancer and strategies for combating resistance. Nat Rev Cancer 2002;2(2):101-12.
6. Amanchy R, Kalume DE, Iwahori A, Zhong J, Pandey A. Phosphoproteome analysis of HeLa cells using stable isotope labeling with amino acids in cell culture (SILAC). J Proteome Res 2005;4(5):1661-71.
7. Atsriku C, Britton DJ, Held JM, et al. Systematic mapping of posttranslational modifications in human estrogen receptor-alpha with emphasis on novel phosphorylation sites. Mol Cell Proteomics 2009;8(3):467-80.
8. Campbell RA, Bhat-Nakshatri P, Patel NM, Constantinidou D, Ali S, Nakshatri H. Phosphatidylinositol 3-kinase/AKT-mediated activation of estrogen receptor alpha: a new model for anti-estrogen resistance. J Biol Chem 2001;276(13):9817-24.
9. Britton DJ, Scott GK, Schilling B, et al. A novel serine phosphorylation site detected in the N-terminal domain of estrogen receptor isolated from human breast cancer cells. J Am Soc Mass Spectrom 2008;19(5):729-40.
10. Wu RC, Qin J, Yi P, et al. Selective phosphorylations of the SRC-3/AIB1 coactivator integrate genomic reponses to multiple cellular signaling pathways. Mol Cell 2004;15(6):937-49.
11. Jiang J, Sarwar N, Peston D, et al. Phosphorylation of estrogen receptor-alpha at Ser167 is indicative of longer disease-free and overall survival in breast cancer patients. Clin Cancer Res 2007;13(19):5769-76.
12. Giamas G, Castellano L, Feng Q, et al. CK1delta modulates the transcriptional activity of ERalpha via AIB1 in an estrogen-dependent manner and regulates ERalpha-AIB1 interactions. Nucleic Acids Res 2009;37(9):3110-23.
13. Chen D, Washbrook E, Sarwar N, et al. Phosphorylation of human estrogen receptor alpha at serine 118 by two distinct signal transduction pathways revealed by phosphorylation-specific antisera. Oncogene 2002;21(32):4921-31.
14. Giamas G, Filipovic A, Jacob J, et al. Kinome screening for regulators of the estrogen receptor identifies LMTK3 as a new therapeutic target in breast cancer. Nature Medicine 2011 (In press).
15. Bowen RL, Stebbing J, Jones LJ. A review of the ethnic differences in breast cancer. Pharmacogenomics 2006;7(6):935-42.
16. Dunn BK, Agurs-Collins T, Browne D, Lubet R, Johnson KA. Health disparities in breast cancer: biology meets socioeconomic status. Breast Cancer Res Treat 2010;121(2):281-92.
17. Chuah BY, Putti T, Salto-Tellez M, et al. Serial changes in the expression of breast cancer-related proteins in response to neoadjuvant chemotherapy. Ann Oncol 2011.
18. Walker RA, Bartlett JM, Dowsett M, et al. HER2 testing in the UK: further update to recommendations. J Clin Pathol 2008;61(7):818-24.
19. Bartlett JM, Ellis IO, Dowsett M, et al. Human epidermal growth factor receptor 2 status correlates with lymph node involvement in patients with estrogen receptor (ER) negative, but with grade in those with ER-positive early-stage breast cancer suitable for cytotoxic chemotherapy. J Clin Oncol 2007;25(28):4423-30.
20. Elston CW, Ellis IO. Pathological prognostic factors in breast cancer. I. The value of histological grade in breast cancer: experience from a large study with long-term follow-up. Histopathology 1991;19(5):403-10.
21. Ellis IO, Galea M, Broughton N, Locker A, Blamey RW, Elston CW. Pathological prognostic factors in breast cancer. II. Histological type. Relationship with survival in a large study with long-term follow-up. Histopathology 1992;20(6):479-89.
22. Parker C, Rampaul RS, Pinder SE, et al. E-cadherin as a prognostic indicator in primary breast cancer. Br J Cancer 2001 ;85(12):1958-63.
23. Madjd Z, Parsons T, Watson NF, Spendlove I, Ellis I, Durrant LG. High expression of Lewis y/b antigens is associated with decreased survival in lymph node negative breast carcinomas. Breast Cancer Res 2005;7(5):R780-7.
24. McShane LM, Altman DG, Sauerbrei W, Taube SE, Gion M, Clark GM. REporting recommendations for tumor MARKer prognostic studies (REMARK). Breast Cancer Res Treat 2006;100(2):229-35.
25. McShane LM, Altman DG, Sauerbrei W, Taube SE, Gion M, Clark GM. Reporting recommendations for tumor marker prognostic studies (remark). Exp Oncol 2006;28(2):99-105.
26. Giamas G, Stebbing J, Vorgias CE, Knippschild U. Protein kinases as targets for cancer treatment. Pharmacogenomics 2007;8(8):1005-16.
27. Giamas G, Man YL, Hirner H, et al. Kinases as targets in the treatment of solid tumors. Cell Signal 2010;22(7):984-1002.

### Example 6: LMTK3 inhibitor screening assay

### Methods

This exemplary assay has been developed to find inhibitors of LMTK3. The LMTK3 kinase domain (amino acids 133-415 of the full length kinase sequence, with an N-terminal GST tag) was expressed in insect cells and isolated via the GST N-terminal epitope.

Kinase reactions are set up in the presence of 1 % DMSO with ATP added at around *K*_{M} (= 10 µM). Kinase activity is monitored using the CisBio KinEASE STK S1 kit using a ratiometric fluorescent read-out at 665 and 620 nm. The assay uses 10 nM LMTK3, 1 µM S1 peptide, 10 mM MgCl₂ in the manufacturer buffer with extension of reaction time to 120 mins at 37 °C.

Screening was performed in 384 low volume black microtitre plates with a final volume of 20 µl. The reaction was stopped with the addition of EDTA and detection reagents and fluorescence read on Pherastar. Data was normalised to the high control as specified by no inhibition and the low controls, equating to no activity (absence of ATP). Inhibitor activity was determined using concentration response curves, and using a four parameter fit to identify pIC50 values. Assay robustness was calculated using Z prime.

### Results

Quality of the assay was measured by calculating Z prime gave an average of 0.60 ± 0.08 (N=12). The following pIC50 values were resolved for staurosporine- like compounds, a family of broad spectrum kinase inhibitors.

| **Compound** | **Average pIC50** | **SEM pIC50** | **N pIC50** | **Average % Inhibition** |
|---|---|---|---|---|
| **K-252a** | 7.10 | 0.19 | 5 | 98.91 |
| **KT5720** | 9.93 | 0.35 | 5 | 98.65 |
| **Staurosporine** | 11.62 | 0.31 | 5 | 108.86 |
| **GF109203X** | 9.88 | 0.19 | 5 | 107.89 |

## Claims

1. A method for aiding in determining prognosis in a patient with breast cancer, or in selecting a therapeutic strategy for a patient with breast cancer, the method comprising the step of assessing the level of LMTK3 nucleic acid, protein or activity in a sample obtained from the patient.

2. The method of claim 1 further comprising the step of selecting a treatment regime making use of the information on the level of LMTK3 nucleic acid, protein or activity in the sample.

3. The method of claim 1 or 2 further comprising the step of assessing the level of ERα nucleic acid, protein or activity in a sample obtained from the patient.

4. The method of claim 2 or 3 wherein if the level of LMTK3 nucleic acid, protein or activity in the sample is an elevated level then the selected treatment regime includes one or more of surgery, chemotherapy, radiotherapy and hormonal therapy; or
wherein if the level of LMTK3 nucleic acid, protein or activity in the sample is an elevated level then the selected treatment regime comprises treating the patient with an inhibitor of LMTK3 activity.

5. The method of claim 2 or 3 wherein if the level of LMTK3 nucleic acid, protein or activity in the sample is not elevated then the selected treatment regime includes one or more of watchful waiting or adjuvant hormonal treatment such as tamoxifen, a GnRH analogue or an aromatase inhibitor such as anastrazole, letrozole or exemestane.

6. A method for aiding in selecting a therapeutic strategy for a patient with breast cancer who is receiving endocrine therapy, or a patient who has previously received or is receiving endocrine therapy and has relapsed, the method comprising the steps of (i) assessing the level of LMTK3 nucleic acid, protein or activity in a sample obtained from the patient and (ii) assessing the endocrine resistance status of the patient, optionally further comprising the step of assessing the level of ERα nucleic acid, protein or activity in a sample obtained from the patient.

7. The method of claim 6, wherein (i) if the level of LMTK3 nucleic acid, protein or activity in the sample is an elevated level; and (ii) if the patient is assessed as having an elevated endocrine resistance status or being resistant to endocrine therapy, then the selected treatment regime comprises treating the patient with an inhibitor of LMTK3 activity in combination with endocrine therapy, for example adjuvant hormonal treatment such as tamoxifen, a GnRH analogue or an aromatase inhibitor such as anastrazole, letrozole or exemestane.

8. A method for aiding in determining whether a patient with breast cancer has a relatively high or relatively low likelihood of disease free survival, the method comprising the step of assessing the level of LMTK3 nucleic acid or protein in a sample obtained from the patient.

9. The method of any one of the preceding claims, wherein the sample obtained from the patient is a sample of the tissue in which breast cancer is suspected or in which breast cancer has been found, or contains cells from said tissue, optionally wherein the sample is any one of a sample of breast obtained by surgical excision, "true cut" biopsy, needle biopsy, nipple aspirate, aspiration of a lump or image-guided biopsy, or biopsy or needle aspirate of a suspected metastatic site such as the liver or lungs or bones.

10. An inhibitor of LMTK3 activity for use in treating a patient with breast cancer or for inhibiting breast cancer cell proliferation in a patient with breast cancer; optionally wherein the patient is a patient in which the level of LMTK3 nucleic acid or protein in a sample from the patient has been determined to be elevated.

11. Use of an inhibitor of LMTK3 activity in the manufacture of a medicament for treating a patient with breast cancer, or for inhibiting breast cancer cell proliferation in a patient with breast cancer; optionally wherein the patient is a patient in which the level of LMTK3 nucleic acid or protein in a sample from the patient has been determined to be elevated.

12. The inhibitor of LKTK3 activity for use of claim 10 or use of claim 11 wherein the inhibitor of LMTK3 activity is an inhibitor of LMTK3 expression, for example an anti LMTK3 siRNA molecule.

13. The inhibitor of LKTK3 activity for use of claim 10 or 12, or use of claim 11 or 12, wherein the patient is administered an additional anti-cancer agent or treatment.

14. The inhibitor of LMTK3 activity for use of claim 13, wherein the additional anti-cancer agent or treatment includes, or further includes, endocrine therapy, for example adjuvant hormonal treatment such as tamoxifen, a GnRH analogue or an aromatase inhibitor such as anastrazole, letrozole or exemestane.

15. An inhibitor of LMTK3 activity for use in treating breast cancer in a patient who is being treated with anti-oestrogen endocrine therapy, optionally wherein the patient is administered a further anti-cancer agent or treatment.

16. An anti-oestrogen endocrine therapeutic for use in treating breast cancer in a patient who is being treated with an inhibitor of LMTK3 activity, optionally wherein the patient is administered a further anti-cancer agent or treatment.

17. A screening method for identifying a compound likely to be useful in treating breast cancer, the method comprising the step of determining the effect of a test compound on LMTK3 nucleic acid, protein or activity level; and selecting a compound that reduces said level, wherein the effect of the test compound is determined *in vitro,* optionally in a breast cancer cell or cell line, or is determined *in vivo* in a non-human test animal.

## Patentansprüche

1. Verfahren zum Helfen beim Bestimmen einer Prognose bei einer Patientin mit Brustkrebs oder beim Auswählen einer therapeutischen Strategie für eine Patientin mit Brustkrebs, wobei das Verfahren den Schritt des Auswertens des Spiegels von LMTK3-Nukleinsäure, -Protein oder-Aktivität in einer von der Patientin erhaltenen Probe umfasst.

2. Verfahren nach Anspruch 1, ferner den folgenden Schritt umfassend: Auswählen eines Therapieplans, der die Informationen zum Spiegel von LMTK3-Nukleinsäure, -Protein oder -Aktivität in der Probe nutzt.

3. Verfahren nach Anspruch 1 oder 2, ferner den Schritt des Auswertens des Spiegels von ERa-Nukleinsäure, -Protein oder-Aktivität in einer von der Patientin erhaltenen Probe umfassend.

4. Verfahren nach Anspruch 2 oder 3, wobei, wenn der Spiegel von LMTK3-Nukleinsäure, -Protein oder -Aktivität in der Probe ein erhöhter Spiegel ist, das ausgewählte Behandlungssystem dann Chirurgie, Chemotherapie, Strahlentherapie und/oder Hormontherapie enthält; oder
wobei, wenn der Spiegel von LMTK3-Nukleinsäure, -Protein oder -Aktivität in der Probe ein erhöhter Spiegel ist, das ausgewählte Behandlungssystem dann das Behandeln der Patientin mit einem Hemmer von LMTK3-Aktivität umfasst.

5. Verfahren nach Anspruch 2 oder 3, wobei, wenn der Spiegel von LMTK3-Nukleinsäure, -Protein oder -Aktivität in der Probe nicht erhöht ist, das ausgewählte Behandlungssystem dann eines oder mehrere der Folgenden umfasst: aufmerksame abwartendBeobachtung und unterstützende Hormontherapie, wie etwa mit Tamoxifen, einem GnRH-Analogon oder einem Aromatasehemmer wie etwa Anastrazol, Letrozol oder Exemestan.

6. Verfahren zum Unterstützen beim Auswählen einer Therapiestrategie für eine Patientin mit Brustkrebs, die eine Endokrintherapie erhält, oder eine Patientin, die zuvor Endokrintherapie erhalten hat oder erhält und einen Rückfall erlitten hat, wobei das Verfahren die folgenden Schritte umfasst: (i) Auswerten des Spiegels von LMTK3-Nukleinsäure, -Protein oder-Aktivität in einer von der Patientin erhaltenen Probe und (ii) Auswerten des Endokrinwiderstandsstatus der Patientin, optional ferner den Schritt des Auswertens des Spiegels von ERa-Nukleinsäure, -Protein oder -Aktivität in einer von der Patientin erhaltenen Probe.

7. Verfahren nach Anspruch 6, wobei, (i) wenn der Spiegel von LMTK3-Nukleinsäure, - Protein oder -Aktivität in der Probe ein erhöhter Spiegel ist; und (ii) wenn festgestellt wird, dass die Patientin einen erhöhten Endokrinwiderstandsstatus aufweist oder gegen Endokrintherapie resistent ist, dann das ausgewählte Behandlungssystem das Behandeln der Patientin mit einem Hemmer von LMTK3-Aktivität in Kombination mit Endokrintherapie, zum Beispiel unterstützender Hormontherapie, wie etwa mit Tamoxifen, einem GnRH-Analogon oder einem Aromatasehemmer wie etwa Anastrazol, Letrozol oder Exemestan umfasst.

8. Verfahren zum Helfen beim Bestimmen, ob eine Patientin mit Brustkrebs eine vergleichsweise hohe oder eine vergleichsweise niedrige Wahrscheinlichkeit eines krankheitsfreien Überlebens aufweist, wobei das Verfahren den Schritt des Auswertens des Spiegels von LMTK3-Nukleinsäure oder-Protein in einer von der Patientin erhaltenen Probe umfasst.

9. Verfahren nach einem der vorhergehenden Ansprüche, wobei die von der Patientin erhaltene Probe eine Probe des Gewebes ist, in dem Brustkrebs vermutet wird oder in dem Brustkrebs gefunden worden ist, oder Zellen aus dem Gewebe enthält, wobei die Probe optional eine Brustgewebeprobe ist, erhalten durch chirurgische Extraktion, "True-Cut"-Biopsie, Nadelbiopsie, Nippelaspirat, Aspiration eines Knotens und/oder bildgeführte Biopsie, oder eine Biopsie oder ein Nadelaspirat einer vermuteten metastatischen Stelle, wie etwa der Leber, der Lungen oder der Knochen.

10. Hemmer für LMTK3-Aktivität für den Gebrauch beim Behandeln einer Patientin mit Brustkrebs oder zum Hemmen von Brustkrebszellproliferation in einer Patientin mit Brustkrebs; wobei die Patientin optional eine Patientin ist, bei der der Spiegel von LMTK3-Nukleinsäure oder -Protein in einer Probe von der Patientin als erhöht bestimmt worden ist.

11. Gebrauch eines Hemmers für LMTK3-Aktivität beim Herstellen eines Medikaments zum Behandeln einer Patientin mit Brustkrebs oder zum Hemmen von Brustkrebszellproliferation in einer Patientin mit Brustkrebs; wobei die Patientin optional eine Patientin ist, bei der der Spiegel von LMTK3-Nukleinsäure oder -Protein in einer Probe von der Patientin als erhöht bestimmt worden ist.

12. Hemmer für LKTK3-Aktivität für den Gebrauch nach Anspruch 10 oder Gebrauch nach Anspruch 11, wobei der Hemmer für LMTK3-Aktivität ein Hemmer für LMTK3-Exprimierung ist, zum Beispiel ein Anti-LMTK3-siRNA-Molekül.

13. Hemmer für LKTK3-Aktivität für den Gebrauch nach Anspruch 10 oder 12 oder Gebrauch nach Anspruch 11 oder 12, wobei der Patientin ein weiteres Antikrebsmittel oder eine Antikrebsbehandlung verabreicht wird.

14. Hemmer für LMTK3-Aktivität für den Gebrauch nach Anspruch 13, wobei das weitere Antikrebsmittel oder die Antikrebsbehandlung Endokrintherapie enthält, oder weiterhin enthält, wie zum Beispiel unterstützende Hormontherapie, wie etwa mit Tamoxifen, einem GnRH-Analogon oder einem Aromatasehemmer wie etwa Anastrazol, Letrozol oder Exemestan.

15. Hemmer für LMTK3-Aktivität für den Gebrauch beim Behandeln von Brustkrebs in einer Patientin, die mit Anti-Östrogen-Endokrintherapie behandelt wird, wobei der Patientin optional ein weiteres Antikrebsmittel oder eine Antikrebsbehandlung verabreicht wird.

16. Antiöstrogentherapie für den Gebrauch beim Behandeln von Brustkrebs in einer Patientin, die mit einem Hemmer für LMTK3-Aktivität behandelt wird, wobei der Patientin optional ein weiteres Antikrebsmittel oder eine Antikrebsbehandlung verabreicht wird.

17. Früherkennungsverfahren zum Identifizieren einer Verbindung, die wahrscheinlich nützlich ist zum Behandeln von Brustkrebs, wobei das Verfahren Folgendes umfasst: den Schritt des Bestimmens der Auswirkung einer Testverbindung auf LMTK3-Nukleinsäure, - Protein oder-Aktivität; und Auswählen einer Verbindung, die den Spiegel verringert, wobei die Auswirkungen der Testverbindung *in vitro,* optional in einer Brustkrebs-Zelle oder-Zelllinie bestimmt werden, oder *in vivo* in einem nicht menschlichen Versuchstier bestimmt werden.

## Revendications

1. Méthode permettant de déterminer plus facilement le pronostic chez une patiente présentant un cancer du sein ou de choisir une stratégie thérapeutique pour une patiente présentant un cancer du sein, la méthode comprenant l'étape d'évaluation du taux d'acide nucléique codant pour la protéine LMTK3, de cette protéine ou de son activité dans un échantillon obtenu chez la patiente.

2. Méthode selon la revendication 1, qui comprend en outre l'étape de sélection d'un schéma de traitement en utilisant les informations concernant le taux d'acide nucléique codant pour la protéine LMTK3, le taux de cette protéine ou son activité dans l'échantillon.

3. Méthode selon la revendication 1 ou 2, qui comprend en outre l'étape d'évaluation du taux d'acide nucléique codant pour le récepteur aux oestrogènes alpha (REα), de cette protéine ou de son activité dans un échantillon obtenu chez la patiente.

4. Méthode selon la revendication 2 ou 3, où le schéma de traitement sélectionné inclut une ou plusieurs des approches suivantes si le taux d'acide nucléique codant pour la protéine LMTK3, le taux de cette protéine ou son activité dans l'échantillon est élevé :
chirurgie, chimiothérapie, radiothérapie et hormonothérapie ; ou
où le schéma de traitement sélectionné comprend le traitement de la patiente par un inhibiteur de l'activité LMTK3 si le taux d'acide nucléique codant pour la protéine LMTK3, le taux de cette protéine ou son activité dans l'échantillon est élevé.

5. Méthode selon la revendication 2 ou 3, où le schéma de traitement sélectionné inclut une ou plusieurs des approches suivantes si le taux d'acide nucléique codant pour la protéine LMTK3, le taux de cette protéine ou son activité dans l'échantillon n'est pas élevé :
surveillance rapprochée ou hormonothérapie adjuvante telle qu'un traitement par le tamoxifène, un analogue de la GnRH ou un inhibiteur de l'aromatase comme l'anastrozole, le létrozole ou l'exemestane.

6. Méthode permettant de sélectionner plus facilement une stratégie thérapeutique pour une patiente présentant un cancer du sein qui reçoit une thérapie endocrinienne ou pour une patiente en rechute qui a précédemment reçu ou qui reçoit une thérapie endocrinienne, la méthode comprenant les étapes (i) d'évaluation du taux d'acide nucléique codant pour la protéine LMTK3, de cette protéine ou de son activité dans un échantillon obtenu chez la patiente et (ii) d'évaluation du statut de la patiente en matière de résistance à la thérapie endocrinienne, la méthode comprenant en outre éventuellement l'étape d'évaluation du taux d'acide nucléique codant pour le REα, de cette protéine ou de son activité dans un échantillon obtenu chez la patiente.

7. Méthode selon la revendication 6, où le schéma de traitement sélectionné comprend le traitement de la patiente par un inhibiteur de l'activité LMTK3 combiné à une thérapie endocrinienne, par exemple une hormonothérapie adjuvante telle qu'un traitement par le tamoxifène, un analogue de la GnRH ou un inhibiteur de l'aromatase comme l'anastrozole, le létrozole ou l'exemestane (i) si le taux d'acide nucléique codant pour la protéine LMTK3, le taux de cette protéine ou son activité dans l'échantillon est élevé et (ii) si les évaluations indiquent que le statut de la patiente en matière de résistance à la thérapie endocrinienne est positif ou que la patiente est résistante à la thérapie endocrinienne.

8. Méthode permettant de déterminer plus facilement si la probabilité de survie sans rechute est relativement élevée ou relativement basse chez une patiente présentant un cancer du sein, la méthode comprenant l'étape qui consiste à évaluer le taux d'acide nucléique codant pour la protéine LMTK3 ou le taux de cette protéine dans un échantillon obtenu chez la patiente.

9. Méthode selon l'une quelconque des revendications précédentes, où l'échantillon obtenu chez la patiente est un échantillon du tissu dans lequel un cancer du sein est suspecté ou dans lequel un cancer du sein a été détecté ou qui contient des cellules dudit tissu, l'échantillon étant éventuellement de l'un quelconque des types suivants : échantillon de tissu mammaire obtenu par exérèse chirurgicale, biopsie de type « *true cut* », biopsie à l'aiguille, aspiration mamelonnaire, aspiration au niveau d'une masse mammaire ou biopsie guidée par imagerie, ou biopsie ou aspiration à l'aiguille d'un site métastatique suspecté comme le foie, les poumons ou les os.

10. Inhibiteur de l'activité LMTK3 pour une utilisation dans le traitement d'une patiente présentant un cancer du sein ou dans l'inhibition de la prolifération des cellules du sein cancéreuses chez une patiente présentant un cancer du sein ; la patiente étant éventuellement une patiente chez laquelle le taux d'acide nucléique codant pour la protéine LMTK3 ou le taux de cette protéine s'est révélé élevé dans un échantillon obtenu chez la patiente.

11. Inhibiteur de l'activité LMTK3 dans la fabrication d'un médicament pour le traitement d'une patiente présentant un cancer du sein ou pour l'inhibition de la prolifération des cellules du sein cancéreuses chez une patiente présentant un cancer du sein ; la patiente étant éventuellement une patiente chez laquelle le taux d'acide nucléique codant pour la protéine LMTK3 ou le taux de cette protéine s'est révélé élevé dans un échantillon obtenu chez la patiente.

12. Inhibiteur de l'activité LKTK3 pour une utilisation selon la revendication 10 ou une utilisation selon la revendication 11, où l'inhibiteur de l'activité LMTK3 est un inhibiteur de l'expression de la LMTK3, par exemple un siARN anti-LMTK3.

13. Inhibiteur de l'activité LKTK3 pour une utilisation selon la revendication 10 ou 12 ou une utilisation selon la revendication 11 ou 12, où la patiente reçoit un agent ou traitement anticancéreux supplémentaire.

14. Inhibiteur de l'activité LMTK3 pour une utilisation selon la revendication 13, où l'agent ou le traitement anticancéreux supplémentaire inclut, ou inclut en outre, une thérapie endocrinienne, par exemple une hormonothérapie adjuvante telle qu'un traitement par le tamoxifène, un analogue de la GnRH ou un inhibiteur de l'aromatase comme l'anastrozole, le létrozole ou l'exemestane.

15. Inhibiteur de l'activité LMTK3 pour une utilisation dans le traitement du cancer du sein chez une patiente qui reçoit une thérapie endocrinienne par un anti-oestrogène, la patiente recevant éventuellement un agent ou traitement anticancéreux supplémentaire.

16. Thérapie endocrinienne par un anti-oestrogène pour une utilisation dans le traitement du cancer du sein chez une patiente qui reçoit un inhibiteur de l'activité LMTK3, la patiente recevant éventuellement un agent ou traitement anticancéreux supplémentaire.

17. Méthode de prospection systématique pour identifier un composé susceptible d'être utile dans le traitement du cancer du sein, la méthode comprenant l'étape qui consiste à déterminer l'effet d'un composé à l'essai sur le taux d'acide nucléique codant pour la protéine LMTK3, le taux de cette protéine ou son activité ; et la sélection d'un composé qui réduit ledit taux, l'effet du composé à l'essai étant évalué *in vitro,* éventuellement sur des cellules du sein cancéreuses ou sur une lignée de cellules du sein cancéreuses ou *in vivo* chez un animal d'expérience non humain.
